(19) Europäisches Patentamt
European Patent Office
Office européen des brevets

(11) **EP 4 553 489 A1**

(12) **EUROPEAN PATENT APPLICATION**
published in accordance with Art. 153(4) EPC

(43) Date of publication:
**14.05.2025  Bulletin 2025/20**

(21) Application number: **23835817.0**

(22) Date of filing: **05.07.2023**

(51) International Patent Classification (IPC):
**G01N 21/64** (2006.01)    **C12Q 1/686** (2018.01)

(52) Cooperative Patent Classification (CPC):
**C12Q 1/686; G01N 21/64**

(86) International application number:
**PCT/KR2023/009459**

(87) International publication number:
**WO 2024/010351 (11.01.2024 Gazette 2024/02)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **05.07.2022  KR 20220082751**

(71) Applicant: **Seegene, Inc.**
**Seoul 05548 (KR)**

(72) Inventors:
- KIM, Sul Gi
  Seoul 05548 (KR)
- EO, Soo Heang
  Seoul 06592 (KR)
- LEE, Han Bit
  Seoul 05546 (KR)
- MIN, Iljae
  Seoul 05620 (KR)

(74) Representative: **Viering, Jentschura & Partner
mbB**
**Patent- und Rechtsanwälte**
**Am Brauhaus 8**
**01099 Dresden (DE)**

(54) **METHOD FOR OBTAINING APPROXIMATE SIGNAL FOR EACH OF MULTIPLE TARGET ANALYTES, AND COMPUTER DEVICE FOR PERFORMING SAME**

(57)    A method for obtaining an approximate signal for each of a plurality of target analytes performed by a computer device includes: obtaining, from a signal generation reaction for a first target analyte and a second target analyte in a sample, a first data set measured at a first detection temperature and a second data set measured at a second detection temperature; and by using the first and the second data set, performing a joint-estimation on (a) a value of a parameter of at least one among an approximate function for approximating signal values dependent on the presence of the first target analyte and an approximate function for approximating signal values dependent on the presence of the second target analyte; and (b) a value of a magnitude parameter indicating a relationship of signal values dependent on the presence of the first target analyte between the first and the second detection temperature.

*FIG.13*

```
                    START

OBTAINING, FROM SIGNAL GENERATION REACTION OF
THE PLURALITY OF CYCLES FOR THE FIRST TARGET ANALYTE
AND THE SECOND TARGET ANALYTE IN THE SAMPLE,
THE FIRST DATA SET MEASURED AT THE FIRST DETECTION      ~S1310
TEMPERATURE AT EACH OF THE PLURALITY OF CYCLES AND
THE SECOND DATA SET MEASURED AT THE SECOND DETECTION
TEMPERATURE AT EACH OF THE PLURALITY OF CYCLES

BY AT LEAST PARTIALLY USING THE FIRST DATA SET AND THE SECOND DATA SET,
PERFORMING THE JOINT-ESTIMATION ON VALUE OF AT LEAST ONE APPROXIMATE FUNCTION
PARAMETER OF AT LEAST ONE APPROXIMATE FUNCTION AND VALUE OF MAGNITUDE PARAMETER;
WHEREIN THE AT LEAST ONE APPROXIMATE FUNCTION INCLUDES AT LEAST ONE SELECTED FROM
THE GROUP CONSISTING OF: THE APPROXIMATE FUNCTION 1-1 FOR APPROXIMATING SIGNAL VALUES
DEPENDENT ON THE PRESENCE OF THE FIRST TARGET ANALYTE AT THE FIRST DETECTION
TEMPERATURE; THE APPROXIMATE FUNCTION 1-2 FOR APPROXIMATING SIGNAL VALUES DEPENDENT    ~S1320
ON THE PRESENCE OF THE FIRST TARGET ANALYTE AT THE SECOND DETECTION TEMPERATURE; AND
THE APPROXIMATE FUNCTION 2 FOR APPROXIMATING SIGNAL VALUES DEPENDENT ON THE PRESENCE
OF THE SECOND TARGET ANALYTE AT THE SECOND DETECTION TEMPERATURE;
WHEREIN THE MAGNITUDE PARAMETER INDICATES RELATIONSHIP BETWEEN SIGNAL VALUES
DEPENDENT ON THE PRESENCE OF THE FIRST TARGET ANALYTE AT THE FIRST DETECTION
TEMPERATURE AND SIGNAL VALUES DEPENDENT ON THE PRESENCE OF THE FIRST TARGET ANALYTE
AT THE SECOND DETECTION TEMPERATURE

                    END
```

EP 4 553 489 A1

**Description**

**TECHNICAL FIELD**

The present disclosure relates to a method for obtaining an approximate signal for each of a plurality of target analytes and a computer device for performing the same.

**BACKGROUND ART**

A nucleic acid amplification reaction known as a Polymerase chain reaction (PCR) includes repeated cycles of denaturation process of double-stranded DNA, annealing process of oligonucleotide primers to a DNA template, and extension process of primers by DNA polymerase (Mullis et al., U.S. Pat. No. 4,683,195, 4,683,202 and 4,800,159; Saiki et al., Science 230:1350-1354 (1985)).

Real-time PCR is a PCR-based technique for detecting target nucleic acids in a sample in real-time. In order to detect a specific target nucleic acid, a signal generation means that emits a detectable fluorescent signal in proportion to the amount of the target nucleic acid during the PCR reaction is used. A fluorescence signal proportional to the amount of the target nucleic acid is detected at each measurement point (cycle) through real-time PCR to obtain a data set including each measurement point and signal values at the measurement points, and an amplification curve or an amplification profile curve indicating the intensity of the detected fluorescence signal compared to the measurement point is obtained from the data set.

In general, an amplification curve by real-time PCR is divided into a baseline region, an exponential region, and a plateau region. The exponential region is a region in which a fluorescence signal emitted in proportion to an increase in a PCR amplification product is increased, and the plateau region is a region in which an increase in the PCR amplification product and the emission of the fluorescence signal reaches a saturation state and an increase in the fluorescence signal no longer appears. In the baseline region, the change in the magnitude of the fluorescence signal at the beginning of the reaction is not remarkable. In the baseline region, the fluorescent signal emitted by the PCR reaction product is not sufficient to be detected. Therefore, in this region, a background signal, which is a fluorescent signal of the reaction sample itself and a fluorescent signal of the measurement system itself, occupies most of the fluorescent signal, rather than the fluorescent signal obtained by amplifying the target analyte.

Various methods have been developed to determine whether there is amplification of a plurality of target analytes from a data set of real-time PCR. Among them, a method of detecting two target nucleic acid sequences in a sample by using different detection temperatures is known (Korean Patent No. 10-2050601). According to the known method, the sample is incubated with two signal generation compositions for detecting two target nucleic acid sequences having respective a detection temperature of relatively high temperature and a detection temperature of relatively low temperature in one reaction well, and the signals generated at the respective detection temperature of relatively high temperature and detection temperature of relatively low temperature can be detected by using a single type of detector. In this case, the signal detected at the detection temperature of relatively high temperature may include a signal for the target nucleic acid sequence having the detection temperature of relatively high temperature. The signal detected at the detection temperature of relatively low temperature may include both a signal for the target nucleic acid sequence having the detection temperature of relatively low temperature and a signal for the target nucleic acid sequence having the detection temperature of relatively high temperature. The presence of the target nucleic acid sequence having the detection temperature of relatively high temperature can be determined by a signal detected at the detection temperature of relatively high temperature, and the presence of the target nucleic acid sequence having the detection temperature of relatively low temperature can be determined by the difference between a signal detected at the detection temperature of relatively high temperature and a signal detected at the detection temperature of relatively low temperature using a reference value. In this case, the reference value is a value obtained in advance, and is obtained from a difference(s) between a signal detected at the detection temperature of relatively high temperature and a signal detected at the detection temperature of relatively low temperature, which are detected as the target nucleic acid sequence having the detection temperature of relatively high temperature is incubated with the signal generation composition in a reaction well(s) other than the above one reaction well.

As described above, in order to detect a plurality of target analytes, a task of extracting signal values corresponding to each of the plurality of target analytes from the signal values detected at each of the plurality of temperatures by using the above-described reference value is generally performed. However, in the related art, the reference value is a value obtained in advance from signals in another reaction wells, and is not a value derived from a signal in the target reaction well in which the plurality of target analytes are to be detected. Accordingly, there is a limitation in that it is difficult to reflect a difference generated according to a detection environment such as a reaction well, a detection device, or the like. In addition, the reference value is obtained only when the target nucleic acid sequence having the detection temperature of relatively low temperature does not exist and the target nucleic acid sequence having the detection temperature of relatively high temperature exists in the reaction well, by obtaining a difference between a signal detected at the detection temperature of relatively high temperature and a signal detected at the detection temperature of relatively low temperature, and thus there is a limitation that the reference value cannot be obtained in an unknown target reaction well in which the presence or absence of the target analyte is unknown.

Accordingly, there is a need for a technology capable of estimating the reference values and signal values corresponding to the plurality of target analytes from the signal value detected at each of the plurality of temperatures without using the previously obtained reference value.

## DETAILED DESCRIPTION OF INVENTION

### TECHNICAL PROBLEMS

A problem to be solved by the present disclosure is to provide a technology for obtaining an approximate signal for each of a plurality of target analytes from signal values detected at each of different temperatures.

In addition, the process of obtaining the approximate signal includes a process of separating or extracting only signal values for a specific target analyte from the detected signal value, and in this process, a predetermined parameter is used. The problem to be solved may include that the value of this parameter is applied as an optimized value for each reaction well.

However, problems to be solved by the present disclosure are not limited to the foregoing, and other unmentioned objects would be apparent to one of ordinary skill in the art from the following description.

### TECHNICAL SOLUTION

In accordance with one embodiment of the present disclosure, there is provided a method for obtaining an approximate signal for each of a plurality of target analytes performed by a computer device is disclosed. The method includes: obtaining, from a signal generation reaction of a plurality of cycles for a first target analyte and a second target analyte in a sample, a first data set measured at a first detection temperature at the plurality of cycles and a second data set measured at a second detection temperature at the plurality of cycles; wherein the first data set includes signal values at the first detection temperature dependent on the presence of the first target analyte in the sample, and the second data set includes signal values at the second detection temperature dependent on the presence of the first target analyte and the second target analyte in the sample; and wherein the signal generation reaction is performed by incubating the sample with a first signal generation composition for detecting the first target analyte in the sample and a second signal generation composition for detecting the second target analyte in the sample; wherein the first signal generation composition and the second signal generation composition include the same label, and wherein signals from the label are not differentiated for each target analyte by a single type of detector; and by at least partially using the first data set and the second data set, performing a joint-estimation on (a) a value of at least one approximate function parameter of at least one approximate function, wherein the at least one approximate function includes at least one selected from the group consisting of: an approximate function *1-1* for approximating signal values dependent on the presence of the first target analyte at the first detection temperature; an approximate function *1-2* for approximating signal values dependent on the presence of the first target analyte at the second detection temperature; and an approximate function *2* for approximating signal values dependent on the presence of the second target analyte at the second detection temperature; and (b) a value of a magnitude parameter, wherein the magnitude parameter indicates a relationship between the signal values dependent on the presence of the first target analyte at the first detection temperature and the signal values dependent on the presence of the first target analyte at the second detection temperature.

In an embodiment, wherein the performing the joint-estimation includes: performing the joint-estimation on a parameter value of a first parameter group including approximate function parameters of the approximate function *1-1* and the approximate function *2* and the magnitude parameter; wherein the magnitude parameter in the first parameter group indicates a ratio between the magnitude at the second detection temperature and the magnitude at the first detection temperature; or, performing the joint-estimation on a parameter value of a second parameter group including approximate function parameters of the approximate function *1-2* and the approximate function 2 and the magnitude parameter; wherein the magnitude parameter in the second parameter group indicates a ratio between the magnitude at the first detection temperature and the magnitude at the second detection temperature.

In an embodiment, wherein the performing the joint-estimation includes: estimating a value of an approximate function parameter *1-1* of the approximate function *1-1* by using the first data set; and performing the joint-estimation on a value of an approximate function parameter *2* of the approximate function 2 and the value of the magnitude parameter, by at least partially using the estimated value of the approximate function parameter *1-1* and the second data set.

In an embodiment, wherein the value of the approximate function *1-1* parameter is estimated in such a manner that a sum of differences between the approximate function *1-1* and the first data set at each of the plurality of cycles is minimized.

In an embodiment, wherein the performing the joint-estimation on the value of the approximate function parameter *2* and the value of the magnitude parameter includes: preparing a value of the approximate function *1-2,* by using the value of the magnitude parameter and the value of the approximate function *1-1* having the estimated value of the approximate function parameter *1-1;* calculating a difference between (a) the second data set and (b) a sum of the approximate function *2* and the approximate function *1-2* at each of the plurality of cycles; and performing the joint-estimation on the value of the approximate function parameter *2* and the value of the magnitude parameter in such a manner that a sum of the difference at each of the plurality of cycles is minimized.

In an embodiment, wherein the value of the approximate function parameter 2 and the value of the magnitude parameter are estimated based on a joint maximum likelihood estimation.

In an embodiment, wherein the performing the j oint-estimation includes: performing the joint-estimation on a value of an approximate function parameter *1-1* of the approximate function *1-1,* a value of an approximate function parameter 2 of the approximate function *2,* and the value of the magnitude parameter, in consideration of (a) a sum of a difference between the first data set and the approximate function *1-1* at each of the plurality of cycles and (b) a sum of a difference at each of the plurality of cycles between (b-1) the second data set and (b-2) the approximate function *2* and the approximate function *1-2.*

In an embodiment, wherein the value of the approximate function parameter *1-1,* the value of the approximate function parameter 2, and the value of the magnitude parameter are estimated based on a joint maximum likelihood estimation.

In an embodiment, wherein the performing the joint-estimation further includes: applying an arbitrary value or a pre-estimated value to one or two parameters among the approximate function parameter *1-1,* the approximate function parameter *2* and the magnitude parameter, and estimating values of the remaining parameters; and applying the estimated values of the remaining parameters and estimating values of the one or two parameters, wherein the estimating values of the remaining parameters and the estimating values of the one or two parameters are performed multiple times.

In an embodiment, wherein each of the first data set and the second data set is a raw data set obtained from a detection device that detects the first target analyte and the second target analyte in the sample; a mathematically transformed data set of the raw data set; a normalized data set of the raw data set; or a normalized data set of the mathematically transformed data set.

In an embodiment, wherein each of the approximate function *1-1,* the approximate function *1-2,* and the approximate function *2* include at least one selected from the group consisting of a sigmoid function, a logistic function, a Gompertz function, and a Chapman function.

In an embodiment, wherein the approximate function *1-1* has an approximate function parameter *1-1* and the approximate function *1-2* has an approximate function parameter *1-2;* each of the approximate function parameter *1-1* and the approximate function parameter *1-2* includes at least one of: a parameter indicating a first slope for the cycle of the signal values dependent on the presence of the first target analyte; a parameter indicating a cycle corresponding to a point at which the first slope is maximum; a parameter indicating signal values in a baseline region; and a parameter indicating signal values in a plateau region, and wherein the approximate function *2* has an approximate function parameter *2;* the approximate function parameter *2* includes at least one of: a parameter indicating a second slope for the cycle of signal values dependent on the presence of the second target analyte; a parameter indicating a cycle corresponding to a point at which the second slope is maximum; a parameter indicating signal values in a baseline region; and a parameter indicating signal values in a plateau region.

In an embodiment, wherein each of the approximate function *1-1,* the approximate function *1-2,* and the approximate function 2 further includes a parameter of a background signal approximate function for a background signal, wherein the parameter of the background signal approximate function in each of the approximate function *1-1* and the approximate function *1-2* includes at least one of: a parameter that approximates a slope of the cycle of a first background signal further included in the first data set; a parameter that approximates signal values in a baseline region; and a parameter that approximates a cycle corresponding to a point at which a magnitude of the first background signal is minimum; and wherein the parameter of the background signal approximate function of the approximate function *2* includes at least one of: a parameter that approximates a slope of the cycle of a second background signal further included in the second data set; a parameter that approximates signal values in a baseline region; and a parameter that approximates a cycle corresponding to a point at which a magnitude of the second background signal is minimum.

In an embodiment, wherein the parameter of the background signal approximate function is a parameter of a constant or a polynomial function.

In an embodiment, wherein the first detection temperature is greater than the second detection temperature.

In an embodiment, wherein the second detection temperature is greater than the first detection temperature.

In an embodiment, wherein the signal generation reaction involves a nucleic acid amplification.

In an embodiment, wherein the performing the j oint-estimation includes j oint-estimating the value of the magnitude parameter for each of two or more cycle sections selected from the plurality of cycles.

In an embodiment, wherein the performing the joint-estimation includes joint-estimating the value of the magnitude parameter for each of the plurality of cycles.

In an embodiment, wherein the performing the joint-estimation includes: estimating a value of an approximate function parameter *1-1* of the approximate function *1-1* by using the first data set; and performing the joint-estimation on a value of an approximate function parameter *2* of the second approximate function *2* and a value of the magnitude parameter, by using the first data set and the second data set.

In an embodiment, wherein the performing the joint-estimation on the value of the approximate function parameter *2* and the value of the magnitude parameter includes: calculating a difference between (a) the second data set; and (b) the first data set and the magnitude parameter; at each of the plurality of cycles, and performing the joint-estimation on the value of the approximate function parameter *2* and the value of the magnitude parameter in such a manner that a sum of the differences at each of the plurality of cycles is minimized.

In accordance with one embodiment of the present disclosure, there is provided a computer program stored in a computer-readable recording medium, programmed to perform each step included in the method.

In accordance with one embodiment of the present disclosure, there is provided a non-transitory computer-readable recording medium storing a computer program programmed to perform each step included in the method.

In accordance with one embodiment of the present disclosure, there is provided a computer device. The computer device includes: a memory configured to store at least one instruction; and a processor configured to execute the one or more instructions stored in the memory, wherein the instructions, when executed by the processor, cause the processor to: obtaining, from a signal generation reaction of a plurality of cycles for a first target analyte and a second target analyte in a sample, a first data set measured at a first detection temperature at the plurality of cycles and a second data set measured at a second detection temperature at the plurality of cycles; wherein the first data set includes signal values at the first detection temperature dependent on the presence of the first target analyte in the sample, and the second data set includes signal values at the second detection temperature dependent on the presence of the first target analyte and the second target analyte in the sample; and wherein the signal generation reaction is performed by incubating the sample with a first signal generation composition for detecting the first target analyte in the sample and a second signal generation composition for detecting the second target analyte in the sample; wherein the first signal generation composition and the second signal generation composition include the same label, and wherein signals from the label are not differentiated for each target analyte by a single type of detector; and by at least partially using the first data set and the second data set, performing a joint-estimation on (a) a value of at least one approximate function parameter of at least one approximate function, wherein the at least one approximate function includes at least one selected from the group consisting of: an approximate function *1-1* for approximating signal values dependent on the presence of the first target analyte at the first detection temperature; an approximate function *1-2* for approximating signal values dependent on the presence of the first target analyte at the second detection temperature; and an approximate function *2* for approximating signal values dependent on the presence of the second target analyte at the second detection temperature; and (b) a value of a magnitude parameter, wherein the magnitude parameter indicates a relationship between the signal values dependent on the presence of the first target analyte at the first detection temperature and the signal values dependent on the presence of the first target analyte at the second detection temperature.

In accordance with one embodiment of the present disclosure, there is provided a method for obtaining an approximate signal for each of a plurality of target analytes performed by a computer device. The method includes: obtaining, from a signal generation reaction of a plurality of cycles for a first target analyte and a second target analyte in a sample, a first data set measured at a first detection temperature at the plurality of cycles and a second data set measured at a second detection temperature at the plurality of cycles; wherein the first data set includes signal values at the first detection temperature dependent on the presence of the first target analyte in the sample, and the second data set includes signal values at the second detection temperature dependent on the presence of the first target analyte and the second target analyte in the sample; and wherein the signal generation reaction is performed by incubating the sample with a first signal generation composition for detecting the first target analyte in the sample and a second signal generation composition for detecting the second target analyte in the sample; wherein the first signal generation composition and the second signal generation composition include the same label, and wherein signals from the label are not differentiated for each target analyte by a single type of detector; and by at least partially using the first data set and the second data set, performing a joint-estimation on (a) a value of at least one approximate function parameter of at least one approximate function, wherein the at least one approximate function includes at least one selected from the group consisting of: an approximate function *1-1* for approximating signal values dependent on the presence of the first target analyte at the first detection temperature; an approximate function *1-2* for approximating signal values dependent on the presence of the first target analyte at the second detection temperature; and an approximate function *2* for approximating signal values dependent on the presence of the second target analyte at the second detection temperature; and (b) a value of a magnitude parameter, wherein the magnitude parameter indicates a relationship between the signal values dependent on the presence of the first target analyte at the first detection temperature and the signal values dependent on the presence of the first target analyte at the second detection temperature; and detecting each of the first target analyte and the second target analyte in the sample by using the j oint-estimation result.

In accordance with one embodiment of the present disclosure, there is provided a method for obtaining an approximate signal for each of a plurality of target analytes performed by a computer device. The method includes: obtaining, from a signal generation reaction of a plurality of cycles for a first target analyte, a second target analyte and a third target analyte in a sample, a first data set measured at a first detection temperature at each of the plurality of cycles, a second data set measured at a second detection temperature at each of the plurality of cycles and a third data set measured at a third detection temperature at each of the plurality of cycles; wherein the first data set includes signal values at the first detection temperature dependent on the presence of the first target analyte in the sample, the second data set includes signal values at the second detection temperature dependent on the presence of the first target analyte and the second target analyte in the sample, and the third data set includes signal values at the third detection temperature dependent on the presence of the first target analyte, the second target analyte and the third target analyte in the sample; wherein the signal generation reaction is performed by incubating the sample with a first signal generation composition for detecting the first target analyte in the sample, a second signal generation composition for detecting the second target analyte in the sample and a third signal generation composition for detecting the third target analyte in the sample; wherein the first signal generation composition, the second signal generation composition and the third signal generation composition include the same label, and wherein signals from the label are not differentiated for each target analyte by a single type of detector; and by at least partially using the first data set to the third data set, performing a joint-estimation on (a) a value of at least one approximate function parameter of at least one approximate function, wherein the at least one approximate function includes at least one selected from the group consisting of: an approximate function *1-1* for approximating signal values dependent on the presence of the first target analyte at the first detection temperature; an approximate function *1-2* for approximating signal values dependent on the presence of the first target analyte at the second detection temperature; an approximate function *1-3* for approximating signal values dependent on the presence of the first target analyte at the third detection temperature; an approximate function *2-2* for approximating signal values dependent on the presence of the second target analyte at the second detection temperature; an approximate function *2-3* for approximating signal values dependent on the presence of the second target analyte at the third detection temperature; and an approximate function *3-3* for approximating signal values dependent on the presence of the third target analyte at the third detection temperature; and (b) a value of at least one magnitude parameter, wherein the at least one magnitude parameter is at least one of a plurality of magnitude parameters indicate a relationship between signal values dependent on the presence of at least one target analyte among the target analytes at any one detection temperature among from the first detection temperature to the third detection temperature and signal values dependent on the presence of the at least one target analyte at another one detection temperature among from the first detection temperature to the third detection temperature.

In an embodiment, wherein the plurality of magnitude parameters include at least one selected from the group consisting: a first magnitude parameter indicates a relationship between signal values dependent on the presence of the first target analyte at the first detection temperature and signal values dependent on the presence of the first target analyte at the second detection temperature; a second magnitude parameter indicates a relationship between signal values dependent on the presence of the first target analyte at the second detection temperature and signal values dependent on the presence of the first target analyte at the third detection temperature; a third magnitude parameter indicates a relationship between signal values dependent on the presence of the second target analyte at the second detection temperature and signal values dependent on the presence of the second target analyte at the third detection temperature; and a fourth magnitude parameter indicates a relationship between signal values dependent on the presence of the first target analyte at the first detection temperature and signal values dependent on the presence of the first target analyte at the third detection temperature.

**EFFECT OF INVENTION**

According to one embodiment of the present disclosure, for a magnitude parameter representing a magnitude relationship of the signal value dependent on the presence of a specific target analyte at each of the two detection temperatures, an optimized value may be estimated for each reaction unit for the corresponding signal generation reaction. As described above, the relationship between signals at two detection temperatures generated in the presence of a specific target analyte may be expressed as a term called the reference value. In the related art, a method of experimentally or statistically measuring such the reference value according to a predetermined procedure before analyzing a signal generation reaction for an arbitrary sample, and applying this previously obtained reference value to the process of extracting the signal value for each target analyte in the process of analyzing the signal generation reaction for the arbitrary sample has been used. However, the related art has a limitation in that a difference generated according to various detection environments such as a reaction well, a detection device, and the like for each reaction may not be reflected, and accordingly, there is a problem in that signal distortion occurs as the reference value is over or under applied, and as a result, detection accuracy of each target analyte decreases. However, according to an embodiment of the present disclosure, the value of the magnitude parameter optimized for the corresponding reaction is estimated for each reaction in which the signal generation reaction for the sample is performed, and applied to the extraction of the signal value. Accordingly, the signal value of each target analyte may be more accurately extracted, and as a result, the detection accuracy of each target analyte may be improved.

In addition, a value of a parameter of an approximate function that approximates signal values generated under the presence of each target analyte from the data sets may be estimated together with the magnitude parameter. These parameters have a correlation that affects each other for the overall error. Among the magnitude parameter and the parameters of each approximate function, one parameter depends on the other parameter, and vice versa. Accordingly, rather than estimating a value in consideration of only one parameter, the optimized values of the parameters are joint-estimated in consideration of the correlation between the parameters together, thereby minimizing the overall error.

In addition, in the related art, there is a disadvantage in that several processes such as a process of obtaining the reference value in advance, a process of extracting signal values corresponding to each target analyte, and a fitting process of an amplification curve function corresponding to the extracted signal value are required. However, according to an embodiment of the present disclosure, as described above, as each approximate function and the value of the magnitude parameter are estimated together by fitting, several processes which have been conventionally required may be integrated into one fitting process, and thus efficient detection in terms of time and cost may be possible.

In addition, in the related art, as the previously obtained reference value is over applied in a specific case, there is a problem in that signal distortion is greatly generated in the signal value extraction process, causing a false-positive possibility. However, according to an embodiment of the present disclosure, the problem caused by such an excessive reference value may be solved, and from this, a more accurate positive/negative decision result may be provided.

According to an additional embodiment, the value of the magnitude parameter may be estimated for each of a plurality of cycles. Accordingly, a more optimized value for the magnitude parameter may be applied for each cycle for each reaction, and the detection accuracy of each target analyte may be further improved than a case to which a single value is applied.

According to an additional embodiment, a value of a parameter of a background signal approximate function that approximates signal values that is independent of the presence of the target analyte may be joint-estimated together. For example, a parameter value of an approximate function approximating a background signal, which has been difficult to analyze in the related art, may be estimated together with the above-described parameters, from a raw data set having no artificial signal distortion caused by a signal analysis process. In addition, by adding functions for various background elements to a model for joint-estimation through subdivided modeling of the background signal, there is an advantage in that it is easy to expand and manage parameters that are subject to the joint-estimation.

The effects of the present disclosure are not limited to those mentioned above and should be understood to include all effects that can be inferred from the detailed description of the disclosure or the configuration of the invention described in the claims.

## BRIEF DESCRIPTION OF THE DRAWINGS

FIG. 1 is a block diagram illustrating a target analyte detection system according to an embodiment.

FIGS. 2A and 2B exemplarily illustrate data sets used in a computer device according to an embodiment and signal values dependent on the first target analyte and signal values dependent on the second target analyte, which are estimated from the data sets.

FIG. 3 schematically shows a block diagram of the computer device according to an embodiment.

FIGS. 4A and 4B illustrate the concept or definition of a sigmoid function and parameters representing the characteristics of the sigmoid function according to an embodiment.

FIG. 5 is a flowchart exemplarily illustrating a method for performing the joint-estimation on the value of the approximate function parameter *2* and the value of the magnitude parameter through the processor by the computer device according to a first embodiment.

FIG. 6 is a flowchart illustrating a detailed method in which the computer device according to a second embodiment performs the joint-estimation on the value of the approximate function parameter *1-1,* the value of the approximate function parameter *2,* and the value of the magnitude parameter through the processor.

FIGS. 7A and 7B exemplarily illustrate the data set including the background signal used in the computer device according to another embodiment and signal values dependent on the first target analyte and signal values dependent on the second target analyte, which are estimated from the data set.

FIG. 8 is a flowchart illustrating a method in which the processor performs the joint-estimation on the value of the approximate function parameter *2* and the value of the magnitude parameter in consideration of the background signal, according to a third embodiment.

FIG. 9 is a flowchart illustrating an example of a method in which the processor performs the joint-estimation on the value of the approximate function parameter *1-1,* the value of the approximate function parameter *2,* and the value of the magnitude parameter in consideration of the background signal, according to a fourth embodiment.

FIGS. 10A and 10D illustrate simulation results according to the third embodiment in which the background signal is considered as a constant.

FIGS. 11A and 11C illustrate simulation results according to the third embodiment in which the background signal is considered as a first-order function.

FIGS. 12A and 12C illustrate simulation results according to the third embodiment in which the background signal is considered as a second-order function.

FIG. 13 is a flowchart exemplarily illustrating a method for obtaining the approximate signal for each of the plurality of target analytes, performed by the computer device according to an embodiment.

FIGS. 14A and 14D illustrate simulation results according to another embodiment in which the values of the magnitude parameter are estimated for each of the plurality of cycles.

FIGS. 15A and 15B illustrate the first to third data set and signal values on the presence of each target analyte at each temperature dependent, which are joint-estimated from the data sets.

FIGS. 16A and 16F illustrate simulation results according to an embodiment in which the joint-estimation by using the first to third data set in which the background signal is considered is performed.

FIG. 17 is a flowchart exemplarily illustrating a method for obtaining the approximate signal for each of the plurality of target analytes, performed by the computer device according to an embodiment.

## MODE FOR CARRYING OUT THE INVENTION

Advantages and features of the disclosure, and methods for achieving same may be apparent from the embodiments described below with reference to the accompanying drawings. However, the disclosure is not limited to the embodiments disclosed herein, and various changes may be made thereto. The embodiments disclosed herein are provided only to inform one of ordinary skilled in the art of the category of the disclosure. The disclosure is defined only by the appended claims.

When determined to make the subject matter of the disclosure unclear, the detailed description of known functions or configurations may be skipped. The terms described below are defined considering the functions in embodiments of the disclosure and may be replaced with other terms according to the intention or practice of the user or operator. Therefore, the terms should be defined based on the overall disclosure.

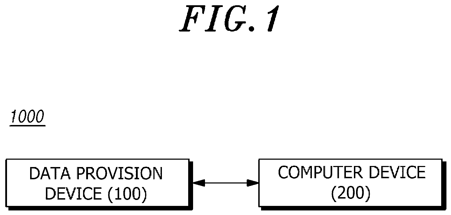

Prior to describing FIG. 1, terms used herein are described.

In the disclosure, the term "target analyte" may refer a variety of substances (e.g., biological and non-biological substances). Specifically, the target analyte may include a biological substance, more specifically at least one of nucleic acid molecules (e.g., DNA and RNA), proteins, peptides, carbohydrates, lipids, amino acids, biological compounds, hormones, antibodies, antigens, metabolites, and cells.

The term "sample" may refer to a biological sample (e.g., cells, tissues, and body fluids) and a non-biological sample (e.g., food, water, and soil). The biological sample may include at least one of, for example, virus, bacteria, tissue, cells, blood (including whole blood, plasma, and serum), lymph, bone marrow, saliva, sputum, swab, aspiration, milk, urine, stool, eye fluid, semen, brain extract, spinal fluid, joint fluid, thymus fluid, bronchial lavage fluid, ascites, and amniotic fluid. Such a sample may or may not contain the above-described target analyte.

Meanwhile, when the above-described target analyte is or includes a nucleic acid molecule, a nucleic acid extraction process as known in the art may be performed on the sample estimated to contain the target analyte. (See Sambrook, J. et al., Molecular Cloning. A Laboratory Manual, 3rd ed. Cold Spring Harbor Press(2001)). The nucleic acid extraction process may vary depending on the type of sample. Further, when the extracted nucleic acid is RNA, a reverse transcription process may be additionally performed to synthesize cDNA (See Sambrook, J. et al., Molecular Cloning. A Laboratory Manual, 3rd ed. Cold Spring Harbor Press(2001).

The term "data set" refers to the data obtained from a signal generation reaction on the target analyte using a signal generation composition (the signal generation composition is described below).

In the case, the term "signal generation reaction" refers to a reaction that generates a signal depending on the properties of the target analyte in the sample, such as activity, amount or presence/absence (or absence), specifically presence/absence (or absence). The signal generation reactions include biological reactions and chemical reactions. Among them, the biological reactions include genetic analysis processes, such as PCR, real-time PCR and microarray analysis, immunological analysis processes, and bacterial growth analysis. Further, the chemical reactions include the process of analyzing the generation, change or destruction of chemicals.

According to an embodiment, the signal generation reaction may be the genetic analysis process, or may be a nucleic acid amplification reaction such as the PCR or isothermal amplification reaction, an enzymatic reaction, or microbial growth. In an amplification reaction, amplification of a target analyte (e.g., a nucleic acid molecule) may or may not be accompanied. More specifically, the amplification reaction may mean an amplification reaction of a signal accompanied by amplification of a target analyte.

The above-described signal generation reactions are accompanied by signal changes. Therefore, the progress of the signal generation reaction may be evaluated by measuring the change in signal.

The signal generation reaction may denote a reaction in which a signal appears or a reaction in which a signal disappears. The signal change may include an increase in signal or a decrease in signal.

Here, the term "signal" means a measurable output. Further, the measured magnitude or change in the signal serves as an indicator qualitatively or quantitatively indicating the properties of the target analyte, specifically, the presence/absence or absence of the target analyte in the sample.

Examples of the indicator include fluorescence intensity, luminescence intensity, chemiluminescence intensity, bioluminescence intensity, phosphorescence intensity, charge transfer, voltage, current, power, energy, temperature, viscosity, light scatter, radioactivity intensity, reflectance, transmittance and absorbance, but are not limited thereto.

The term "signal generation composition" as described above means a means for providing a signal indicative of the properties of the target analyte to be analyzed, specifically the presence/absence or absence of the target analyte. The signal generation composition includes a label itself or a label-attached oligonucleotide.

Among them, the label includes a fluorescent label, a luminescent label, a chemiluminescent label, an electrochemical label, and a metal label. The label may be used as a label itself, such as an intercalating dye. Or, the label may be used as a single label or interactive dual label including a donor molecule and an acceptor molecule, in the form attached to one or more oligonucleotides.

The signal generation composition may include an enzyme with nucleolytic activity to generate signals (e.g., an enzyme with 5' nucleolytic activity or an enzyme with 3' nucleolytic activity).

Meanwhile, various methods are known for generating a signal indicating the presence/absence of a target analyte, particularly a target nucleic acid molecule, by the signal generation composition. Representative examples may include: TaqManTM probe method (U.S. Patent No. 5,210,015), molecule beacon method (Tyagi, Nature Biotechnology v.14 MARCH 1996), Scorpion method (Whitcombe et al., Nature Biotechnology 17:804-807(1999)), Sunrise or Amplifluor method (Nazarenko et al., Nucleic Acids Research, 25(12):2516-2521(1997), and U.S. Patent No. 6,117,635), Lux method (U.S. Patent No. 7,537,886), CPT(Duck P, et al. Biotechniques, 9:142-148(1990)), LNA method (U.S. Patent No. 6,977,295), Plexor method (Sherrill CB, et al., Journal of the American Chemical Society, 126:4550-4556(2004)), Hybeacons (D. J. French, et al., Molecular and Cellular Probes 13:363-374(2001) and U.S. Patent No. 7,348,141), Dual-labeled, self-quenched probe; U.S. Patent No. 5,876,930), hybridization probe (Bernard PS, et al., Clin Chem 2000, 46, 147-148), PTOCE(PTO cleavage and extension) method (WO 2012/096523), PCE-SH(PTO Cleavage and Extension-Dependent Signaling Oligonucleotide Hybridization) method (WO 2013/115442), PCE-NH(PTO Cleavage and Extension-Dependent Non-Hybridization) method (PCT/KR2013/012312) and CER method (WO 2011/037306).

The above-described term "signal generation reaction" may include a signal amplification reaction. In this case, the term "amplification reaction" means a reaction that increases or decreases the signal generated by the signal generation composition. Specifically, the amplification reaction refers to a reaction for increasing (or amplifying) the signal generated by the signal generation composition depending on the presence/absence of the target analyte.

The amplification reaction may or may not be accompanied by amplification of the target analyte (e.g., nucleic acid molecule). More specifically, the amplification reaction may refer to a signal amplification reaction accompanied by amplification of the target analyte.

Meanwhile, the data set obtained through the amplification reaction may include an amplification cycle.

The term "cycle" refers to a unit of change in a predetermined condition in a plurality of measurements accompanied by the change in the condition. For example, the change in the predetermined condition means an increase or decrease in temperature, reaction time, number of reactions, concentration, pH, or number of copies of the measurement target (e.g., nucleic acid). Thus, the cycle may be a time or process cycle, unit operation cycle, or reproductive cycle.

More specifically, the term "cycle" means one unit of repetition when a reaction of a predetermined process is repeated or a reaction is repeated based on a predetermined time interval. For example, in the case of a nucleic acid amplification reaction, one cycle means a reaction including denaturation of nucleic acids, annealing of primers, and extension of primers. In this case, the change in the predetermined condition is an increase in the number of repetitions of the reaction, and a repeating unit of the reaction including the above series of steps is set as one cycle.

Meanwhile, the amplification reaction for amplifying the signal indicating the presence/absence of the target analyte may be performed in a manner in which the signal is also amplified while the target is amplified (e.g., real-time PCR method). Alternatively, according to an embodiment, the amplification reaction may be performed in a manner in which only a signal indicating the presence/absence of the target is amplified without amplifying the target analyte (e.g., CPT method (Duck P, et al., Biotechniques, 9:142-148(1990)), Invader assay (U.S.Patent Nos. 6,358,691 and 6,194,149)).

Meanwhile, the above-described target analyte, particularly target nucleic acid molecules, may be amplified by various methods: polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA)) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)) and Q-beta Replicase (Lizardi et al., BiolTechnology 6:1197(1988)), loop-mediated isothermal amplication(LAMP, Y. Mori, H. Kanda and T. Notomi, J. Infect. Chemother., 2013, 19, 404-411), recombinase polymerase amplication(RPA, J. Li, J. Macdonald and F. von Stetten, Analyst, 2018, 144, 31-67).

The amplification reaction amplifies the signal while accompanying the amplification of the target (specifically, the target nucleic acid molecule). For example, the amplification reaction is carried out by PCR, specifically real-time PCR, or isothermal amplification reaction (e.g., LAMP or RPA).

Meanwhile, a data set obtained by the signal generation reaction includes a plurality of data points including cycles of the signal generation reaction and signal values at the cycles.

Here, the term "signal value" refers to a value(s) obtained by quantifying the signal level (e.g., signal intensity) actually measured in a cycle(s) of a signal generation reaction(s), in particular, an amplification reaction, according to a predetermined scale, or a modified value thereof. The modified value may mean a mathematically processed signal value of the actually measured signal value. Examples of mathematically processed signal values of actually measured signal values (i.e., signal values of raw data sets) may denote logarithmic values or derivatives.

The term "data point" means a coordinate value that includes signal values and cycles. As used herein, the term "data" refers to all information that constitutes a data set. For example, each cycle and signal value of the amplification reaction may be data.

Data points obtained from the signal generation reaction, specifically the amplification reaction, may be represented by coordinate values on a Cartesian coordinate system. In the Cartesian coordinate system, the X axis represents the cycles of the amplification reaction and the Y axis represents the signal value measured in each cycle or the processed value of the signal value.

The term "data set" refers to a collection of the data points. For example, the data set may be a collection of data points obtained directly through an amplification reaction performed under the presence of the signal generation composition or may be a modified data set of such data sets. The data set may be some or all of a plurality of data points obtained by an amplification reaction or modified data points thereof.

The data set may include a data set obtained by processing a plurality of data sets. When an analysis of a plurality of target analytes is performed in one reaction well, data sets for the plurality of target analytes may be obtained through processing of data sets obtained from reactions performed in the one reaction well. For example, a data set for a plurality of target analytes made in one reaction well may be obtained by processing a plurality of data sets obtained from signals measured at different temperatures. For example, a method of detecting signals generated at different detection temperatures using a single type of detector in order to detect two target nucleic acid sequences in a sample is known (see Korean Patent No. 10-2050601).

The data set may be obtained from a signal generation reaction of a plurality of cycles for at least two target analytes in a sample. Here, the at least two target analytes may include two target analytes different from each other, such as a first target analyte and a second target analyte, or may include three or more target analytes different from each other according to an embodiment.

In addition, the data sets may be measured at least two temperatures. Here, the at least two temperatures may include two different temperatures such as a first temperature and a second temperature, or may include three or more different temperatures according to an embodiment.

Among the data sets described above, a first data set refers to a data set obtained from a first signal generation reaction of a plurality of cycles by a first signal generation composition for detecting a first target analyte in a sample, specifically, a data set measured at a first temperature at each of the plurality of cycles. In addition, a second data set refers to a data set obtained from a second signal generation reaction of the plurality of cycles by a second signal generation composition for detecting a second target analyte in the sample, specifically, a data set measured at a second temperature at each of the plurality of cycles. In addition, according to an embodiment, the above-described data sets may further include a m-th data set refers to a data set obtained from a m-th signal generation reaction of the plurality of cycles by a m-th signal generation composition for detecting a m-th target analyte in the sample, specifically, a data set measured at a m-th temperature at each of the plurality of cycles. Here, the m-th temperature indicates the detection temperature of the m-th target analyte. As such, it can be seen that, depending on the embodiment, the above-described data sets may further include one or more additional data sets measured at different temperatures for different target analytes.

The signal generation reaction may be performed by incubating the sample with the first signal generation composition for detecting the first target analyte and the second signal generation composition for detecting the second target analyte in one reaction well. Here, the first temperature is a detection temperature of the first target analyte, and the second temperature is a detection temperature of the second target analyte. For example, when the first target analyte is present in the sample, signal values dependent on the first target analyte may be generated at each of the first temperature and the second temperature by the first signal generation reaction. In addition, when the second target analyte is present in the sample, signal values dependent on the second target analyte may be generated at the second temperature by the second signal generation reaction. Accordingly, when both the first target analyte and the second target analyte are present in the sample, the first data set may include signal values at the first temperature dependent on the first target analyte, and the second data set may include signal values at the second temperature dependent on the first target analyte and signal values at the second temperature dependent on the second target analyte. Throughout the specification, this "temperature" and "detection temperature" may be used interchangeably.

According to an embodiment, the signal generation reaction may be performed by incubating the sample with the first to m-th signal generation compositions. For example, in a case in which m is 3, when the first target analyte is present in the sample, signal values dependent on the first target analyte may be generated at each of the first temperature, the second temperature, and a third temperature by the first signal generation reaction. In addition, when the second target analyte is present in the sample, signal values dependent on the second target analyte may be generated at the second temperature and the third temperature by the second signal generation reaction. In addition, when a third target analyte is present in the sample, signal values dependent on the third target analyte may be generated at a third temperature by a third signal generation reaction. Accordingly, when all of the first target analyte to the third target analyte are present in the sample, the first data set may include signal values at the first temperature dependent on the first target analyte, the second data set may include signal values at the second temperature dependent on the first target analyte and signal values at the second temperature dependent on the second target analyte, and the third data set may include signal values at the third temperature dependent on the first target analyte, signal values at the third temperature dependent on the second target analyte, and signal values at the third temperature dependent on the third target analyte.

The first signal generation composition and the second signal generation composition include the same label, and signals from the label may not be differentiated for each target analyte by a single type of detector. In addition, according to an embodiment, the m-th signal generation composition may also include the same label as the first signal generation composition and the second signal generation composition. Here, not being differentiated for each target analyte by the single type of detector means that signals are indistinguishable from each other by the single type of detector due to their same or substantially the same signal characteristics (e.g., optical characteristics, emission wavelengths, and electrical signals). For example, when the same label (e.g., FAM) is used for the detection of two target analytes and a single type of detector is used to detect the emission wavelength from the FAM, the signals are not detected in a distinguishable manner.

The first temperature and the second temperature are temperatures at which the signal generation composition emits a signal. The signal generated at the first temperature may include a fluorescence signal (e.g., relative fluorescence unit (RFU)) generated depending on the presence or absence of the first target analyte in the sample. The signal generated at the second temperature may include a fluorescence signal generated depending on the presence or absence of the first target analyte in the sample and a fluorescence signal generated depending on the presence or absence of the second target analyte in the sample.

In an embodiment, the first temperature and the second temperature may be selected from a temperature in a range of 75°C to 40°C. For example, each of the first temperature and the second temperature may be any one of 72°C, 65°C, 60°C, 55°C, and 50°C, and the first temperature and the second temperature may be different from each other.

In an embodiment, the first temperature may be greater than the second temperature. For example, the first temperature may be 72°C, and the second temperature may be 60°C. In another embodiment, the second temperature may be greater than the first temperature. For example, the second temperature may be 72°C, and the first temperature may be 60°C.

Additionally, the third temperature different from the first temperature and the second temperature described above may also be assumed. For example, the first temperature, the second temperature, and the third temperature may be selected at a temperature in the range of 90°C to 40°C. As an example, each may be any one of 85°C, 83°C, 75°C, 72°C, 65°C, 60°C, 55°C, and 50°C, and the first to third temperatures may be different temperatures.

In an embodiment, the first temperature may be greater than the second temperature, and the second temperature may be greater than the third temperature. For example, the first temperature may be about 83°C, the second temperature may be about 72°C, and the third temperature may be about 60°C. In another embodiment, the first temperature may be less than the second temperature, and the second temperature may be less than the third temperature. For example, the third temperature may be 83°C, the second temperature may be 72°C, and the first temperature may be 60°C. According to an embodiment, one or more m-th temperatures described above may be considered, and may be selected as a temperature different from the first temperature and the second temperature, respectively, within a temperature range of, for example, 90°C to 40°C.

Throughout the following specification, an embodiment of estimating parameters used to obtain an approximate signal for each of the first target analyte and the second target analyte has been described, but it can be seen that an embodiment of estimating parameters used to obtain an approximate signal for each of three or more target analytes in the similar manner may be modified and implemented.

In an embodiment, the data set may be a raw data set obtained from the detection device, a mathematically transformed data set of the raw data set, and normalized data of the raw data set, or a standardized data set of the mathematically transformed data set.

Here, the raw data set refers to a data set including signal values obtained directly from a signal generating reaction. For example, the raw data set includes a set of signals obtained from the data provision device 100 (e.g., a detecting device), subjected to basic signal processing on the data provision device 100, and then passed to the signal analysis step.

In addition, the mathematically processed data set refers to a data set transformed through mathematical processing from the raw data set. For example, the mathematically processed data set is a baseline subtracted data set obtained by removing at least some background signal from the raw data set. Baseline subtracted data sets can be obtained by various methods known in the art (e.g., US Pat. No. 8,560,247).

Further, the term "normalization" refers to a process of reducing or eliminating signal deviations between data sets for a plurality of responses. In addition, the standardization is an aspect of correction or adjustment that corrects or modifies data (especially signal values) of a data set for analysis purposes.

In an embodiment, the data set for the target analyte includes not greater than 200, not greater than 150, not greater than 100, not greater than 80, not greater than 60, not greater than 50, not greater than 45, not greater than 40, and not greater than 30 data points.

In an embodiment, the data set for the target analyte includes not less than 2, not less than 5, not less than 10, not less than 15 and not less than 20 data points.

The term "magnitude parameter" describes a magnitude relationship of signal values at different detection temperatures when two or more signal values are measured at two or more different detection temperatures. In an embodiment, the different detection temperatures may be the first temperature and the second temperature, and the magnitude parameter means a parameter that represents a relationship between (a) the magnitude of signal values dependent on the presence of a specific target analyte at the first temperature and (b) the magnitude of signal values dependent on the presence of the specific target analyte at the second temperature. For example, the magnitude parameter is one or more values for reflecting a pattern (rule) of signal value change dependent on a specific target analyte in the first temperature and the second temperature, and may refer to a parameter used for converting, transferring, adjusting, or modifying signal values detected at the first temperature to signal values at the second temperature.

In an embodiment, the magnitude parameter may have one value. For example, the magnitude parameter may be expressed in the form of a constant indicating the magnitude relationship of signal values dependent on the specific target analyte (e.g., the first target analyte) at the first temperature and the second temperature.

In another embodiment, the magnitude parameter may have two or more values. For example, the magnitude parameter may be expressed in the form of a set of two or more constant values representing a magnitude relationship of signal values dependent on the specific target analyte (e.g., the first target analyte) at the first temperature and the second temperature in one or more of the plurality of cycles. For another example, a magnitude approximate function indicating the magnitude relationship of signal values dependent on the specific target analyte (e.g., the first target analyte) at the first temperature and the second temperature is defined in the form of a polynomial function (e.g., a first-order function, a second-order function) having the cycle as an independent variable, and the magnitude parameter may be one or more parameters (e.g., coefficients of the first-order function) of the magnitude approximate function.

Related to the magnitude parameter described above, the first temperature and the second temperature are mainly described in the specification, but the present invention is not limited thereto. For example, as described above, when the third temperature is assumed, the magnitude parameter may define not only a magnitude relationship between the first temperature and the second temperature but also a magnitude relationship between the first temperature and the third temperature and a magnitude relationship between the second temperature and the third temperature for each target analyte. However, throughout the specification, for convenience of description, embodiments of the magnitude parameter for the first temperature and the second temperature are mainly described, and these embodiments may be equally applied to a case to which the m-th temperature is applied, such as the magnitude parameter for the first temperature and the third temperature and the magnitude parameter for the second temperature and the third temperature. Examples of this magnitude parameter will be described later.

The above-described data set may be plotted, by which an amplification curve may be obtained.

Meanwhile, the term "difference" may mean a difference in magnitude between two elements to be compared, or may mean an error corresponding to the difference in magnitude. For example, the difference between A and B may mean $(A-B)$, $(B-A)$, $(A/B)$, $(B/A)$, $|A-B|$, $(A-B)^2$, and a root value for $(A-B)^2$, or may mean an error based on loss function (e.g., mean squared error, root mean squared error, etc.) for a data set.

Hereinafter, various embodiments of the present disclosure will be described with reference to the drawings.

FIG. 1 is a block diagram illustrating a target analyte detection system 1000 according to an embodiment.

Referring to FIG. 1, the target analyte detection system 1000 may include at least one of a data provision device 100 and a computer device 200. However, the target analyte detection system 1000 is not limited to that shown in FIG. 1.

For example, the target analyte detection system 1000 may include at least two data provision devices 100 or at least two computer devices 200.

The data provision device 100 may obtain a data set from a signal generation reaction of the plurality of cycles for the first target analyte and the second target analyte in a sample, and directly or indirectly provide the obtained data set to the computer device 200. As described above, the data set may include the first data set measured at the first temperature and the second data set measured at the second temperature by the signal generation reaction. As described above, the third data set measured at the third temperature may be included in the data set.

In an embodiment, the data provision device 100 may include a detection device (e.g., a nucleic acid amplification device) implemented to detect the first target analyte and the second target analyte in the sample. For example, the data provision device 100 may directly obtain the data set by controlling the environment (temperature, etc.) of the reaction well in which the sample is accommodated so that the signal generation reaction is performed, and may directly or indirectly provide the obtained data set to the computer device 200. In another embodiment, the data provision device 100 may include the detection device and a computer implemented to execute a program that processes and provides a detection result (e.g., a nucleic acid amplification result) generated by the detection device.

The computer device 200 may be implemented to obtain an approximate signal for each of a plurality of target analytes. Here, the process of obtaining the approximate signal for each of the plurality of target analytes may include not only a process of separating or extracting signal values dependent on each of two or more target analytes in the sample from the data set, but also a process of approximating the separated or extracted signal value. The separation or extraction process and the approximation process will be described in detail later through an embodiment.

Here, a predetermined parameter is used in the process of separating or extracting the signal value, and the above-described computer device 200 may estimate or set a value of the parameter as an optimal value for each reaction well in which the sample is accommodated. A process of estimating or setting the value of the parameter will be described in detail later through an embodiment.

In an embodiment, the computer device 200 may obtain the data set from the data provision device 100. For example, the computer device 200 may be electrically connected to the data provision device 100 or through a network, and may obtain the data set from the data provision device 100. Here, the network may be configured through various communication networks such as wired and wireless networks, and for example, may be configured as various communication networks such as a Local Area Network (LAN), a Metropolitan Area Network (MAN), and a Wide Area Network (WAN).

In an embodiment, the computer device 200 may be implemented as a computer operating through a computer program designed to realize the functions described in the present specification. In an embodiment, the computer device 200 may be implemented to include the data provision device 100, or the following functions performed by the computer device 200 may be implemented to be included in the data provision device 100. For example, the following functions may be mounted on the nucleic acid amplification device, and may be implemented as software in a computer in which a program for providing a nucleic acid amplification result generated by the nucleic acid amplification device is executed.

Meanwhile, the target analyte detection system 1000 may further include other components in addition to the components shown in FIG. 1. Alternatively, one of the components illustrated in FIG. 1 may be omitted.

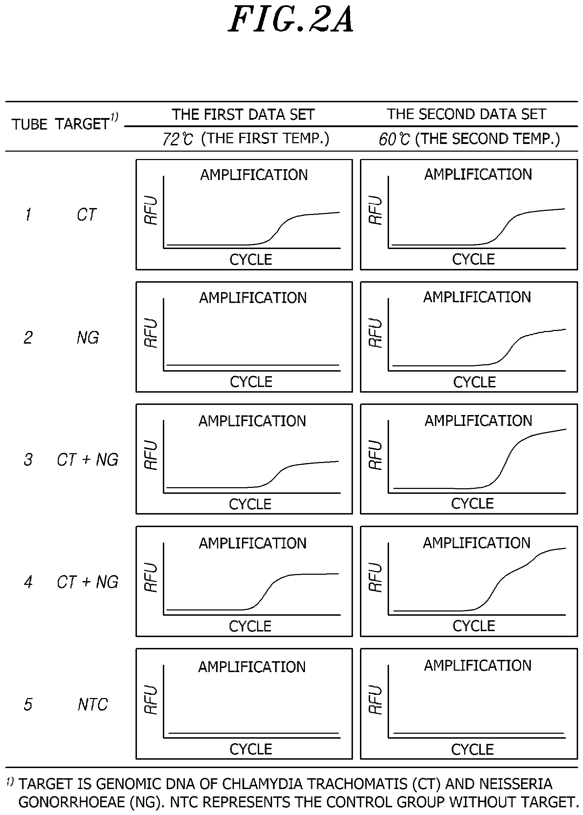

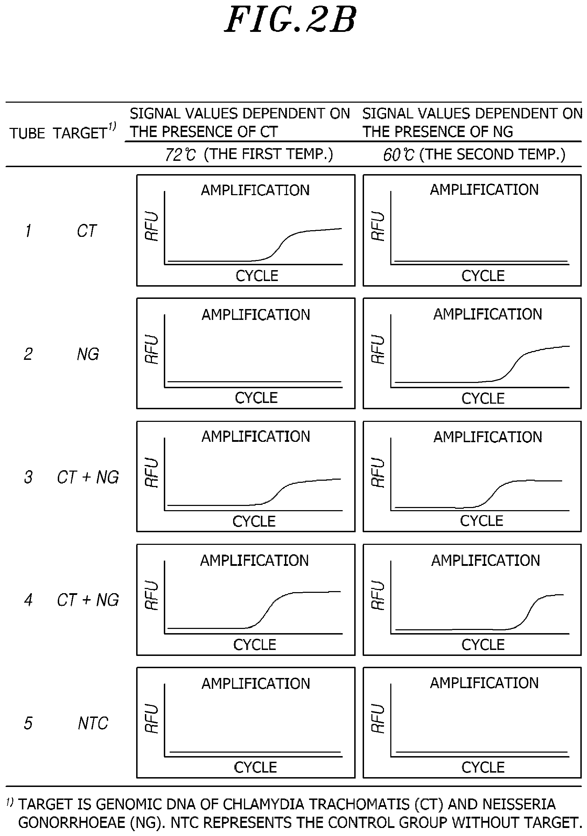

Hereinafter, various examples of the data set used in the computer device 200 according to an embodiment and signal values dependent on each of two or more target analytes estimated from the data set will be described first with reference to FIGS. 2A and 2B.

In FIG. 2A, the first data set and the second data set are exemplarily illustrated. Referring to FIG. 2A, the first temperature and the second temperature are 72°C and 60°C., respectively. The first target analyte and the second target analyte are genome DNA of Chlamydia trachomatis (CT) and Neisseria gonorrhoeae (NG), respectively. It should be clear that these kinds of temperature and target analytes are merely examples.

Referring to FIG. 2A, as described above, the first data set measured at the first temperature (e.g., 72°C.) may include signal values dependent on the presence of the first target analyte among the first target analyte (e.g., CT) and the second target analyte (e.g., NG). The second data set measured at the second temperature (e.g., 60°C.) may include both signal values dependent on the presence of the first target analyte and signal values dependent on the presence of the second target analyte.

Specifically, in the case of tube 1 in which only CT is present among CT and NG, an amplification curve dependent on the presence of CT appears in each of the first data set and the second data set, but there is a difference in the magnitude of the amplification curve in each data set due to the temperature difference. In the case of tube 2 in which only NG is present, an amplification curve dependent on the presence of NG appears only in the second data set. In the case of tube 3 in which CT and NG with similar high concentrations are present together, an amplification curve dependent on the presence of CT is shown in the first data set. In addition, an amplification curve dependent on the presence of CT and an amplification curve dependent on the presence of NG are combined in the second data set, but the shapes of the amplification curves are similar due to similar concentrations and thus appear as one amplification curve. In the case of tube 4 in which CT and NG with different concentrations are present together, for example, when CT is a relatively high concentration and NG is a relatively low concentration, an amplification curve dependent on the presence of CT is shown in the first data set. In addition, an amplification curve dependent on the presence of CT and an amplification curve dependent on the presence of NG are combined in the second data set, and appear as a bumpy curve having different shapes of the amplification curves due to different concentrations. Meanwhile, in the case of tube 5 of the control group NTC in which neither CT nor NG is present, an amplification curve dependent on the presence of CT and an amplification curve dependent on the presence of NG are not shown in both the first data set and the second data set.

FIG. 2B exemplarily illustrates signal values dependent on the presence of the first target analyte at the first temperature and signal values dependent on the presence of the second target analyte at the second temperature, which are estimated from the first data set and the second data set shown in FIG. 2A.

Referring to FIG. 2B, each of the signal value dependent on the presence of the first target analyte (e.g., CT) at the first temperature (e.g., 72°C) and the signal value dependent on the presence of the second target analyte (e.g., NG) at the second temperature (e.g., 60°C.) may be extracted from the first data set and the second data set. For example, when only one among CT and NG is present, the amplification curve dependent on the presence of corresponding target analyte at the detection temperature of the corresponding target analyte may be extracted as shown in tubes 1 and 2. In addition, when both CT and NG are present, the amplification curve dependent on the presence of each target analyte at the detection temperature of each target analyte may be extracted as shown in tubes 3 and 4, but a number of cycles in which the slope of the amplification curve is maximum may be different according to the concentration difference. For example, in the case of a target analyte with a relatively low concentration, the number of cycles in which the slope of the amplification curve is maximum may be relatively pushed back compared to a target analyte at a relatively high concentration.

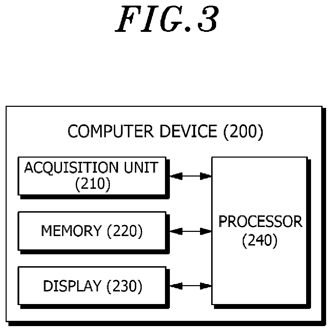

Hereinafter, the configuration and operation of the computer device 200 will be described in detail with reference to FIGS. 3 to 13.

FIG. 3 schematically shows a block diagram of the computer device 200 according to an embodiment. Referring to FIG. 3, the computer device 200 may include an acquisition unit 210, a memory 220, a display 230, and a processor 240.

The acquisition unit 210 is implemented to obtain the first data set and the second data set from a signal generation reaction of the plurality of cycles for the first target analyte and the second target analyte in a sample. In an embodiment, the acquisition unit 210 may obtain the first data set and the second data set from the data provision device 100. In another embodiment, when the signal generation reaction may be performed in the computer device 200, although not shown in the drawings, the acquisition unit 210 may obtain the first data set and the second data set from this performed result.

In an embodiment, the acquisition unit 210 may include a transceiver (not shown). In this case, the acquisition unit 210 may receive the first data set and the second data set from the data provision device 100 by using the transceiver. In another embodiment, the acquisition unit 210 may include a data input port (not shown). In this case, the acquisition unit 210 may read the first data set and the second data set generated by the data provision device 100 and stored in the corresponding device, from a storage device (e.g., a universal serial bus (USB)) electrically connected to the computer device 200 by using the data input port. It is obvious that the implementation examples of the acquisition unit (210) is not limited to the above-described embodiments.

The memory 220 may store at least one instruction or data to be executed by the processor 240, which will be described later. In an embodiment, the memory 220 may include at least one of a read only memory (ROM) and a random access memory (RAM).

The display 230 may display an image, a graph, a table, a text, or the like obtained as at least one instruction is executed by the processor 240. For example, the display 230 may display the detection result for the target analyte as text or the like, which is detected using the values of the parameters estimated by the processor 240 or the estimation result

The display 230 may comprehensively mean a data output device for displaying data. For example, the display 230 may be implemented as a liquid crystal display, a thin film transistor-liquid crystal display, an organic light-emitting diode, a flexible display, a three-dimensional (3D) display, or the like.

The processor 240 may control the overall operation of the computer device 200, and perform a series of operations for estimating a value(s) of a parameter(s) used to obtain an approximate signal(s) for a target analyte(s). For example, the processor 240 may refer to a central processing unit (CPU), a graphics processing unit (GPU), a micro controller unit (MCU), or a dedicated processor on which methods according to embodiments are performed. The processor 240 may be implemented to include at least one core.

In addition, it can be understood by those of ordinary skill in the art that other general-purpose components other than the components illustrated in FIG. 3 may be further included in the computer device 200. For example, the computer device 200 may further include a bus line used for transmission of data between the components, various input/output devices, and an interface therefor. In addition, one or more of the components illustrated in FIG. 3 may be omitted.

The processor 240 may provide various functions described below by executing at least one instruction stored in the memory 220. In an embodiment, various functions described below may be implemented as one or more computer programs, and instructions and data for execution of each computer program may be stored in the memory 220 and executed by the processor 240, but are not limited thereto.

The processor 240 may be implemented to estimate a value(s) of the approximate function parameter and a value(s) of the magnitude parameter by at least partially using the first data set and the second data set. Here, the approximate function parameter indicates a parameter included in at least one approximate function from among the approximate function *1* and the approximate function *2*.

Hereinafter, the approximate function *1*, the approximate function *2*, the approximate function parameter, and the magnitude parameter will be described first, and an operation of estimating a value of the parameters will be described in detail later.

In an embodiment, the approximate function *1* includes a function that approximates signal values dependent on the presence of the first target analyte in the sample at the first temperature or the second temperature. As described above, the signal dependent on the presence of the first target analyte may be generated at each of the first temperature and the second temperature by the signal generation reaction of the plurality of cycles for the first target analyte. In addition, due to the different temperatures, there may be a difference in the amount of signals generated at each temperature. In an embodiment, the approximate function *1* may include a function (hereinafter, referred to as an approximate function *1-1)* that approximates the signal value amplified depending on the presence of the first target analyte at the first temperature, and in another embodiment, may include a function (hereinafter, referred to as an approximate function *1-2)* that approximates the signal value amplified depending on the presence of the first target analyte at the second temperature. Meanwhile, hereinafter, the embodiment of the approximate function *1-1* is mainly described, but is not limited thereto. In addition, although the terms of the approximate function *1* are used in some of the embodiments described below, the approximate function *1* may be interpreted as the approximate function *1-1* and/or the approximate function *1-2* according to the embodiments.

In some example embodiments, the approximate function *1* may include at least one selected from the group consisting of a sigmoid function, a logistic function, a Gompertz function, and a Chapman function. For example, each of the approximate function *1-1* and/or the approximate function 1-2 may include a function represented by any one of the following Math Figure 1 to 6, but is not limited thereto, and may be represented in various other ways.

[Math Figure 1]

$$f(t) = \alpha_1 + \frac{\alpha_2 - \alpha_1}{1 + 10^{\alpha_4(\alpha_3 - t)}}$$

[Math Figure 2]

$$f(t) = \alpha_1 + \frac{\alpha_2 - \alpha_1}{1 + (t/\alpha_3)^{\alpha_4}}$$

[Math Figure 3]

$$f(t) = \alpha_1 + (\alpha_2 - \alpha_1)10^{-e^{(\alpha_3 - t)/\alpha_4}}$$

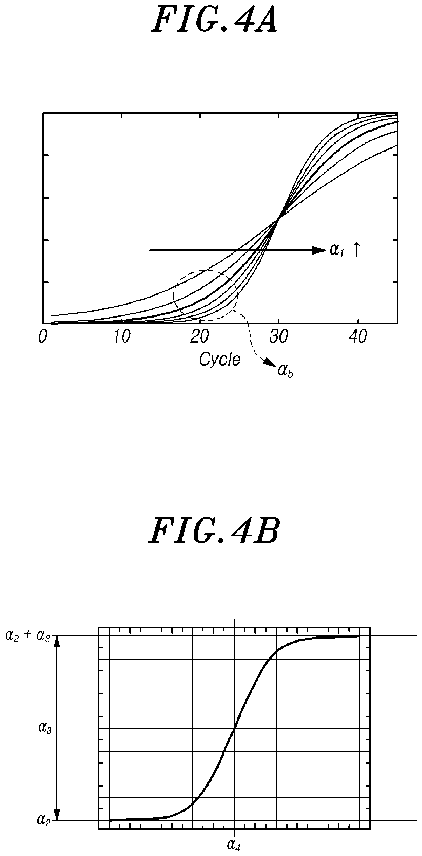

[Math Figure 4]

$$f(t) = \alpha_1 + (\alpha_2 - \alpha_1)(1 - 10^{-\alpha_4 t})^{\alpha_3}$$

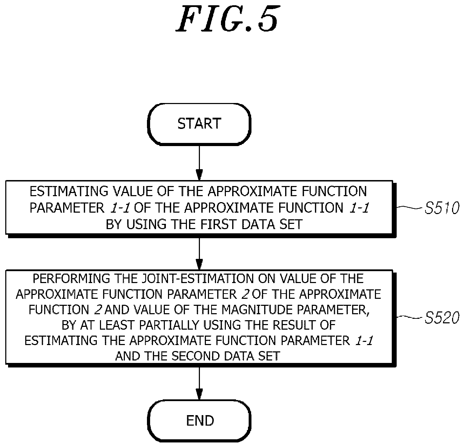

[Math Figure 5]

$$f(t) = \frac{\alpha_2}{\left(1 + e^{(\alpha_1(t-\alpha_3))}\right)^{\alpha_4}}$$

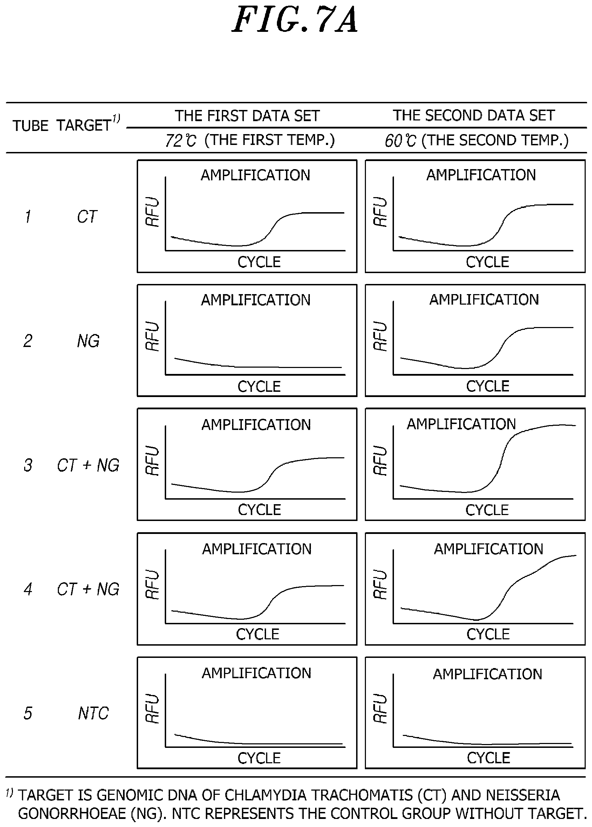

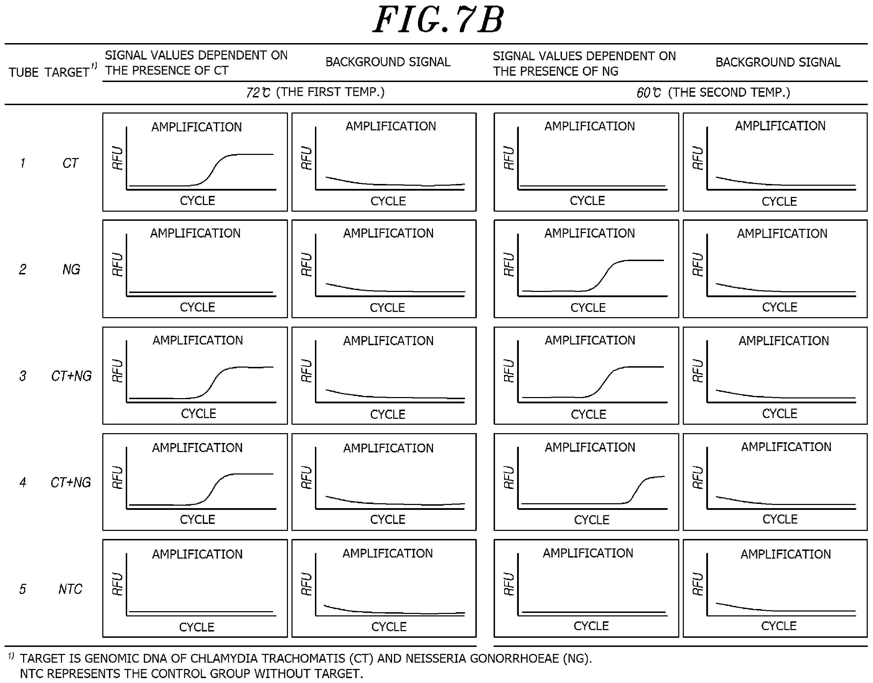

[Math Figure 7]

$$f(t) = \alpha_2 + \frac{\alpha_3}{\left(1 + e^{(\alpha_1(t-\alpha_4))}\right)^{\alpha_5}}$$

(wherein t represents the cycle and $\alpha_1$, $\alpha_2$, $\alpha_3$, $\alpha_4$, and $\alpha_5$ represent parameters included in the corresponding approximate function)

In an embodiment, a parameter(s) of the approximate function *1* (hereinafter, referred to as an approximate function parameter *1*) may represent a characteristic of a line represented by signal values of the plurality of cycles, wherein the signal values here are signal values that depend on the presence of the first target analyte. For example, the approximate function parameter *1-1* of the approximate function *1-1* may include parameters (e.g., $\alpha_1$ to $\alpha_5$ of Equations 1 to 6) for a shape, a gradient, a start point, and an end point of a line indicated by signal values of the plurality of cycles, wherein the signal values here are signal values that depend on the presence of the first target analyte at the first detection temperature. **In** addition, the approximate function parameter *1-2* of the approximate function *1-2* may include parameters for a shape, a gradient, a start point, and an end point of a line indicated by signal values of the plurality of cycles, wherein the signal values here are signal values that depend on the presence of the first target analyte at the second detection temperature. Throughout the specification, the approximate function parameter may be understood as a concept including a singular or plural number.

Specifically, the approximate function parameter *1* may include at least one of: (a) a parameter indicating a first slope for a cycle of the signal value dependent on the presence of the first target analyte; (b) a parameter indicating a cycle corresponding to a point at which the first slope is maximum; (c) a parameter indicating signal values in a baseline region of the signal value dependent on the presence of the first target analyte; and (d) a parameter indicating signal values in a plateau region of the signal value dependent on the presence of the first target analyte. For example, when the approximate function *1* is expressed by any one of Math Figure 1 to 6, each of $\alpha_1$ to $\alpha_5$ in Math Figure 1 to 6 may be the approximate function parameter *1*, and may be a parameter indicating the above first slope, the cycle corresponding to the point at which the first slope is maximum, the signal value in the baseline region or the signal value in the plateau region, a degree of steep slope of a curve at some points, or the like.

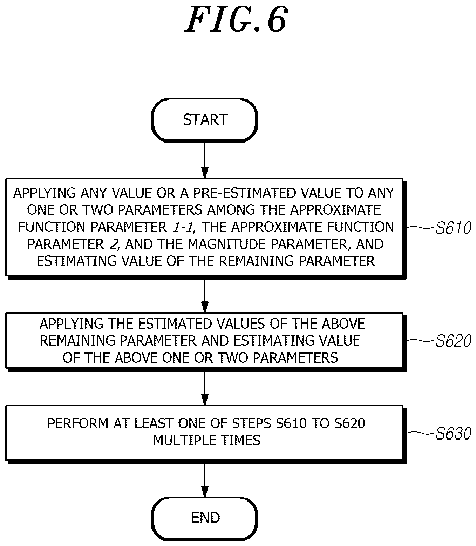

For example, referring to FIGS. 4A and 4B illustrating parameters of the sigmoid function according to Math Figure 6, $\alpha_1$ is a parameter indicating the degree of steepness of the slope in the S-shaped curve indicated by the sigmoid function, and may be a parameter indicating the above-described first slope. For example, as the $\alpha_1$ increases, the overall slope of the curve represented by the sigmoid function may increase, and the signal value may rapidly increase for a relatively short cycle to reach the saturation point of the curve, and may represent the above-described first slope. In addition, $\alpha_2$ is a parameter indicating signal values in the baseline region of the signal value dependent on the presence of the corresponding target analyte, and $\alpha_3$ is a parameter indicating signal values between the baseline region and the plateau region of the signal value dependent on the presence of the corresponding target analyte. In addition, $\alpha_4$ is a parameter indicating a cycle corresponding to a point at which a slope of signal values with respect to cycles in the exponential region is maximum among signal values dependent on the presence of a corresponding target analyte. For example, as $\alpha_4$ increases, the saturation point of the curve indicated by the sigmoid function may be delayed backward. In addition, $\alpha_5$ is a parameter indicating the degree to which the S-shaped curve indicated by the sigmoid function is asymmetrical, and may be, for example, a parameter indicating the degree to which a partial slope is steep in an initial local section where the increase in slope within the exponential region is remarkable (e.g., the cycle section from the cycle where the slope increases by more than the set value until the signal value reaches the inflection point). For example, as the $\alpha_5$ increases, the curve represented by the sigmoid function may not be a symmetrical S-shaped curve, but may be an asymmetrical S-shaped curve in which the initial gradient increases relatively steeply compared to the subsequent gradient. The above-described parameters are exemplary, and may be implemented in various modified forms according to the type of an approximate function or a concrete form of a mathematical formula.

In an embodiment, the approximate function *1* may include a target signal approximate function *1* that approximates signal values dependent on the presence of the first target analyte in the sample, and a background signal approximate function *1* that approximates a background signal (hereinafter, referred to as a first background signal) independent of the presence of the first target analyte in the first data set. The "background signal" will be described later. For example, the approximate function *1-1* may include a target signal approximate function *1-1* (e.g., $f_{11}(\alpha_{11},t)$) that approximates the signal values dependent on the presence of the first target analyte in the sample at the first temperature, and a background signal approximate function *1-1* (e.g., $g_{11}(\beta_{11},t)$) that approximates the first background signal at the first temperature. Alternatively, the approximate function 1-2 may include a target signal approximate function *1-2* (e.g., $f_{12}(\alpha_{12},t)$) that approximates the signal values dependent on the presence of the first target analyte in the sample at the second temperature, and a background signal approximate function *1-2* (e.g., $g_{12}(\beta_{12},t)$) that approximates the first background signal at the second temperature. Here, $\alpha_{11}$ refers to parameters of the target signal approximate function *1-1,* and $\alpha_{12}$ refers to parameters of the target signal approximate function *1-2.* The target signal approximate function *1-1* and/or the target signal approximate function *1-2* may include the embodiments of the approximate function *1* described above, and the parameters of the corresponding target signal approximate function may include the embodiments of the approximate function parameter *1* described above (e.g., $\alpha_1 \sim \alpha_5$ in the above Math Figures).

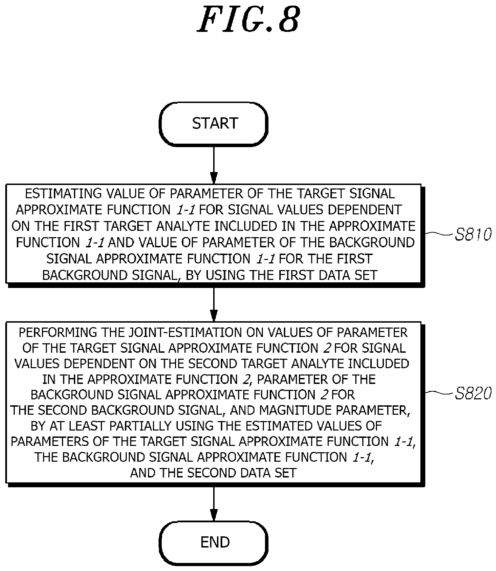

In an embodiment, the background signal approximate function *1* may be a constant or a polynomial function. For example, each of the background signal approximate function *1-1* and/or the background signal approximate function *1-2* may be expressed by Math Figure 7 below as the constant, or may be expressed by any one of Math Figure 8 to 10 below as the polynomial function of the second order or less, but is not limited thereto, and may be modified and expressed in various other forms.

[Math Figure 7]

$$g(t) = \beta_0$$

[Math Figure 8]

$$g(x) = \beta_0 + \beta_1 \cdot t$$

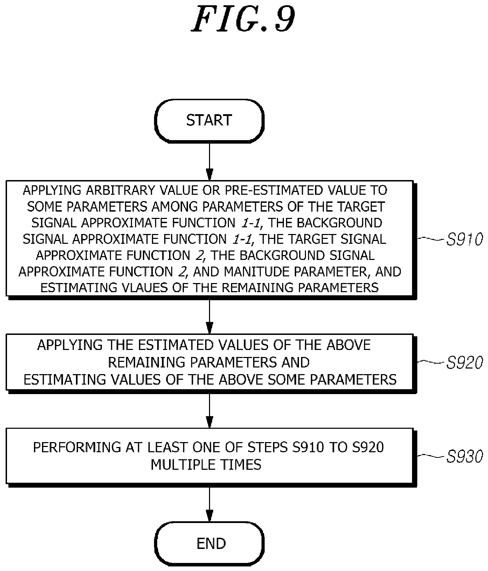

[Math Figure 9]

$$g(x) = \beta_0 + \beta_1 \cdot t + \beta_2 \cdot t^2$$

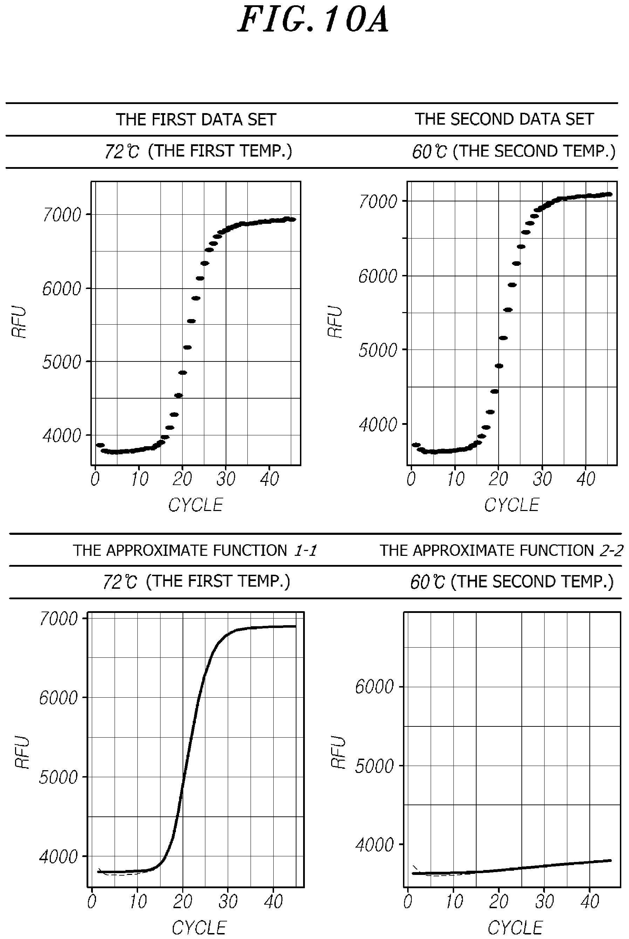

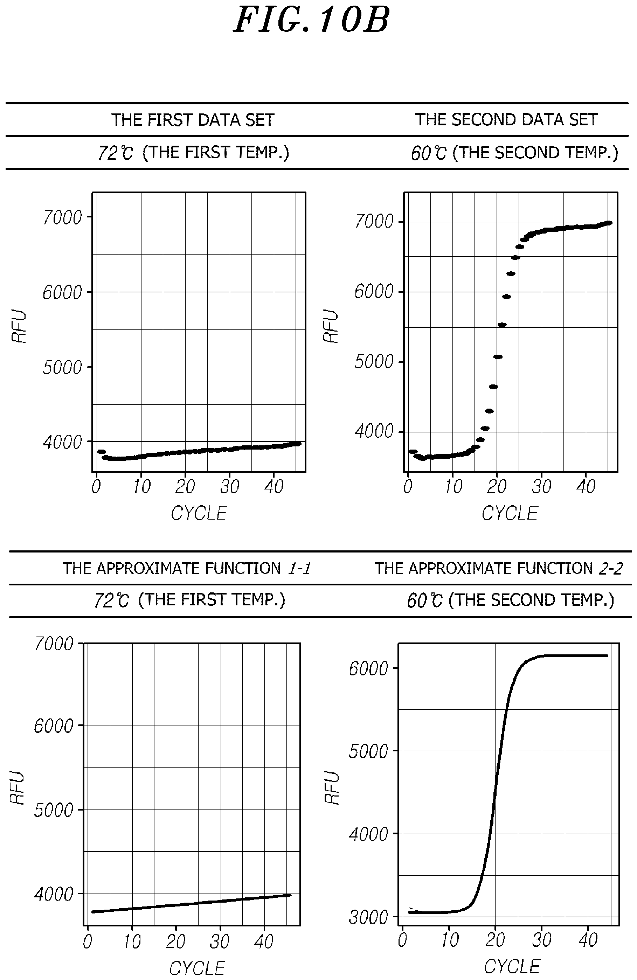

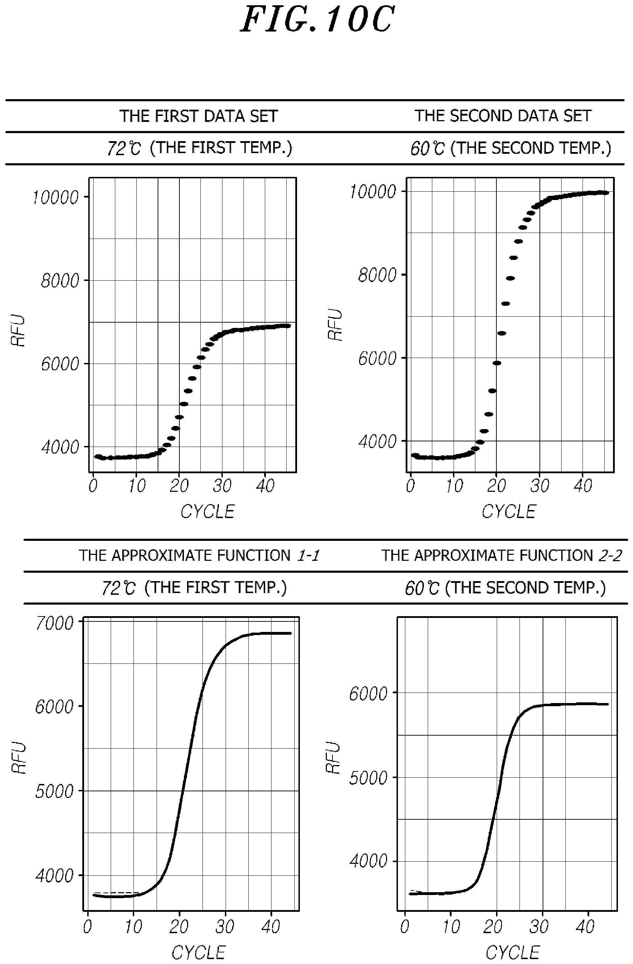

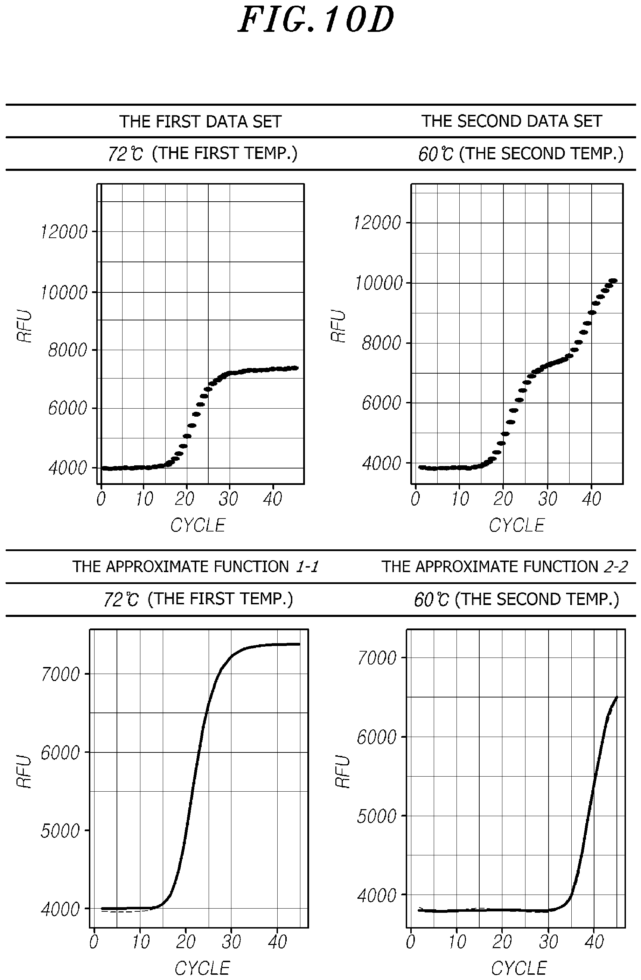

[Math Figure 10]

$$g(x) = \beta_1 \cdot (t - t_0)^2 + \beta_0$$

(wherein t denotes the cycle as well, $\beta_0$, $\beta_1$, $\beta_2$, and $t_0$ denote parameters included in the corresponding background signal approximate function. For example, $(\beta_2 > 0)$ may be assumed in Math Figure 9, and $(\beta_1 > 0)$ and $(t_0 \approx 45)$ may be assumed in Math Figure 10)

In an example embodiment, the approximate function parameter of the background signal approximate function *1* may indicate a characteristic of a line indicated by the first background signal. In detail, the approximate function parameter of the background signal approximate function *1* may include at least one of: (a) a parameter that approximates a slope of the cycle of the first background signal further included in the first data set; (b) a parameter that approximates signal values in the baseline region of the first background signal; and (c) a parameter that approximates the cycle corresponding to a point at which the magnitude of the first background signal is minimum. For example, the approximate function parameter of the background signal approximate function *1* may include parameters (e.g., $\beta_0$ to $\beta_2$) for a shape, a slope, a start point, and a minimum point of a line indicated by the first background signal.

In an embodiment, the approximate function 2 represents a function that approximates signal values dependent on the presence of the second target analyte in the sample. As described above, the signal dependent on the presence of the second target analyte may be generated at the second temperature from among the first temperature and the second temperature by the second signal generation reaction of the plurality of cycles for the second target analyte. For example, the approximate function 2 may be a function that approximates the signal value amplified depending on the presence of the second target analyte among the signal values included in the second data set measured at the second temperature, and may be interpreted as an approximate function *2-2* at the second temperature with reference to the embodiment of the approximate function *1-1* or the approximate function *1-2* described above.

In an embodiment, the approximate function 2 may include any one selected from the group consisting of the sigmoid function, the logistic function, the Gompertz function, and the Chapman function. Likewise, the approximate function 2 may include a function represented by any one of Math Figures 1 to 6, but is not limited thereto.

In an embodiment, a parameter(s) included in the approximate function 2 (hereinafter, referred to as an approximate function parameter 2) may represent a characteristic of a line indicated by the signal value dependent on the presence of the second target analyte.

Specifically, the approximate function parameter 2 may include at least one of: (a) a parameter indicating a second slope for a cycle of the signal value dependent on the presence of the second target analyte; (b) a parameter indicating a cycle corresponding to a point at which the second slope is maximum; (c) a parameter indicating signal values in the baseline region of the signal value dependent on the presence of the second target analyte; and (d) a parameter indicating signal values in the plateau region of the signal value dependent on the presence of the second target analyte. Likewise, the approximate function parameter 2 may include the above-described embodiments with respect to the approximate function parameter *1*.

In an embodiment, the approximate function 2 may include a target signal approximate function 2 that approximates signal values dependent on the presence of the second target analyte in the sample, and a background signal approximate function 2 that approximates a background signal (hereinafter, referred to as a second background signal) independent of the presence of the second target analyte in the second data set. For example, the approximate function 2 may include the target signal approximate function 2-2 (e.g., $f_{22}(\alpha_{22},t)$) that approximates signal values dependent on the presence of the second target analyte in the sample at the second temperature, and a background signal approximate function 2-2 (e.g., $g_{22}(\beta_{22},t)$) that approximates the second background signal at the second temperature. Here, $\alpha_{22}$ refers to parameters of the target signal approximate function *2-2*, and $\beta_{22}$ refers to parameters of background signal approximate function *2-2*. The target signal approximate function *2-2* may include the embodiments of the approximate function 2 described above, and the parameters included in the target signal approximate function *2-2* may include the embodiments of the approximate function parameter *2* described above.

In an embodiment, the background signal approximate function *2* may be the constant or the polynomial function. For example, the background signal approximate function *2-2* may be expressed by one of Math Figures 7 to 10.

The approximate function parameter of the background signal approximate function *2* may indicate a characteristic of a line indicated by the second background signal. Similarly, the approximate function parameter of the background signal approximate function *2* may include at least one of: (a) a parameter that approximates a slope of a cycle of the second background signal further included in the second data set; (b) a parameter that approximates signal values in the baseline region of the second background signal; and (c) a parameter that approximates a cycle corresponding to a point at which a magnitude of the second background signal is minimum. Likewise, the approximate function parameter included in the background signal approximate function 2 may include the above-described embodiments with respect to the approximate function parameter included in the background signal approximate function *1*.

In an embodiment, the background signal may be a signal obtained depending on the type of at least one of the detection device (e.g., CFX96) in which the signal generation reaction for the target analyte is performed, the reaction well in which the sample is accommodated (e.g., a well shape, an accommodation method, etc.), and a plate in which the reaction well is placed (e.g., 12x8 arrangement structure). For example, the first background signal may be generated by the type of detection device, the shape of the reaction well, etc., that are causes other than the presence of the first target analyte in the first data set. In addition, the second background signal may be generated by the type of detection device, the shape of the reaction well, etc., that are causes other than the presence of one or more among the first target analyte and the second target analyte in the second data set. In addition, according to an embodiment, a m-th background signal including a third background signal may also be interpreted in the same manner.

According to an embodiment, the background signal may be subdivided into a plurality of preset background elements (e.g., a baseline element in the detection device, a noise element due to channel interference, an element for correcting a signal pattern difference due to the use of a specific signal generation composition (e.g., a label, an enzyme, etc.), etc.). According to an embodiment, the background signal approximate function for approximating this background signal may be expressed in the form of a linear function of a constant or polynomial function in which all the corresponding background elements are considered, or may be expressed in the form of a nonlinear function obtained by combining (e.g., summing) the constant or polynomial function for each background element. As such, the background signal approximate function may be extended in a form in which several background elements are considered in the above manner.

In an embodiment, the magnitude parameter indicates a magnitude relationship between (a) the signal value dependent on the presence of the first target analyte at the first detection temperature and (b) the signal value dependent on the presence of the first target analyte at the second detection temperature. In a specific embodiment, the magnitude parameter may be a parameter indicating a ratio of (a) the signal value dependent on the presence of the first target analyte at the second temperature to (b) the signal value dependent on the presence of the first target analyte at the first temperature (or vice versa). For example, when the signal value of an end cycle dependent on the presence of the first target analyte at the first temperature is a, and the signal value of the end cycle dependent on the presence of the first target analyte at the second temperature is b (see tube 1 in FIG. 2A), the magnitude parameter may mean a ratio of b to a (e.g., b/a), a ratio of a to b (e.g., a/b), or a value corresponding thereto. For example, the magnitude parameter is a cut-off ratio used in the art, may be a parameter for a constant or a set of constants that approximates the ratio, and may be used to specify a portion of the signal value dependent on the presence or absence of the first target analyte at the second temperature.

In the related art, a statistical value of the magnitude parameter was previously obtained from signal values of a plurality of experimental data in which information about which target analyte is present with what quantities in a sample is provided in advance, and the value of the magnitude parameter was previously set based on the statistical value. After that, when detecting a target analyte in any sample where it is unknown whether any target analyte is present, the value of the preset magnitude parameter was uniformly applied to the any sample. Accordingly, it is difficult to reflect a difference generated due to different detection environments in the process of detecting the target analyte, such as a composition in a sample, the type of the reaction well or a the detection device, and thus there is a problem in that detection accuracy is reduced.

However, in the present disclosure, whenever detecting the target analyte for any sample where it is unknown whether any target analyte is present in the tube, this magnitude parameter may be estimated. Accordingly, an optimized value is obtained for each reaction well in which the sample is accommodated or for each reaction performed on the sample to apply a more accurate magnitude parameter. In addition, numerous procedures for setting the magnitude parameter conventionally required can be omitted, thereby having a cost-effective advantage.

The processor 240 may perform a j oint-estimation on (a) a value(s) of at least one of the approximate function parameter *1* and the approximate function parameter 2; and (b) a value(s) of the magnitude parameter, by at least partially using at least one of the first data set and the second data set. Here, the joint-estimation means that two or more parameters are jointly estimated at the same time. For example, it indicates a case where one parameter depends on another parameter, and vice versa. In an embodiment, one or more of various known algorithms such as a residual analysis-based linear regression, a generalized additive model-based non-linear regression, a joint maximum likelihood estimation, and a Bayesian optimization may be used for the j oint-estimation, but are not limited thereto.

In an embodiment, the processor 240 may perform the joint-estimation on a value(s) of the approximate function parameter *1,* a value(s) of the approximate function parameter *2,* and a value(s) of the magnitude parameter, by at least partially using the first data set and the second data set. For example, the processor 240 may calculate the value of the approximate function parameter *1,* the value of the approximate function parameter 2, and the value of the magnitude parameter, which enable a total error to be minimized by considering both (a) a difference between the first data set and a first approximate data set calculated using the approximate function *1,* and (b) a difference between the second data set and a second approximate data set calculated using all of the approximate function *1,* the approximate function *2,* and the magnitude parameter, for the plurality of cycles.

In another embodiment, the processor 240 may perform the j oint-estimation on a value(s) of the approximate function parameter *2* and a value(s) of the magnitude parameter, by at least partially using the second data set. For example, the processor 240 may calculate a value of the approximate function parameter *2* and a value of the magnitude parameter, which minimize a difference between the second approximate data set and the second data set for the plurality of cycles.

Meanwhile, the joint-estimation process may be performed in a method of joint-estimating a value(s) of the approximate function parameter and a value(s) of the magnitude parameter, wherein the approximate function here includes at least one selected from the group consisting of the approximate function *1-1,* the approximate function *1-2,* and the approximate function 2. According to an embodiment, the joint-estimation may be performed on a parameter value(s) of a first parameter group including the approximate function parameter of the approximate function *1-1* and the approximate function *2* and the magnitude parameter. The magnitude parameter in the first parameter group may indicate a ratio of a signal magnitude at the second detection temperature to a signal magnitude at the first detection temperature. According to another embodiment, the j oint-estimation may be performed on a parameter value(s) of a second parameter group including the approximate function parameter of the approximate function *1-2* and the approximate function *2,* and the magnitude parameter. The magnitude parameter in the second parameter group may indicate a ratio of a signal magnitude at the first detection temperature to a signal magnitude at the second detection temperature. Hereinafter, the former embodiment will be mainly described, but it can be seen that the latter embodiment may also be modified and implemented in a similar manner.

Various embodiments of the function/operation of the processor 240 presented above will be described in more detail below.

1) Statistical model for the data set

The processor 240 may prepare a joint-estimation for the above-described parameters based on a pre-stored statistical model. Here, preparing the joint-estimation may be understood as a concept encompassing an operation of loading or storing a statistical model in the memory 220 or executing one or more instructions stored in the memory 220 in order to perform a parameter estimation function using the statistical model.

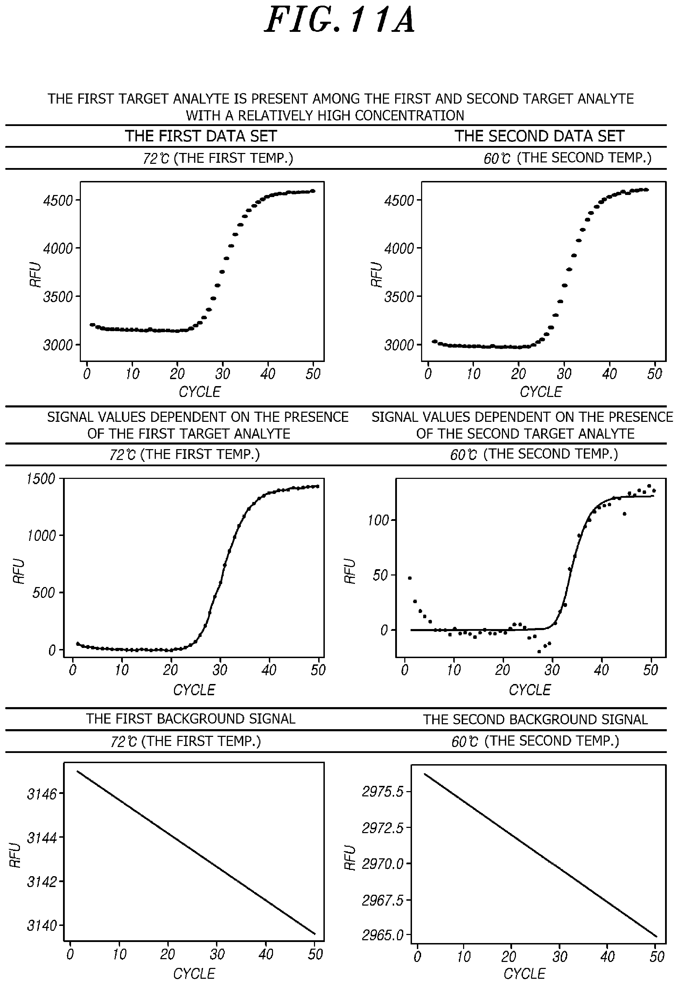

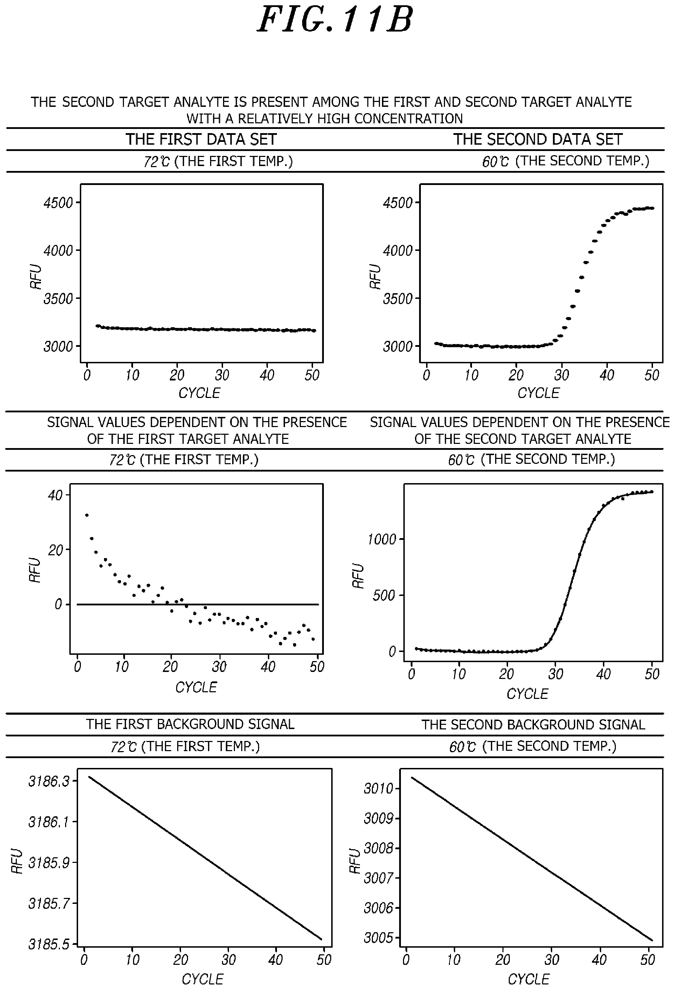

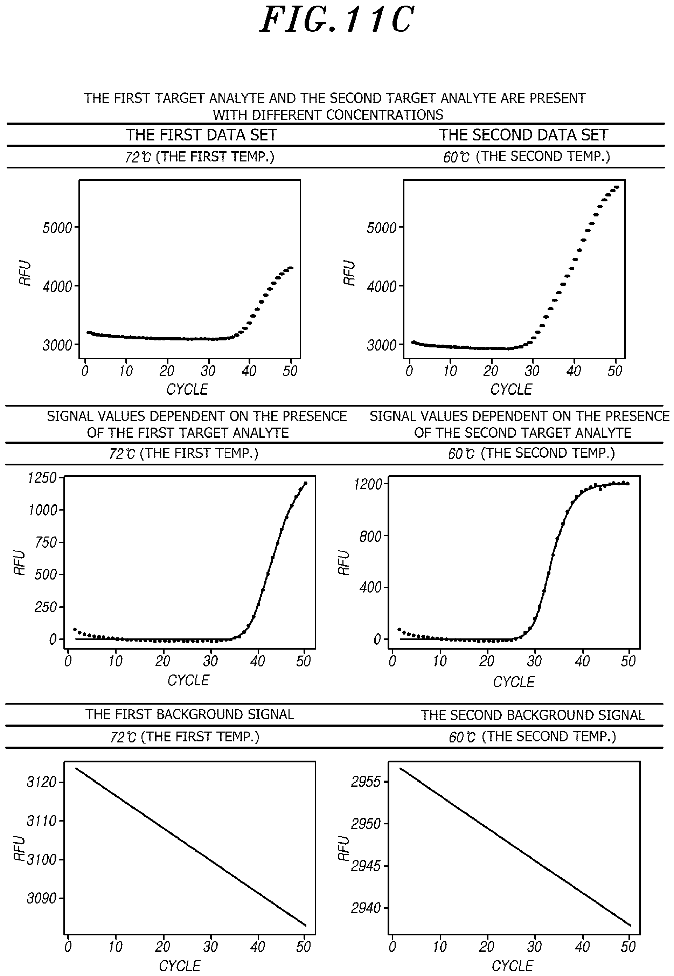

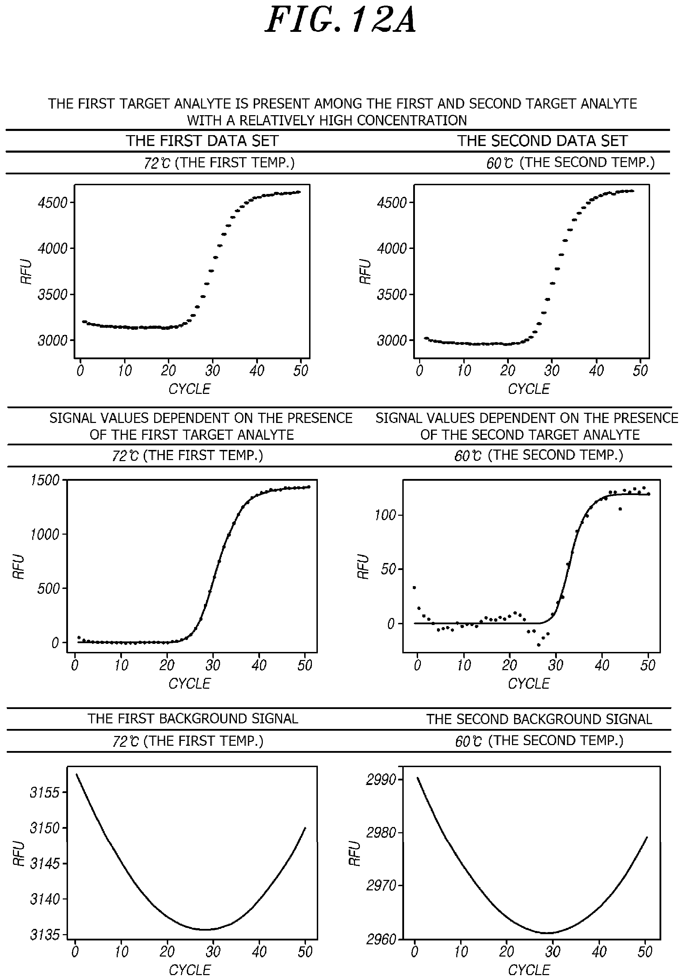

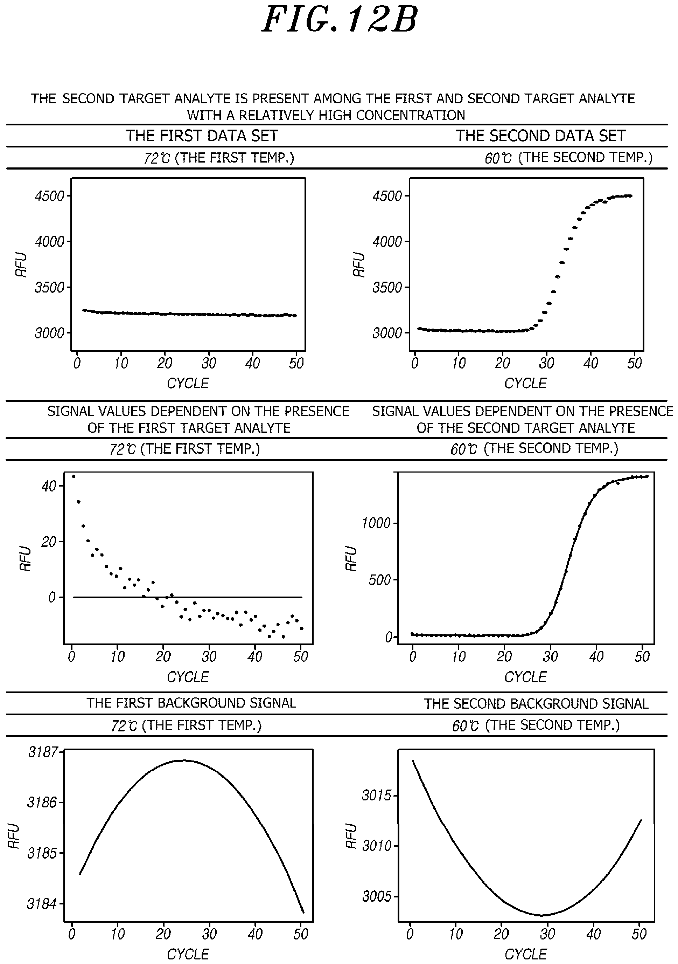

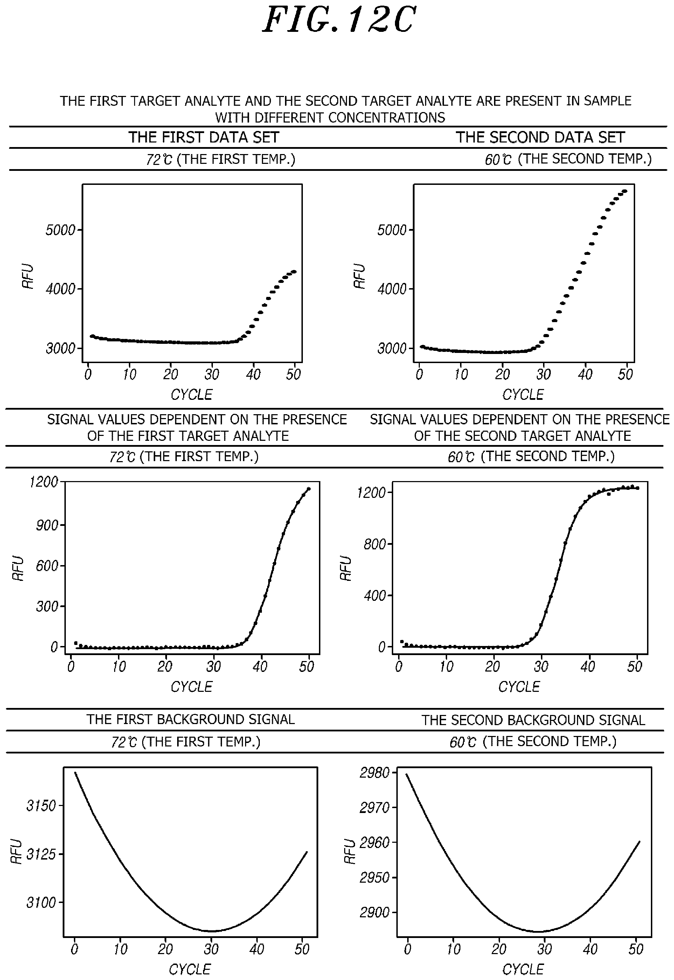

Specifically, in the statistical model, a mathematical relationship for separating signal values dependent on the presence of the first target analyte from the first data set measured at the first temperature may be defined. In addition, in the statistical model, a mathematical relationship for excluding signal values dependent on the presence of the first target analyte from the second data set measured at the second temperature and separating signal values dependent on the presence of the second target analyte may be defined. For example, the statistical model may include Math Figure 11 below defining a relationship between the first data set and the approximate function *1-1* and defining a relationship between the second data set, the approximate function *1-1*, the approximate function 2, and the magnitude parameter. Math Figure 11 may be expressed as Math Figures 12 to 13.

[Math Figure 11]

$$\begin{pmatrix} y_{1,t} \\ y_{2,t} \end{pmatrix} = \begin{pmatrix} 1 & 0 \\ CR & 1 \end{pmatrix} \begin{pmatrix} f_{11}(\alpha_{11},t) \\ f_{22}(\alpha_{22},t) \end{pmatrix} + \begin{pmatrix} e_1 \\ e_2 \end{pmatrix}$$

[Math Figure 12]

$$y_{1,t} = f_{11}(\alpha_{11},t) + e_1$$

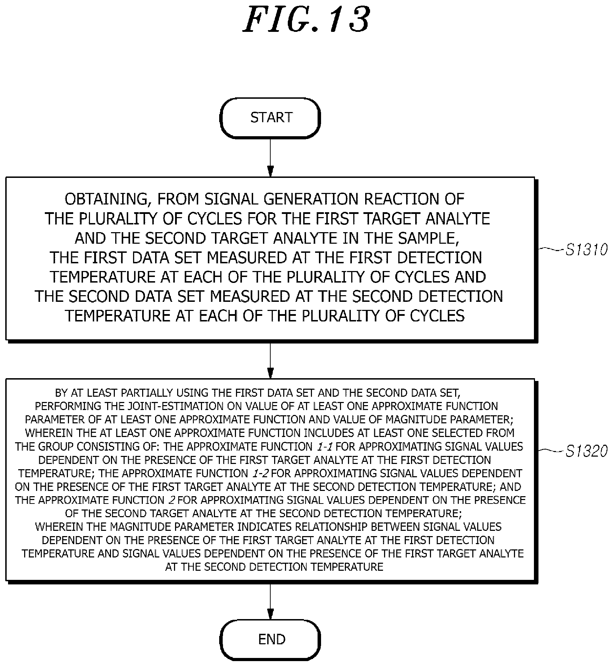

[Math Figure 13]

$$y_{2,t} = CR * f_{11}(\alpha_{11},t) + f_{22}(\alpha_{22},t) + e_2$$

Throughout the specification, t denotes the cycle, $y_{1,t}$ denotes the first data set measured at the first temperature at each of the plurality of cycles, and $y_{2,t}$ denotes the second data set measured at the second temperature at each of the plurality of cycles. $\alpha_{11}$ denotes the approximate function parameter *1-1*, $\alpha_{22}$ denotes the approximate function parameter *2-2*, and CR denotes the magnitude parameter. $f_{11}(\alpha_{11,t})$ represents the approximate function *1-1* (specifically, the target signal approximate function *1-1*) in which the approximate function parameter *1-1* and the cycle are input variables, and $f_{22}(\alpha_{22,t})$ represents the approximate function *2-2* (specifically, the target signal approximate function *2-2*) in which the approximate function parameter *2-2* and the cycle are input variables. Further, each of $e_1$ and $e_2$ represents a first error representing an error component in the first data set and a second error representing an error component in the second data set, respectively. Here, the error component means a component that cannot be described based on a predetermined rule or criterion, and for example, may be expressed as a residual component corresponding to a difference between the data set and an approximate data set approximated through the approximate function.

For example, referring to Math Figures 11 to 13, the first data set may be defined as the sum of the approximate function *1-1* and the first error, and the second data set may be defined as a sum of: (a) a product of the approximate function *1-1* and the magnitude parameter; (b) the approximate function 2; and (c) the second error. According to embodiments, when the approximate function *1* is the approximate function *1-2* approximating the signal value at the second temperature, as a modified form of Math Figures 11 to 13, the first data set may be defined as a sum of: (a) a product of an inverse of the magnitude parameter and the approximate function *1-2*; and (b) the first error. The second data set may be defined as a sum of: (a) the approximate function *1-2*; (b) the approximate function 2; and (c) the second error, and the following objective functions may be modified in a similar manner.

The processor 240 may perform the joint-estimation on the above-described parameters based on the statistical model. In an embodiment, the statistical model may include one or more objective functions for estimation of values of optimized parameters that approximate the data set. The processor 240 may obtain a value(s) of a parameter(s) that causes the objective function to be a maximum value or a minimum value and calculate the value as an estimate of the optimized parameter. Hereinafter, various embodiments in which the joint-estimation is performed on values of parameters by using various objective functions based on likelihood will be described.

2) the joint-estimation according to a first embodiment

FIG. 5 is a flowchart exemplarily illustrating a method for performing the joint-estimation on the value of the approximate function parameter *2* and the value of the magnitude parameter through the processor 240 by the computer device 200 according to the first embodiment.

Referring to FIG. 5, in step S510, the processor 240 may estimate a value of the approximate function parameter *1-1* of the approximate function *1-1* by using the first data set. For example, the processor 240 may calculate the value of the approximate function parameter *1-1* that best approximates signal values dependent on the first target analyte at the first temperature from the first data set.

In an embodiment, the value of the approximate function parameter *1-1* may be estimated in such a manner that the sum of differences between the approximate function *1-1* and the first data set at each of the plurality of cycles is minimized. As described above, the difference between the approximate function *1-1* and the first data set may simply mean a magnitude difference, or may mean an absolute value, a square, or a mean squared error, a root mean squared error, or the like corresponding to the magnitude difference.

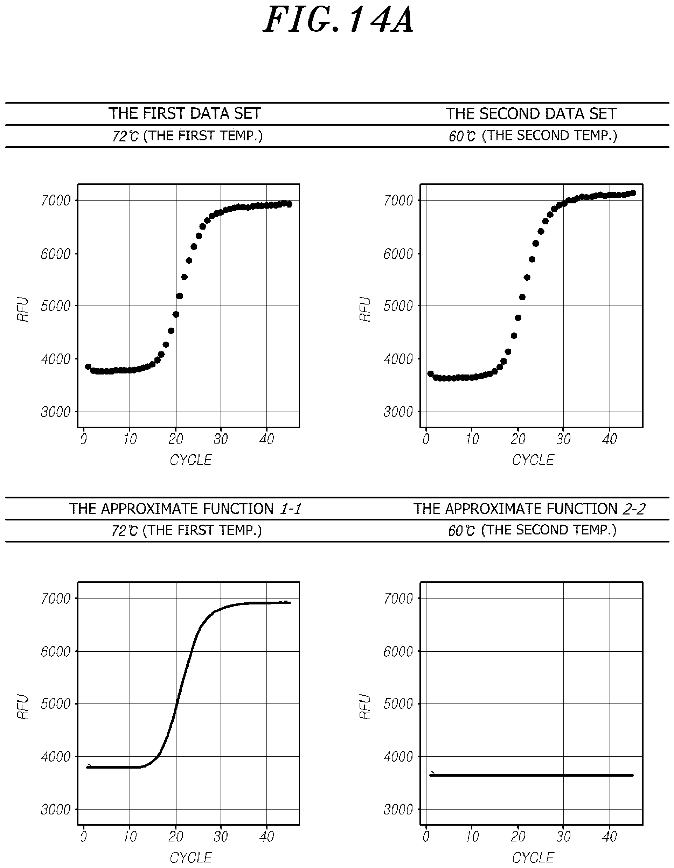

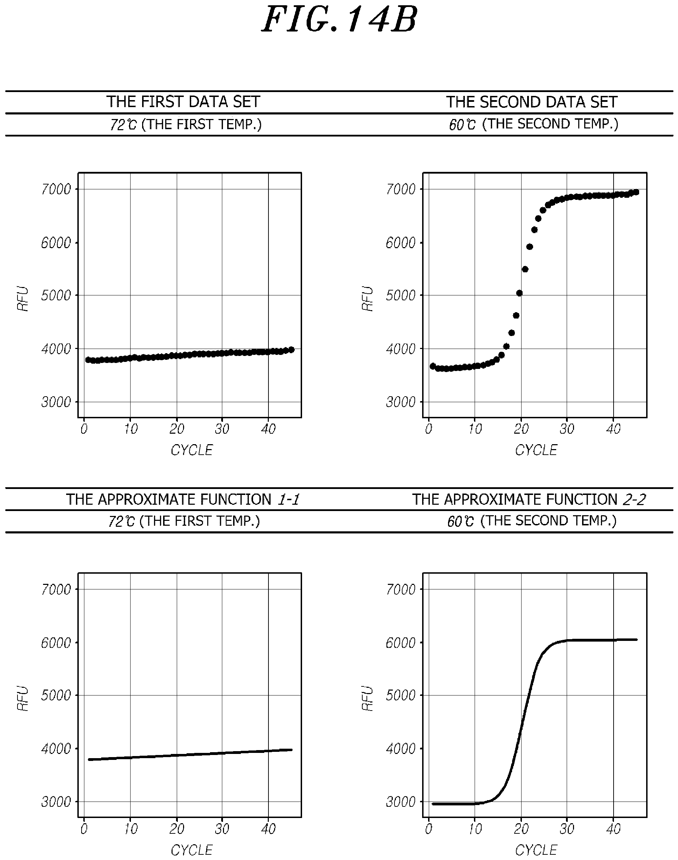

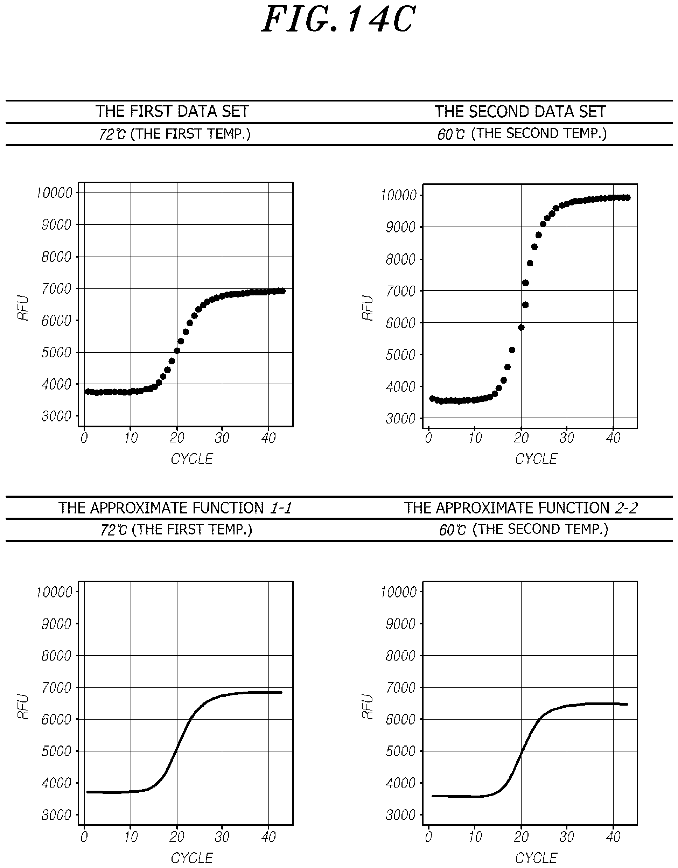

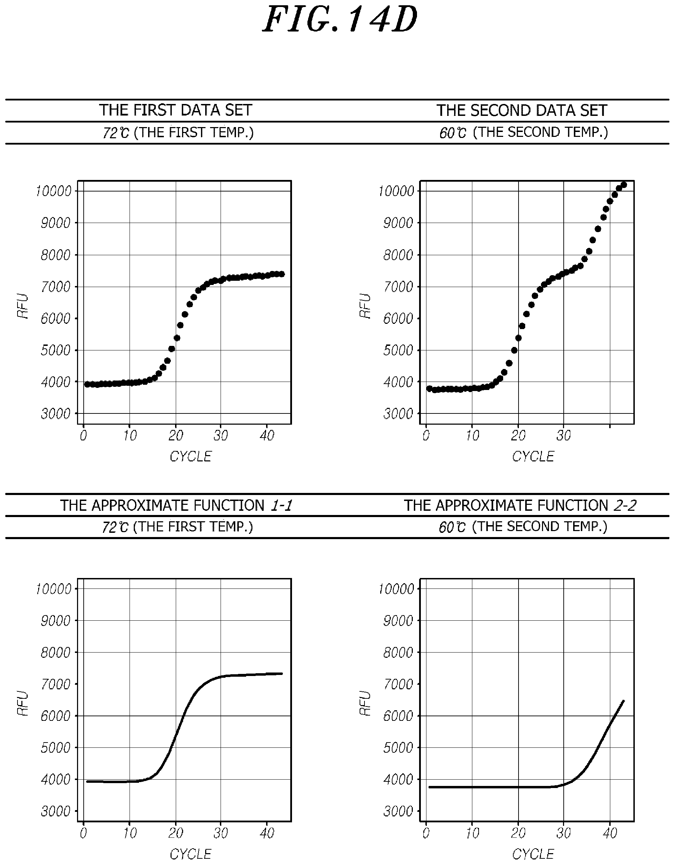

More specifically, the processor 240 may estimate a value(s) of the approximate function 1-1 parameter by using a first objective function for the first data set. In an embodiment, the first objective function may be determined based on a difference (e.g., the first error) between the first data set and the approximate function *1-1* at each of the plurality of cycles. As an example, the first objective function may be expressed as Math Figure 14 below, but is not limited thereto, on the assumption that the first error follows a normal distribution of $N(0, \sigma^2_{first})$. For example, the first objective function may be expressed as a sum of a difference between the approximate function *1* and the first data set at each of the plurality of cycles, may be expressed as an average of the sum of the corresponding difference, or may be expressed in various other modified forms.

[Math Figure 14]

$$l_1(\alpha_{11}) = \frac{1}{n}\sum_{t=1}^{n}(y_{1,t} - f_{11}(\alpha_{11}, t))^2$$

(where $l_1(\alpha_{11})$ represents the first objective function used for estimating the value of the approximate function parameter *1-1*, and n represents a final cycle (e.g., 45) among the cycles)

The processor 240 may calculate a value(s) of the approximate function parameter *1-1* that causes the first objective function to be a maximum value or a minimum value. In an embodiment, to find the minimum value (or maximum value) of the first objective function, at least one of a differentiation method, a difference method, a step difference method, a ratio approach, a linear regression analysis, and a gradient descent method of the signal value may be used. For example, an optimized value of the approximate function parameter *1*, $\alpha_{11}$, may be derived through an operation of finding extremum by partial differentiation for the approximate function parameter *1-1*,$\alpha_{11}$, to be optimized in the first objective function.

In an embodiment, the value of the approximate function parameter *1-1* may be estimated based on maximum likelihood estimation method or maximum likelihood estimate method. Here, being based on maximum likelihood estimation or maximum likelihood estimate may be understood as a concept including a log-likelihood estimation (or estimate) and a negative log-likelihood estimation (or estimate) obtained by adding a predetermined mathematical operation (e.g., a log operation, a negative operation, or the like) to the calculation result of the maximum likelihood estimation or the maximum likelihood estimate.

In step S520, the processor 240 may perform the joint-estimation on a value(s) of the approximate function parameter *2* of the approximate function *2* and a value(s) of the magnitude parameter, by at least partially using the result of estimating the approximate function parameter *1-1* and the second data set. For example, when the value of the approximate function parameter *1-1* estimated in step S510 is applied, the processor 240 may calculate the value of the magnitude parameter and the value of the approximate function parameter *2* that best approximate the signal value dependent on the first target analyte at the second temperature and the signal value dependent on the second target analyte at the second temperature in the second data set.

In an embodiment, each of the value of the approximate function parameter *2* and the value of the magnitude parameter may be estimated in such a manner that a difference between (a) the second data set and (b) the sum of the approximate function *2* and the signal value dependent on the first target analyte at the second temperature (or the approximate function *1-2*) is minimized, at each of the plurality of cycles,. Similarly, the difference may mean a simple magnitude difference, an absolute value, a square or a mean squared error, a root mean squared error, or the like of the corresponding magnitude difference.

In detail, the processor 240 may prepare the signal value (or the value of the approximate function *1-2*) dependent on the first target analyte at the second temperature by using the value of the magnitude parameter and the value of the approximate function *1-1* having the estimated value of the approximate function parameter *1*. The processor 240 may calculate a difference between (a) the second data set; and (b) a sum of the approximate function *2* and the signal value (or the approximate function *1-2*) dependent on the first target analyte at the second temperature, at each of the plurality of cycles. The processor 240 may perform the joint-estimation on the value of the approximate function parameter *2* and the value of the magnitude parameter so that the sum of the differences at the plurality of cycles is minimized.

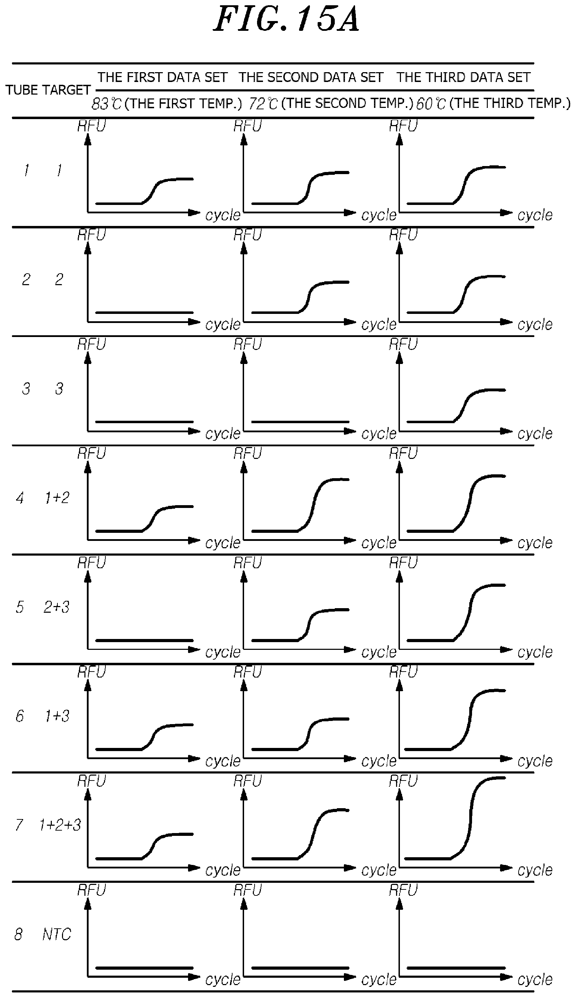

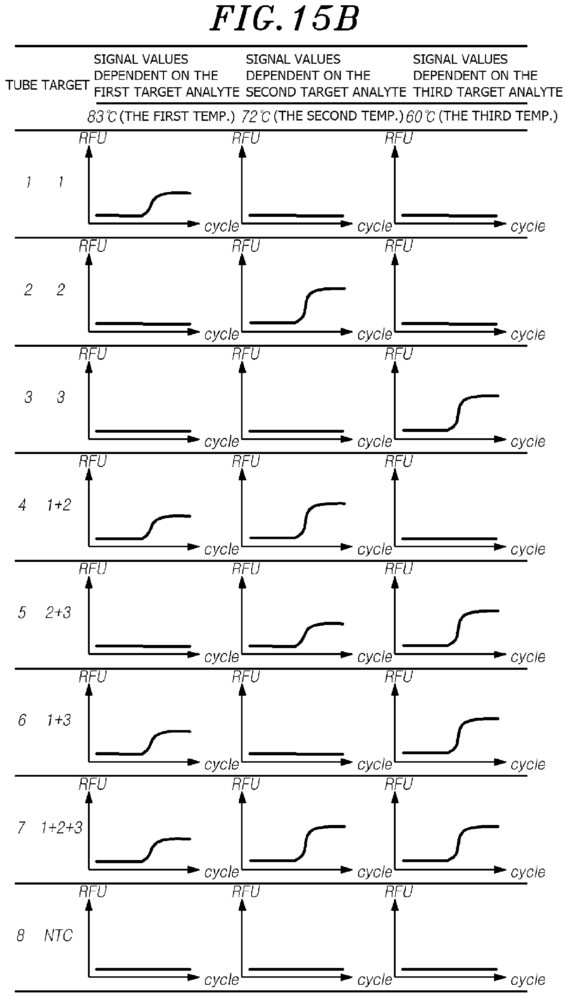

For example, the processor 240 may perform the joint-estimation on the value of the approximate function parameter 2 and the value of the magnitude parameter, by using a second objective function for the second data set. In an embodiment, the second objective function may output a difference (e.g., the second error) between (a) the second data set; and (b) a sum of the approximate function *2* and the signal value (or the approximate function *1-2*) dependent on the first target analyte at the second temperature, at each of the plurality of cycles. For example, when it is assumed that the second error follows a normal distribution of $N(0, \sigma^2_{second})$, the second objective function may be expressed as Math Figure 15 below, but is not limited thereto. Referring to Math Figure 15, the above-described "the signal value (or the approximate function *1-2*) dependent on the first target analyte at the second temperature at each of the plurality of cycles" may be calculated as the product of the magnitude parameter and the approximate function *1-1* to which the value of the approximate function parameter *1* estimated in step S510 is applied (e.g., $CR*f_{11}(\alpha_{11}, t)$), and according to an embodiment, the corresponding calculation result may correspond to the approximate function *1-2* (e.g., $f_{12}(\alpha_{12}, t)=CR*f_{11}(\alpha_{11}, t)$) at each of the plurality of cycles. In addition, "the sum of the approximate function *2* and the signal value (or the approximate function *1-2*) dependent on the first target analyte at the second temperature at each of the plurality of cycles" may be calculated as the sum of the above-described operation results (e.g., $f_{22}(\alpha_{22}, t) + CR*f_{11}(\alpha_{11}, t)$).

[Math Figure 15]

$$l_2(\alpha_{22}, CR) = \frac{1}{n}\sum_{t=1}^{n}(y_{2,t} - CR * f_{11}(\alpha_{11}, t) - f_{22}(\alpha_{22}, t))^2$$

(where $l_2(\alpha_{22})$ represents the second objective function used for estimation of an approximate function parameter *2-1* and the magnitude parameter)

The processor 240 may calculate a value(s) of the approximate function parameter *2* and a value(s) of the magnitude parameter that enable the second objective function to be the maximum value or the minimum value. For example, the processor 240 may obtain the signal value (e.g., $CR*f_{11}(\alpha_{11})$) dependent on the first target analyte at the second temperature or the value (e.g., $f_{12}(\alpha_{12}, t)$) of the approximate function *1-2*, by using the magnitude parameter and the value of the approximate function *1-1* having the estimated value of the approximate function parameter *1*. In addition, the processor 240 may obtain a difference (e.g., $y_{2,t} - CR*f_{11}(\alpha_{11}) - f_{22}(\alpha_{22})$) between (a) the second data set (e.g., $y_{2,t}$); and (b) a sum of the approximate function *2-2* (e.g., $f22(\alpha22)$) and the signal value (e.g., $CR*f_{11}(\alpha_{11})$) dependent on the first target analyte at the second temperature, at each of the plurality of cycles. In addition, the processor 240 may perform the joint-estimation on the value of the approximate function parameter *2-2* and the value of the magnitude parameter so that the sum of the above differences at the plurality of cycles is minimized.

Meanwhile, in the above-described joint-estimation process, instead of by using the result estimated with respect to the approximate function parameter *1-1* and the second data set, the joint estimation may be performed by using the first data set and the second data set.

In detail, the processor 240 may estimate a value(s) of the approximate function parameter *1-1* of the approximate function *1-1* by using the first data set, and may perform the joint-estimation on a value of(s) the approximate function parameter *2* and a value of the magnitude parameter by using the first data set and the second data set. In an embodiment, the processor 240 may calculate a difference between (a) the second data set; and (b) the first data set and the magnitude parameter;, at each of the plurality of cycles, and may perform the joint-estimation on the value of the approximate function parameter *2* and the value of the magnitude parameter so that the sum of the differences at each of the plurality of cycles is minimized. As a specific example, $f_{11}(\alpha_{11}, t)$ described in Math Figure 15 may correspond to a result of estimating the approximate function parameter *1-1*, and the corresponding $f_{11}(\alpha_{11}, t)$ in Math Figure 15 may be replaced with $y_{1,t}$ corresponding to the first data set. In this case, referring to Math Figure 12, there is an advantage in that an error corresponding to the first error due to the approximate function *1-1* may be improved in the process of estimating the approximate function *2* and the magnitude parameter.

In another embodiment, each of the value of the approximate function parameter *2* and the value of the magnitude parameter may be estimated in such a manner that the total error determined based on the difference between (a) the second data set; and (b) the sum of the approximate function *2* and the signal value (or the approximate function *1-2)* dependent on the first target analyte at the second temperature, at each of the plurality of cycles, is minimized. In an embodiment, the total error is a value obtained by scaling the first error and the second error, and for example, may be calculated by summing or arithmetically averaging the values of the first error and the second error which are scaled in units through standardization for making an average be 0 and a variance be 1 or mean normalization for making an average be 0 and a value range from -1 to 1.

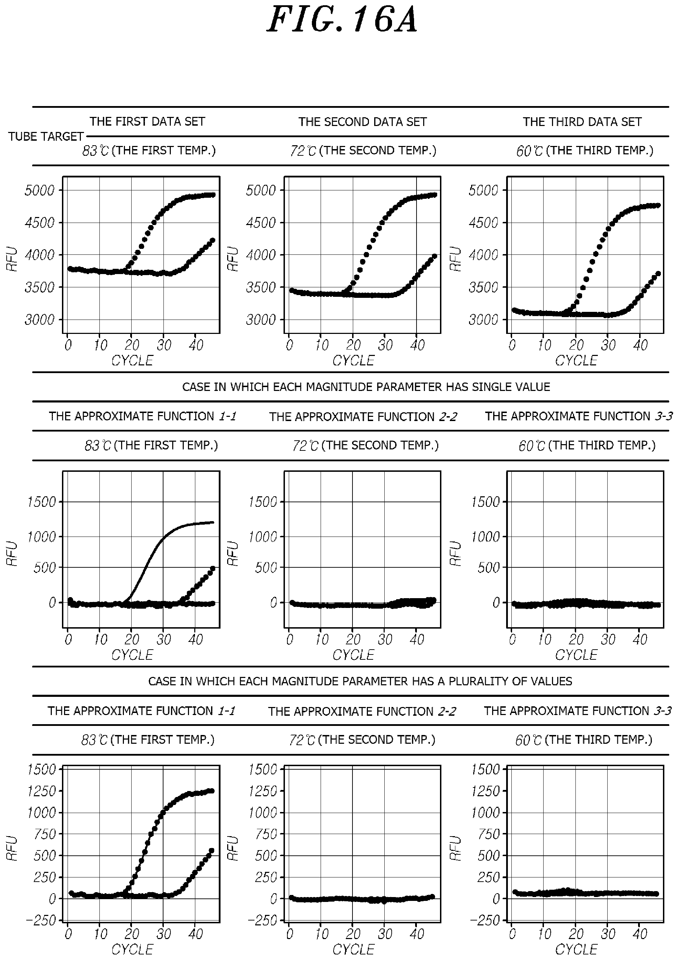

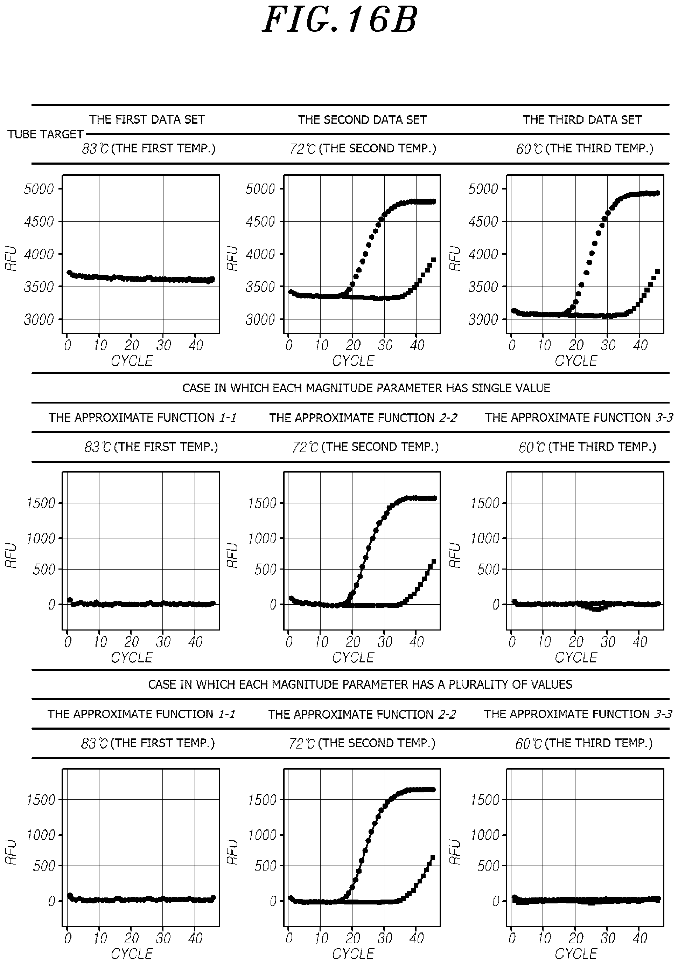

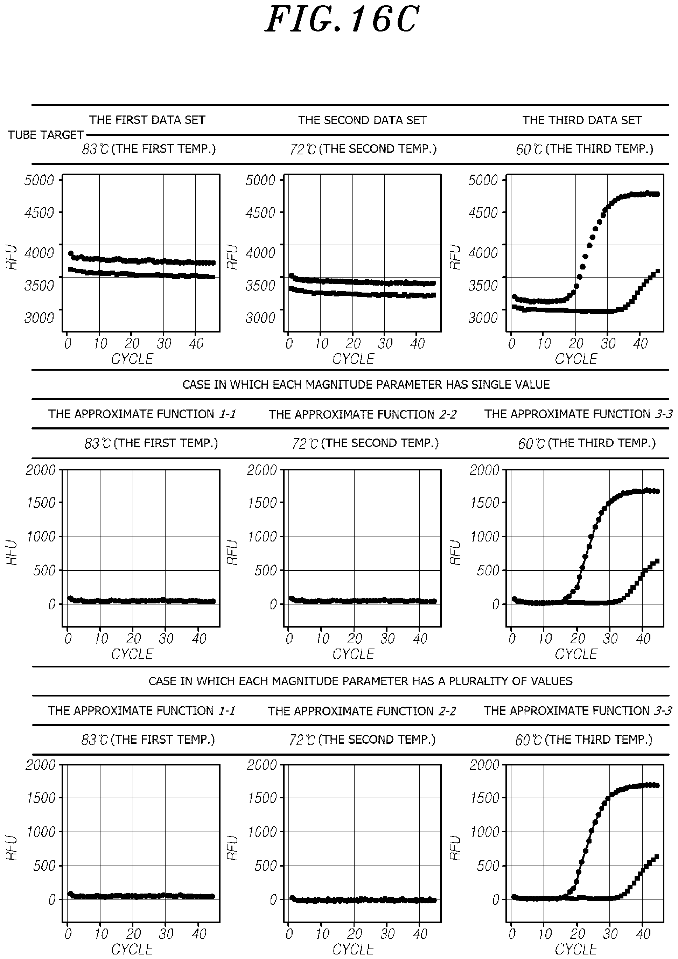

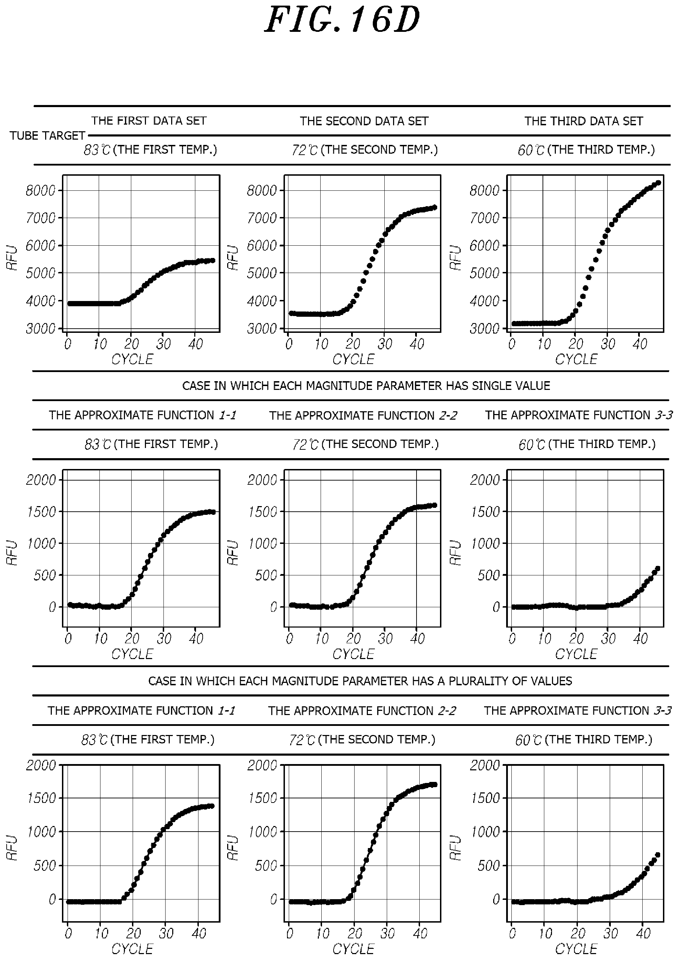

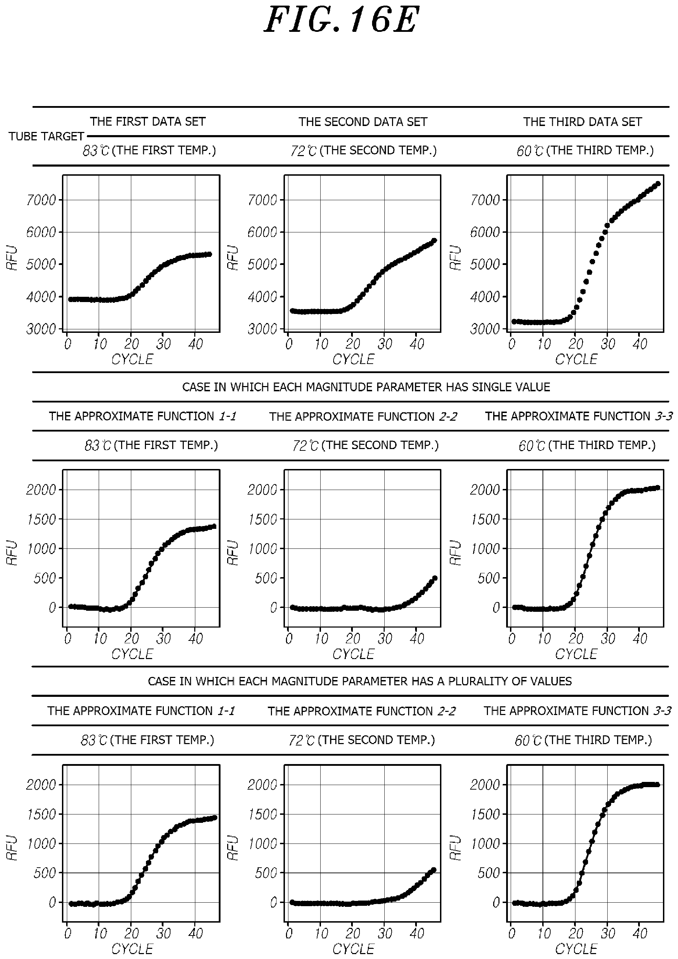

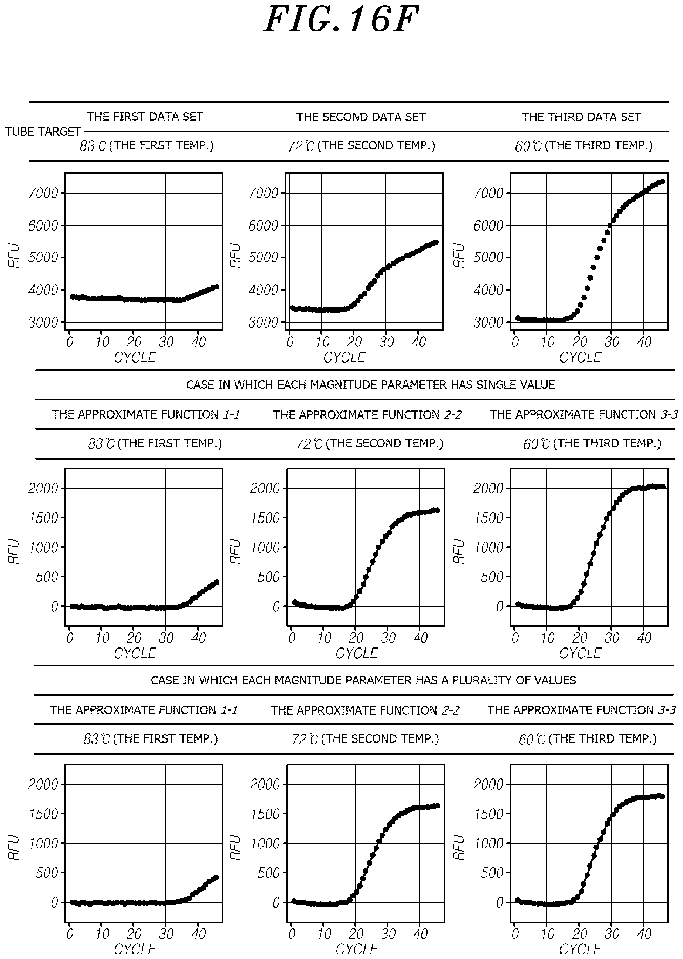

For example, the processor 240 may perform the joint-estimation on the value of the approximate function parameter *2-2* and the value of the magnitude parameter, by using an entire objective function for the first data set and the second data set. In an embodiment, the entire objective function may output an overall error determined based on a sum of: (a) a sum of a difference (e.g., the first error) between the first data set and the approximate function *1-1* at each of the plurality of cycles; and (b) a sum of a difference (e.g., the second error) between (b1) the second data set and (b2) a sum of the approximate function *2-2* and the signal value (or the approximate function *1-2*) dependent on the first target analyte at the second temperature, at each of the plurality of cycles. For example, when it is assumed that the first error follows a normal distribution of $N(0, \sigma^2_1)$ and the second error follows a normal distribution of $N(0, \sigma^2_1)$, the entire objective function may be expressed as Math Figure 16 below, but is not limited thereto.

[Math Figure 16]

$$l_{joint}(\alpha_{11}, \alpha_{22}, CR)$$

$$= \frac{1}{\sigma_1^2} \sum_{t=1}^{n} (y_{1,t} - f_{11}(\alpha_{11}, t))^2$$

$$+ \frac{1}{\sigma_2^2} \sum_{t=1}^{n} (y_{2,t} - CR * f_{11}(\alpha_{11}, t) - f_{22}(\alpha_{22}, t))^2$$

(where $l_{joint}(\alpha_{11}, \alpha_{22}, CR)$ represents the entire objective function used for the joint estimation of at least some of the approximate function parameter *1-1*, the approximate function parameter *2-2*, and the magnitude parameter. $\sigma_1$ represents the standard deviation of the first error for the plurality of cycles when the statistical sample is the first data set (specifically, a set of the first errors in the plurality of cycles). $\sigma_2$ represents the standard deviation of the second error for the plurality of cycles when the statistical sample is the second data set (specifically, a set of the second errors in the plurality of cycles))

In an embodiment, the processor 240 may apply the pre-estimated value of the approximate function parameter *1-1* to the approximate function *1-1* in the entire objective function, and may calculate the value of the approximate function parameter *2* and the value of the magnitude parameter that enable the entire objective function to be a maximum value or a minimum value. For example, the processor 240 may apply the value of the approximate function parameter *1-1* estimated in the previous step to the approximate function *1-1* included in the entire objective function, and perform the joint-estimation on the value of the approximate function parameter *2-2* and the value of the magnitude parameter so that the entire objective function is minimized. In this case, a preset value may be applied to each of the variance (e.g., $\sigma_1^2$) of the first error and the variance (e.g., $\sigma_2^2$) of the second error in the entire objective function. For example, $\sigma_1^2$ may be estimated as {n/(n-1) *(an output value of the first objective function)}, and $\sigma_2^2$ may be estimated as {n/(n-1)*(an output value of the second objective function)} or $\sigma_1^2$. As described above, in the entire objective function, a process of scaling a unit so that a normal distribution for the first error and the second error follows N(0, 1) may be added through an operation of dividing each of the variance of the first error and the variance of the second error for each of the output value of the first objective function and the output value of the second objective function.

Likewise, to find the minimum value (or maximum value) of the second objective function or a third objective function, at least one of the differentiation method, the difference method, the step difference method, the ratio approach, the linear regression analysis, and the gradient descent method of the signal value may be used. For example, an optimized values of the approximate function parameter *2-2*, $\alpha_{22}$, and the magnitude parameter CR may be derived together through the joint-estimation that finds extremum by partial differentiation for each of the approximate function parameter *2-2*, $\alpha_{22}$, and the magnitude parameter CR to be optimized in the second objective function or the third objective function.

In an embodiment, each of the value of the approximate function parameter *2* and the value of the magnitude parameter may be estimated based on the joint maximum likelihood estimation. Likewise, being based on the joint maximum likelihood estimation may be understood as a concept encompassing the joint log-likelihood estimation, the joint negative log-likelihood estimation, and the like.

3) the joint-estimation according to a second embodiment

The processor 240 according to the second exemplary embodiment may perform the joint-estimation on the value of the approximate function parameter *1-1*, the value of the approximate function parameter *2*, and the value of the magnitude parameter, by at least partially using the first data set and the second data set. In an embodiment, the processor 240 may perform the joint-estimation on the approximate function parameter *1*, the approximate function parameter *2*, and the magnitude parameter, in consideration of (a) the sum of the differences (e.g., the first error) between the first data set and the approximate function *1-1* at each of the plurality of cycles and (b) the sum of the difference (e.g., the second error) between the approximate function *2* and the sum of the signal values (or the approximate function *1-2*) dependent on the first target analyte at the second temperature.

In detail, the processor 240 may perform the joint-estimation on the value of the approximate function parameter *1-1*, the value of the approximate function parameter *2*, and the value of the magnitude parameter by using the entire objective function for the first data set and the second data set. As described above, the entire objective function may be expressed as Math Figure 16. For example, referring to Math Figure 16, the processor 240 may calculate the value of the approximate function parameter *1-1*, the value of the approximate function parameter *2*, and the value of the magnitude parameter that make the entire objective function the minimum value.

FIG. 6 is a flowchart illustrating a detailed method in which the computer device 200 according to the second embodiment performs the j oint-estimation on the value of the approximate function parameter *1-1*, the value of the approximate function parameter *2*, and the value of the magnitude parameter through the processor 240.

Referring to FIG. 6, in step S610, the processor 240 may apply any value or a pre-estimated value to any one or two parameters among the approximate function parameter *1-1*, the approximate function parameter *2*, and the magnitude parameter, and estimate the value of the remaining parameter. For example, the processor 240 may apply a randomly determined value within a preset range to each of the approximate function parameter *2-2* and the magnitude parameter in the entire objective function, and may estimate the value of the approximate function parameter *1-1* that makes the entire objective function have a minimum value. In this case, as described above, it is assumed that $\sigma_1^2 = \{n/(n-1)*(\text{an output}$ value of the first objective function)$\}$, and $\sigma_2^2 = \sigma_1^2$.

In step S620, the processor 240 may apply the estimated values of the above remaining parameter and estimate the value of the above one or two parameters. For example, the processor 240 may apply the value estimated in the previous step to the approximate function parameter *1-1* in the entire objective function, and may estimate the value of the magnitude parameter and the value of the approximate function parameter *2-2* that make the entire objective function to have a minimum value. In this case, for example, $\sigma_2^2=\{n/(n-1)*(\text{an output value of the second objective function})\}$ may be estimated.

In step S630, the processor 240 may perform at least one of steps S610 to S620 multiple times. For example, the processor 240 may apply the value of the approximate function parameter 2-2 and the value of the magnitude parameter estimated in the previous step to the entire objective function, estimate $\sigma_1^2=\{n/(n-1)*(\text{an output value of the first objective}$ function)$\}$, and estimate the value of the approximate function parameter *1-1* that makes the entire objective function have a minimum value. In addition, the processor 240 may apply the value of the approximate function parameter *1-1* estimated in the previous step to the entire objective function, estimate $\sigma_2^2=\{n/(n-1)*(\text{an output value of the second objective}$ function)$\}$, and estimate the value of the approximate function parameter *2-2* and the value of the magnitude parameter that make the entire objective function have a minimum value, and these operations may be repeatedly performed. In an embodiment, a backfitting algorithm may be used in the process of the joint-estimation on the value of the approximate function parameter *1-1*, the value of the approximate function parameter *2*, and the value of the magnitude parameter, which cause the entire objective function to be the minimum value, through such repetition.

In an embodiment, the processor 240 may repeatedly perform the operation of applying the value of the parameter estimated in the previous step to the entire objective function and estimating the values of the parameter not estimated in the previous step in such a manner that the entire objective function becomes the minimum value, until a preset condition is satisfied. Here, the preset condition may be a condition that the count of repetitions (e.g., learning rate) is equal to or greater than a preset value, and may be a condition that the output value of the entire objective function is within a predetermined value range or in a specific state (e.g., minimization, optimization), but is not limited thereto.

4) Another embodiment of the statistical model in which the background signal is considered

In the above, the operation of estimating the value of the approximate function parameter and the value of the magnitude parameter according to an embodiment of the statistical model in which the background signal is not considered has been described. However, in another embodiment, the processor 240 may perform the joint-estimation on the value of the above-described parameters based on another embodiment of the statistical model in which the background signal is considered.

Hereinafter, various examples of the data set including the background signal, signal values dependent on each of two or more target analytes estimated from the data set and the background signal used in the computer device 200 according to another embodiment will be described first with reference to FIGS. 7A and 7B.

FIG. 7A exemplarily illustrates the first data set and the second data set. In FIG. 7A, the first data set and the second data set under similar conditions to those in FIG. 2A are illustrated as an example. It can be seen that the data set further includes an exemplary background signal unlike in FIG. 2A. Likewise, such a form of the background signal is for exemplary purposes only.

FIG. 7B exemplarily illustrates signal values dependent on the presence of the first target analyte at the first temperature, the background signal at the first temperature, signal values dependent on the presence of the second target analyte at the second temperature, and the background signal at the second temperature, which are estimated from the first data set and the second data set shown in FIG. 7A. In FIG. 7B, when signal values dependent on the presence of the first target analyte at the first temperature and signal values dependent on the presence of the second target analyte at the second temperature are extracted from the first data set and the second data set, unlike FIG. 2A, the first background signal independent of the presence of the first target analyte measured at the first temperature and the second background signal independent of the presence of the first target analyte or the second target analyte measured at the second temperature, may be extracted together.

As described above, the approximate function *1* may further include the background signal approximate function *1* for approximating the first background signal, the approximate function *2* may further include the background signal approximate function *2* for approximating the second background signal, and the processor 240 may perform the joint-estimation on one or more approximate function parameters of the background signal approximate function *1* and the background signal approximate function *2* together in the above-described operation of the joint-estimation.

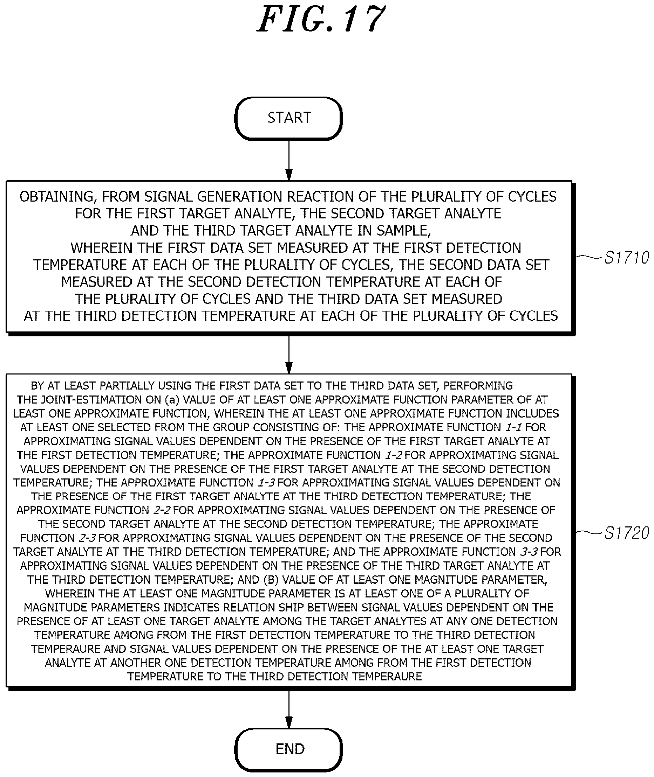

Specifically, in the statistical model, a mathematical relationship for separating the first background signal and the signal values dependent on the presence of the first target analyte from the first data set measured at the first temperature may be defined. In addition, in the statistical model, a mathematical relationship for excluding the signal values dependent on the presence of the first target analyte from the second data set measured at the second temperature and separating the signal values dependent on the presence of the second target analyte and the second background signal may be defined. For example, the statistical model may include Math Figure 17 below, and Math Figure 17 may be expressed as Math Figure 18 to 19.

[Math Figure 17]

$$\begin{pmatrix} y_{1,t} \\ y_{2,t} \end{pmatrix} = \begin{pmatrix} 1 & 0 \\ CR & 1 \end{pmatrix} \begin{pmatrix} f_{11}(\alpha_{11}, t) \\ f_{22}(\alpha_{22}, t) \end{pmatrix} + \begin{pmatrix} g_{11}(\beta_{11}, t) \\ g_{22}(\beta_{22}, t) \end{pmatrix} + \begin{pmatrix} e_1 \\ e_2 \end{pmatrix}$$

[Math Figure 18]

$$y_{1,t} = f_{11}(\alpha_{11}, t) + g_{11}(\beta_{11}, t) + e_1$$

[Math Figure 19]

$$y_{2,t} = CR * f_{11}(\alpha_{11}, t) + f_{22}(\alpha_{22}, t) + g_{22}(\beta_{22}, t) + e_2$$

Here, $\beta_{11}$ denotes the approximate function parameter of the background signal approximate function 1, and $\beta_{22}$ denotes the approximate function parameter of the background signal approximate function *2*. In addition, "$f_{11}(\alpha_{11},t)+\beta_{11}(\beta_{11},t)$" represents the approximate function *1-1* in which the background signal is considered. Specifically, $f_{11}(\alpha_{11},t)$ represents the target signal approximate function *1-1* in which the approximate function parameter of the target signal approximate function *1-1* and the cycle are input variables, and $g_{11}(\beta_{11},t)$ represents the background signal approximate function *1* at the first temperature in which the approximate function parameter of the background signal approximate function *1* and the cycle are input variables. In addition, "$f_{22}(\alpha_{22},t)+g_{22}(\beta_{22},t)$" represents the approximate function *2-2* in which the background signal is considered. Specifically, $f_{22}(\alpha_{22},t)$ represents the target signal approximate function *2-2* in which the approximate function parameter of the target signal approximate function *2-2* and the cycle are input variables, and $g_{22}(\beta_{22},t)$ represents the background signal approximate function *2* at the second temperature in which the approximate function parameter of the background signal approximate function *2* and the cycle are input variables.

For example, referring to Math Figures 17 to 19, the first data set may be defined as the sum of the target signal approximate function *1-1*, the background signal approximate function *1*, and the first error. The second data set may be defined as the sum of: the product of the target signal approximate function *1-1* and the magnitude parameter; the target signal approximate function *2-2*; the background signal approximate function *2*; and the second error. Similarly, according to an embodiment, when the approximate function *1* is the approximate function *1-2* that approximates the signal value at the second temperature, as a modified form of Math Figures 17 to 19, the first data set may be defined as the sum of: the product of an inverse of the magnitude parameter and the target signal approximate function *1-2*; the background signal approximate function *1*; and the first error, and the second data set may be defined as a sum of: the target signal approximate function *1-2*; the target signal approximate function *2-2*; the background signal approximate function *2*; and the second error. The following objective functions may be modified in a similar manner.

The processor 240 may perform the joint-estimation on the values of the above-described parameters based on another embodiment of the statistical model in which the background signal is considered. In a similar manner to the above-described embodiments, another embodiment of the statistical model may include one or more objective functions for estimating the optimized value of the parameter, and the processor 240 may obtain the value of the parameter that causes the objective function to be the maximum value or the minimum value and calculate the value as the optimized estimate of the parameter. Likewise, hereinafter, various embodiments in which the joint-estimation for values of the parameters is performed by using various objective functions will be described.

5) the joint-estimation according to a third embodiment in which the background signal is considered

In the j oint-estimation performed according to the third embodiment, the value of at least one parameter of the background signal approximate function *1* and the background signal approximate function 2 may be further joint-estimated.

FIG. 8 is a flowchart illustrating a method in which the processor 240 performs the joint-estimation on the value of the approximate function parameter *2* and the value of the magnitude parameter in consideration of the background signal, according to the third embodiment. FIG. 8 may be understood with reference to the embodiments described above with reference to FIGS. 1 to 7, and a description of overlapping or similar technical principles will be omitted.

Referring to FIG. 8, in step S810, the processor 240 may estimate the value of the parameter of the target signal approximate function *1-1* for signal values dependent on the first target analyte included in the approximate function *1-1* and the value of the parameter of the background signal approximate function *1-1* for the first background signal by using the first data set. In a similar manner to the first embodiment, each of the value of the parameter of the target signal approximate function *1-1* and the value of the parameter of the background signal approximate function *1-1* may be joint-estimation in such a manner that the sum of the differences between the first data set and the approximate function *1-1* for which the first background signal is considered at each of the plurality of cycles is minimized.

In detail, the processor 240 may estimate the value of the parameter of the target signal approximate function *1-1* and the value of a parameter of the background signal approximate function *1-1* by using the first objective function for the first data set. For example, the first objective function may be expressed as Math Figure 20 below, assuming that the first error follows a normal distribution of $N(0, \sigma_1^2)$. The processor 240 may calculate the value of the parameter of the target signal approximate function *1-1* and the value of a parameter of the background signal approximate function *1-1*, in such a manner that the first objective function has a minimum value.

[Math Figure 20]

$$ l_1(\alpha_{11}, \beta_{11}) = \frac{1}{n} \sum_{t=1}^{n} (y_{1,t} - f_{11}(\alpha_{11}, t) - g_{11}(\beta_{11}, t))^2 $$

In step S820, the processor 240 may perform the joint-estimation on the value of the parameter of the target signal approximate function *2* for signal values dependent on the second target analyte included in the approximate function *2*, the value of the parameter of the background signal approximate function *2* for the second background signal, and the value of the magnitude parameter, by at least partially using the estimated value of the parameter of the target signal approximate function *1-1*, the estimated value of the parameter of the background signal approximate function *1-1*, and the second data set. In a manner similar to the first embodiment, each of the value of the parameter of the target signal approximate function *2*, the value of the parameter of the background signal approximate function *2*, and the value of the magnitude parameter may be joint-estimated in such a manner that a difference at each of the plurality of cycles between (a) the second data set; and (b) the sum of the approximate function *2* and the signal values (or the approximate function *1-2*) dependent on the first target analyte at the second temperature; is minimized.

As a specific embodiment, the processor 240 may perform the joint-estimation on the value of the parameter of the target signal approximate function *2-2*, the value of the parameter of the background signal approximate function *2-2*,

and the value of the magnitude parameter, by using the second objective function for the second data set. For example, the second objective function may be expressed as Math Figure 21 below, assuming that the second error follows a normal distribution of N(0, $\sigma_2{}^2$). The processor 240 may calculate the value of the parameter of the target signal approximate function 2-2, the value of the parameter of the background signal approximate function 2-2, and the value of the magnitude parameter, which enable the second objective function to have a minimum value.

[Math Figure 21]

$$l_2(\alpha_{22}, \beta_{22}, CR) = \frac{1}{n} \sum_{t=1}^{n} (y_{2,t} - CR * f_{11}(\alpha_{11}, t) - f_{22}(\alpha_{22}, t) - g_{22}(\beta_{22}, t))^2$$

As another specific embodiment, the processor 240 may perform the joint-estimation on the value of the parameter of the target signal approximate function 2, the value of the parameter of the background signal approximate function 2, and the value of the magnitude parameter, by using the entire objective function for the first data set and the second data set. For example, the entire objective function may be expressed as Math Figure 22 below, assuming that the first error follows a normal distribution of N(0, $\sigma_1{}^2$) and the second error follows a normal distribution of N(0, $\sigma_2{}^2$). The processor 240 may apply the values of the parameters previously estimated in the previous step to the approximate function 1-1 in the entire objective function, and may calculate the value of the parameter of the target signal approximate function 2-2, the value of the parameter of the background signal approximate function 2-2, and the value of the magnitude parameter, which enable the entire objective function to have a minimum value.

[Math Figure 22]

$$l_{joint}(\alpha_{11}, \alpha_{22}, \beta_{11}, \beta_{22}, CR)$$

$$= \frac{1}{\sigma_1{}^2} \sum_{t=1}^{n} (y_{1,t} - f_{11}(\alpha_{11}, t) - g_{11}(\beta_{11}, t))^2$$

$$+ \frac{1}{\sigma_2{}^2} \sum_{t=1}^{n} (y_{2,t} - CR * f_{11}(\alpha_{11}, t) - f_{22}(\alpha_{22}, t) - g_{22}(\beta_{22}, t))^2$$

6) the joint-estimation according to a fourth embodiment in which the background signal is considered

In the joint-estimation performed according to the fourth embodiment, the value of at least one parameter among the background signal approximate function 1 and the background signal approximate function 2 may be further joint-estimated.

FIG. 9 is a flowchart illustrating an example of a method in which the processor 240 performs the joint-estimation on the value of the approximate function parameter 1-1, the value of the approximate function parameter 2, and the value of the magnitude parameter in consideration of the background signal, according to a fourth embodiment. Likewise, FIG. 9 may be understood with reference to the embodiments described above with reference to FIGS. 1 to 8.

Referring to FIG. 9, in step S910, the processor 240 may apply an arbitrary value or a pre-estimated value to some parameters among the parameter of the target signal approximate function 1-1, the parameter of the background signal approximate function 1-1, the parameter of the target signal approximate function 2, the parameter of the background signal approximate function 2, and the magnitude parameter, and may estimate values of the remaining parameters. For example, in a manner similar to the second embodiment, the processor 240 may apply a randomly determined value within a preset range to each of the value of the parameter of the target signal approximate function 2-2, the value of the parameter of the background signal approximate function 2-2, and the magnitude parameter in the entire objective function, and may perform the joint-estimation on the value of the parameter of the target signal approximate function 1-1 and the value of the parameter of the background signal approximate function 1-1 in such a manner that the entire objective function has a minimum value. For example, the entire objective function may be expressed as Math Figure 22, and similarly, it is assumed that $\sigma_1{}^2$ = {n/(n-1)*(an output value of the first objective function)}, and $\sigma_2{}^2 = \sigma_1{}^2$.

In step S920, the processor 240 may apply the estimated values of the above remaining parameters and estimate the values of the above some parameters. For example, the processor 240 may apply the value estimated in the previous step to the parameter of the target signal approximate function *1-1* and the background signal approximate function *1-1* in the entire objective function, and perform the joint-estimation on the value of the parameter of the target signal approximate function *2-2*, the value of the parameter of the background signal approximate function *2-2*, and the value of the magnitude parameter in such a manner that the entire objective function becomes the minimum value. Likewise, it is assumed that $\sigma_2^2$ = {n/(n-1)*(an output value of the second objective function)}.

In step S930, the processor 240 may perform at least one of steps S910 to S920 multiple times. In an embodiment, the processor 240 may repeatedly perform the operation of applying the values of the parameter estimated in the previous step to the entire objective function and estimating the values of the parameter not estimated in the previous step in such a manner that the entire objective function becomes the minimum value until the above-described preset condition is satisfied.


7) Simulation results according to an embodiment of the present invention

FIGS. 10A to 10D illustrate simulation results according to the third embodiment in which the background signal is considered as a constant. Specifically, according to the third embodiment, the results are illustrated in which the processor 240 estimates the values of the parameters of the target signal approximate function *1-1* and the background signal approximate function *1-1* by using the first data set, and then performs the joint-estimation on the values of the parameters of the target signal approximate function *2-2* and the background signal approximate function *2-2* and the values of the magnitude parameter by using the estimated values and the second data set. In addition, Math Figure 6 is used for the target signal approximate functions *1-1* and *2-2*, Math Figure 7 is used for the background signal approximate functions *1-1* and *2-2* as the background signal is considered as a constant, and the parameter value in each background signal approximate function was set to be estimated as a value corresponding to the estimate of $\alpha_2$, which is the parameter indicating signal values in the baseline region. In addition, Math Figure 20 is used for the joint-estimation on the value of the parameter of the approximate function *1-1*, and Math Figure 22 is used for the joint-estimation on the value of the parameter of the approximate function *2-2* and the value of the magnitude parameter. In addition, a predetermined statistically calculated value range (e.g., 0.7 to 1.3) is set in the magnitude parameter.

More specifically, the first data set and the second data set in the case corresponding to each of tubes 1 to 4 shown in FIG. 7 are shown in the upper graph of each of FIGS. 10A to 10D. In addition, the bottom graph shows simulation results for the approximate function *1-1* and the approximate function *2-2* to which the values of the parameters estimated from the corresponding first data set and second data set according to the third embodiment are applied. In the bottom graph, a solid line graph shows output values of the approximate function *1-1* and the approximate function *2-2* corresponding to an estimate (including signal values dependent on each target analyte and the background signal), and a dotted line graph shows signal values corresponding to raw data of each target analyte in which an error is included.

FIG. 10A illustrates a case in which only the first target analyte is present among the first target analyte and the second target analyte in the sample, and the values of the parameters $\alpha_{11-1}$ to $\alpha_{11-5}$ of the target signal approximate function *1-1* (see Math Figure 6), the values of the parameters $\alpha_{22-1}$ to $\alpha_{22-5}$ of the target signal approximate function *2-2* (see Math Figure 6), the values of the parameters $\beta_{1-0}$ of the background signal approximate function *1-1* (see Math Figure 7), the values of the parameters $\beta_{2-0}$ of the background signal approximate function *2-2* (see Math Figure 7), and the values of the magnitude parameter, which are estimated to minimize the total error according to the third embodiment, are shown in Table 1 below. According to the estimated values of the parameters, the approximate function *1-1* and the approximate function *2-2* may be visualized as shown in the lower graph of FIG. 10A, and it can be seen that each approximate function is appropriately estimated from the first data set and the second data set. In this case, it can be seen that the approximate function *2-2* has relatively little amplification amount compared to the approximate function *1-1*, but in detail, it may appear as an S-shaped curve according to the characteristics of the sigmoid function.

[Table 1]

| parameter | $\alpha_{11-1}$ | $\alpha_{11-2}$ | $\alpha_{11-3}$ | $\alpha_{11-4}$ | $\alpha_{11-5}$ |
|---|---|---|---|---|---|
| estimate | -0.3748 | 3800.7797 | 3096.9367 | 18.7310 | 2.2438 |
| parameter | $\alpha_{22-1}$ | $\alpha_{22-2}$ | $\alpha_{22-3}$ | $\alpha_{22-4}$ | $\alpha_{22-5}$ |
| estimate | -0.0888 | 3632.4944 | 184.6181 | 1.3826 | 9.9872 |
| parameter | $\beta_{1-0}$ | $\beta_{2-0}$ | CR | | |
| estimate | 3800.7797 | 3632.4944 | 1.0778 | | |

FIG. 10B illustrates a case in which only the second target analyte is present among the first target analyte and the second target analyte in the sample, and similarly, the values of the parameters estimated to minimize the overall error according to the third embodiment are shown in Table 2 below. Similarly, each approximate function to which the estimated values of the parameters are applied may be visualized as shown in the lower graph of FIG. 10B, and it can be seen that the values are appropriately estimated from the first data set and the second data set. Similarly, the approximate function *1* may be amplified in a relatively small amount compared to the approximate function *2*, but in detail, it may appear as an S-shaped curve according to the characteristics of the sigmoid function.

[Table 2]

| parameter | $\alpha_{11-1}$ | $\alpha_{11-2}$ | $\alpha_{11-3}$ | $\alpha_{11-4}$ | $\alpha_{11-5}$ |
|---|---|---|---|---|---|
| estimate | -0.0120 | 3324.8089 | 1236.0936 | -34.5013 | 1.9741 |
| parameter | $\alpha_{22-1}$ | $\alpha_{22-2}$ | $\alpha_{22-3}$ | $\alpha_{22-4}$ | $\alpha_{22-5}$ |
| estimate | -0.5563 | 3055.867 | 3073.1690 | 20.0603 | 1.1632 |
| parameter | $\beta_{1-0}$ | $\beta_{2-0}$ | CR | | |
| estimate | 3324.8089 | 3055.867 | 1.2167 | | |

FIG. 10C illustrates a case in which the first target analyte and the second target analyte in the sample are present together with similar high concentrations, and similarly, the values of the parameters estimated to minimize the overall error according to the third embodiment are shown in Table 3 below. Similarly, each approximate function to which the estimated values of the parameters are applied may be visualized as shown in the lower graph of FIG. 10C, and although the value of the magnitude parameter is on the boundary of the maximum range, it can be seen that each approximate function is separated from the first data set and the second data set to a reasonable degree.

[Table 3]

| parameter | $\alpha_{11-1}$ | $\alpha_{11-2}$ | $\alpha_{11-3}$ | $\alpha_{11-4}$ | $\alpha_{11-5}$ |
|---|---|---|---|---|---|
| estimate | -0.3283 | 3785.2543 | 3079.8843 | 18.0131 | 2.8270 |
| parameter | $\alpha_{22-1}$ | $\alpha_{22-2}$ | $\alpha_{22-3}$ | $\alpha_{22-4}$ | $\alpha_{22-5}$ |
| estimate | -0.5220 | 3621.7654 | 2244.2201 | 19.6574 | 1.3194 |
| parameter | $\beta_{1-0}$ | $\beta_{2-0}$ | CR | | |
| estimate | 3785.2543 | 3621.7654 | 1.3 | | |

FIG. 10D illustrates a case in which the first target analyte is present with a relatively high concentration and the second target analyte is present with a relatively low concentration together in the sample, and similarly, the values of the parameters estimated to minimize the overall error according to the third embodiment are shown in Table 4 below. Similarly, each approximate function to which the estimated values of the parameters are applied may be visualized as shown in the lower graph of FIG. 10D, and it can be seen that each approximate function is appropriately separated from the first data set and the second data set.

[Table 4]

| parameter | $\alpha_{11-1}$ | $\alpha_{11-2}$ | $\alpha_{11-3}$ | $\alpha_{11-4}$ | $\alpha_{11-5}$ |
|---|---|---|---|---|---|
| estimate | -0.3525 | 3996.2212 | 3329.1281 | 17.9658 | 2.8845 |
| parameter | $\alpha_{22-1}$ | $\alpha_{22-2}$ | $\alpha_{22-3}$ | $\alpha_{22-4}$ | $\alpha_{22-5}$ |
| estimate | -0.3374 | 3801.8109 | 3039.4187 | 34.2169 | 4.7607 |
| parameter | $\beta_{1-0}$ | $\beta_{2-0}$ | CR | | |
| estimate | 3996.2212 | 3801.8109 | 1.1056 | | |

As described above, according to an embodiment of the present disclosure, the value of the approximate function parameter *1-1*, the value of the approximate function parameter *2-2*, and the value of the magnitude parameter in which the background signal is considered may be joint-estimated by using the first data set and the second data set, and it can be seen that the algorithm according to the present disclosure operates well as shown in FIG. 10.

8) Simulation result according to another embodiment of the present invention

FIGS. 11A to 11D illustrate simulation results according to the third embodiment in which the background signal is considered as a first-order function, and FIGS. 12A to 12C illustrate simulation results according to the third embodiment in which the background signal is considered as a second-order function. It was simulated under similar conditions to the simulation described above. In the simulation of FIG. 11, Math Figure 8 is used for each background signal approximate function as the background signal is considered as the first-order function, and in the simulation of FIG. 12, Math Figure 10 is used for each background signal approximate function as the background signal is considered as the second-order function.

In detail, in the graphs of the upper end of FIGS. 11A to 11C and FIGS. 12A to 12C, the first data set and the second data set in cases corresponding to tubes 1, 2, and 4 illustrated in FIG. 7 are illustrated. In addition, the middle and bottom graphs show simulation results for the signal values dependent on the presence of the first target analyte at the first temperature (the target signal approximate function *1-1*), the signal values dependent on the presence of the second target analyte at the second temperature (the target signal approximate function *2-2*), the first background signal (the background signal approximate function *1-1*), and the second background signal (the background signal approximate function *2-2*) to which the values of the parameters estimated from the corresponding first data set and second data set are applied according to the simulation. Similarly, in the middle graph, the solid line graph shows output values of the approximate function *1-1* and the approximate function *2-2* corresponding to estimates, and the dotted line graph shows signal values corresponding to raw data of each target analyte in which the background signal is removed and the error is reflected.

Referring to FIGS. 11 and 12, in various cases in which the background signal is considered as a polynomial, it can be seen that the signal value dependent on the presence of each target analyte and the background signal at each temperature are appropriately estimated from the first data set and the second data set.

9) Another embodiment in which the magnitude parameter has two or more values

In the above, the operation of estimating the value of the approximate function parameter and the value of the magnitude parameter according to an exemplary embodiment in which the magnitude parameter has one value has been described. However, the processor 240 may perform the above-described joint-estimation according to another embodiment in which the magnitude parameter has two or more values.

According to another embodiment, the magnitude parameter may have two or more values. As a specific example, the magnitude parameter may have two or more values applied to each of two or more cycle sections selected from the plurality of cycles. Here, each cycle section may be a continuous section or a discontinuous section. In an example in which the cycle section is the continuous section, the magnitude parameter may have, for example, three values applied to each of a first cycle section (e.g., 1 to 10), a second cycle section (e.g., 11 to 30), and a third cycle section (e.g., 31 to 45), which are continuously divided from a preset cycle section (e.g., 1 to 45). In an example in which the cycle section is the discontinuous section, the magnitude parameter may have two values applied to each of the first cycle sections (e.g., 10 to 15, 30 to 33, . . . , 44) and the second cycle sections (e.g., 16 to 29, 34 to 39, ... , 45) which are continuously or discontinuously divided from among the preset cycle sections (e.g., 1 to 45). According to an embodiment, the cycle section may be selected from a part of the entire cycle period, or may be selected from the remaining period excluding one or more preset periods (e.g., 1 to 5) of the entire cycle period.

According to another embodiment described above, in the above-described joint-estimation process, the value of the magnitude parameter may be joint-estimated for each of two or more cycle sections selected from the plurality of cycles. For example, in the process of performing the joint-estimation on the value of the approximate function parameter *2-2* and the value of the magnitude parameter or in the process of performing the joint-estimation on the value of the approximate function parameter *1-1*, the value of the approximate function parameter *2-2*, and the value of the magnitude parameter according to at least one of the above-described embodiments, the value of the magnitude parameter may be estimated for each of two or more cycle sections. For example, a count of values of the magnitude parameter is 2, and there are different CR values corresponding to each of cycle sections 1 and 2, and each CR value may be estimated so that the total error is minimized by applying the CR for each cycle section in the above-described joint-estimation process.

In another embodiment, the magnitude parameter may have a plurality of values for the plurality of cycles. For example, the magnitude parameter may be processed in the form of a set of data points, each of which contains values for the cycle and the magnitude parameter, or in the form of a two-dimensional array where the cycle is a row and the magnitude parameter is a column. As another example, the magnitude parameter may be processed in the form of a one-dimensional array that is a bundle of values of the magnitude parameter arranged according to the corresponding cycle order.

As a specific example, in the above-described joint-estimation process, the value of the magnitude parameter may be joint-estimated for each of the plurality of cycles. For example, in Math Figures 11, 15, and 16 according to the first embodiment, in Math Figures 11 and 16 according to the second embodiment, in Math Figures 17, 21, and 22 according to the third embodiment, or in Math Figures 17 and 22 according to the fourth embodiment, CR included in the corresponding Math Figures may be replaced with CRt representing CR in each of the plurality of cycles t. For example, the magnitude parameter is a set of CR values corresponding to n (e.g., n=45) per-cycle values (e.g., $CR_t=\{1.22, 1.10, ... , 1.15\}$), and the CR value per cycle may be estimated in such a manner that the overall error is minimized in the above-described joint-estimation process.

In another embodiment, the magnitude parameter may be one or more parameters included in a magnitude approximate function defined in the form of a polynomial function. For example, the magnitude parameter may be a coefficient (e.g., a, b) of the magnitude approximate function (e.g., y=ax+b) that is the first-order function, the CR included in the above-described Math Figures may be replaced with the corresponding magnitude approximate function, and in the above-described joint-estimation process, the corresponding coefficient values may be estimated as values of the corresponding magnitude parameter together with the values of the approximate function parameter *1* and/or the approximate function parameter *2*.

FIGS. 14A to 14D illustrate simulation results in which the value of the magnitude parameter is estimated for each of the plurality of cycles according to another embodiment described above. The simulation process has been simulated in the same or similar manner as described above with reference to FIGS. 10A to 10D, and the upper graph and the lower graph of each of FIGS. 14A to 14D may also be interpreted in the same manner. In addition, an embodiment in which the background signal is a constant was applied for convenience in the corresponding simulation process. Similarly, when the background signal is the first-order function or the second-order function, simulated results were obtained appropriately.

FIG. 14A illustrates a case in which only the first target analyte is present, and the values of the parameters estimated to minimize the overall error according to the above-described embodiment are shown in Table 5 below, and in particular, the values of the magnitude parameters are estimated for each of the plurality of cycles. Each approximate function to which the estimated values of the parameters are applied may be visualized as shown in the lower graph.

[Table 5]

| parameter | $\alpha_{11-1}$ | $\alpha_{11-2}$ | $\alpha_{11-3}$ | $\alpha_{11-4}$ | $\alpha_{11-5}$ |
|---|---|---|---|---|---|
| estimate | -0.3813 | 3782.0864 | 3114.4171 | 19.2229 | 19.2976 |
| parameter | $\alpha_{22-1}$ | $\alpha_{22-2}$ | $\alpha_{22-3}$ | $\alpha_{22-4}$ | $\alpha_{22-5}$ |
| estimate | -0.4060 | 3629.8903 | 0.0667 | 17.7326 | 0.5737 |
| parameter | $\beta_{1-0}$ | $\beta_{2-0}$ | | | |
| estimate | 3782.0864 | 3629.8903 | | | |
| parameter | $CR_t$ (t=1~45) | | | | |
| estimate | {1.2077, 1.1217, 1.0944, 1.1002, 1.1503, 1.1222, 1.2832, 1.0606, 1.0770, 1.1037, 1.1228, 1.0805, 1.0616, 1.0661, 1.1975, 1.0945, 1.0699, 1.0696, 1.0765, 1.0864, 1.0881, 1.0920, 1.0949, 1.0946, 1.0930, 1.1004, 1.0953, 1.0969, 1.1025, 1.0983, 1.1016, 1.1004, 1.1031, 1.1097, 1.1068, 1.1084, 1.1131, 1.1155, 1.1130, 1.1154, 1.1157, 1.1166, 1.1177, 1.1212, 1.1231} | | | | |

FIG. 14B illustrates a case in which only the second target analyte is present, and the values of the parameters estimated to minimize the total error according to the above-described embodiment are as shown in Table 6 below, and each approximate function to which the estimated values of the parameters are applied may be visualized as shown in the lower graph.

[Table 6]

| parameter | $\alpha_{11-1}$ | $\alpha_{11-2}$ | $\alpha_{11-3}$ | $\alpha_{11-4}$ | $\alpha_{11-5}$ |
|---|---|---|---|---|---|
| estimate | -0.0176 | 3191.4973 | 1107.8981 | 0.0008 | 0.9374 |
| parameter | $\alpha_{22-1}$ | $\alpha_{22-2}$ | $\alpha_{22-3}$ | $\alpha_{22-4}$ | $\alpha_{22-5}$ |
| estimate | -0.5270 | 2944.4611 | 3096.2790 | 20.2336 | 1.0846 |
| parameter | $\beta_{1-0}$ | $\beta_{2-0}$ | | | |
| estimate | 3191.4973 | 2944.4611 | | | |
| parameter | $CR_t$ (t=1~45) | | | | |

(continued)

| estimate | {1.2316, 1.1727, 1.0773, 1.1555, 1.1538, 1.1438, 1.1884, 1.1396, 1.1544, 1.1417, 1.1263, 1.1397, 1.1222, 1.1093, 1.0709, 1.0540, 1.0599, 1.0666, 1.0799, 1.1179, 1.1312, 1.1894, 1.2080, 1.1697, 1.1540, 1.1574, 1.1496, 1.1320, 1.1347, 1.1327, 1.1419, 1.1424, 1.1209, 1.1308, 1.0948, 1.0874, 1.1298, 1.1178, 1.1328, 1.0982, 1.1178, 1.1210, 1.1108, 1.1085, 1.0957} |
|---|---|

FIG. 14C illustrates a case in which the first target analyte and the second target analyte are present together with similar high concentrations, the values of the parameters estimated to minimize the total error according to the above-described embodiment are as shown in Table 7 below, and each approximate function to which the estimated values of the parameters are applied may be visualized as shown in the lower graph.

[Table 7]

| parameter | $\alpha_{11\text{-}1}$ | $\alpha_{11\text{-}2}$ | $\alpha_{11\text{-}3}$ | $\alpha_{11\text{-}4}$ | $\alpha_{11\text{-}5}$ |
|---|---|---|---|---|---|
| estimate | -0.3355 | 3762.3107 | 3100.8932 | 18.8212 | 2.2680 |
| parameter | $\alpha_{22\text{-}1}$ | $\alpha_{22\text{-}2}$ | $\alpha_{22\text{-}3}$ | $\alpha_{22\text{-}4}$ | $\alpha_{22\text{-}5}$ |
| estimate | -0.3826 | 3600.6706 | 2892.1740 | 19.5341 | 1.7422 |
| parameter | $\beta_{1\text{-}0}$ | $\beta_{2\text{-}0}$ | | | |
| estimate | 3762.3107 | 3600.6706 | | | |
| parameter | $CR_t$ (t=1~45) | | | | |
| estimate | {1.0816, 1.2632, 1.0659, 1.0921, 1.1986, 1.1075, 1.0542, 1.2779, 1.2123, 1.1832, 1.2878, 1.0837, 1.2410, 1.0743, 1.1327, 1.0797, 1.1188, 1.1776, 1.2655, 1.2924, 1.2937, 1.2777, 1.2491, 1.2089, 1.1746, 1.1583, 1.1241, 1.1104, 1.1043, 1.0930, 1.0879, 1.0837, 1.0868, 1.0832, 1.0830, 1.0844, 1.0868, 1.0915, 1.0912, 1.0926, 1.0977, 1.0988, 1.0986, 1.1011, 1.1009} | | | | |

FIG. 14D illustrates a case in which the first target analyte is present together with a relatively high concentration and the second target analyte is present together with a relatively low concentration, and the values of the parameters estimated to minimize the total error according to the above-described embodiment are shown in Table 8 below, and each approximate function to which the estimated values of the parameters are applied may be visualized as shown in the lower graph.

[Table 8]

| parameter | $\alpha_{11\text{-}1}$ | $\alpha_{11\text{-}2}$ | $\alpha_{11\text{-}3}$ | $\alpha_{11\text{-}4}$ | $\alpha_{11\text{-}5}$ |
|---|---|---|---|---|---|
| estimate | -0.3629 | 3963.4209 | 3359.4326 | 18.9660 | 2.1515 |
| parameter | $\alpha_{22\text{-}1}$ | $\alpha_{22\text{-}2}$ | $\alpha_{22\text{-}3}$ | $\alpha_{22\text{-}4}$ | $\alpha_{22\text{-}5}$ |
| estimate | -0.2528 | 3785.8577 | 3646.6662 | 37.6415 | 2.0981 |
| parameter | $\beta_{1\text{-}0}$ | $\beta_{2\text{-}0}$ | | | |
| estimate | 3963.4209 | 3785.8577 | | | |
| parameter | $CR_t$ (t=1~45) | | | | |
| estimate | | | {1.2747, 1.2757, 1.0511, 1.1271, 1.1981, 1.2575, 1.2662, 1.0766, 1.2023, 1.1651, 1.1770, 1.1661, 1.2298, 1.2983, 1.1815, 1.1272, 1.0663, 1.0616, 1.1499, 1.0811, 1.0983, 1.1048, 1.0936, 1.0939, 1.0876, 1.0876, 1.0860, 1.0837, 1.0840, 1.0725, 1.0736, 1.0586, 1.0599, 1.0546, 1.0507, 1.0533, 1.0728, 1.1071, 1.1281, 1.1543, 1.1663, 1.1532, 1.1371, 1.1218, 1.1040} | | |

In the above, the embodiment in which joint-estimation is performed on the parameter value of the first parameter group including the approximate function parameter included in the approximate function *1-1* and the approximate function *2* and the magnitude parameter has been described. However, as described above, according to another embodiment, the joint-estimation may be performed on the parameter value of the second parameter group including the approximate function parameter included in the approximate function *1-2* and the approximate function *2* and the magnitude parameter.

For example, the statistical model may include at least one of Math Figures 23 to 27 below. Here, $\alpha_{12}$ represents the approximate function parameter *1-2*, and $f_{12}(\alpha_{12},t)$ represents the approximate function *1-2* (specifically, the target signal approximate function *1-2*) in which the approximate function parameter *1-2* and the cycle are input variables. In addition, the first objective function, the second objective function, and the entire objective function may be expressed as in Math Figures 28, 29, and 30 below. As described above, it can be seen that the joint-estimation process by using at least some of Math Figures 23 to 30 may also be performed in the same or similar manner as in the above-described embodiments.

[Math Figure 23]

$$\begin{pmatrix} y_{1,t} \\ y_{2,t} \end{pmatrix} = \begin{pmatrix} \dfrac{1}{CR} & 0 \\ 1 & 1 \end{pmatrix} \begin{pmatrix} f_{12}(\alpha_{12},\ t) \\ f_{22}(\alpha_{22},\ t) \end{pmatrix} + \begin{pmatrix} e_1 \\ e_2 \end{pmatrix}$$

[Math Figure 24]

$$y_{1,t} = \frac{1}{CR} * f_{12}(\alpha_{12},\ t) + e_1$$

[Math Figure 25]

$$y_{2,t} = f_{12}(\alpha_{12},\ t) + f_{22}(\alpha_{22},\ t) + e_2$$

[Math Figure 26]

$$f_{12}(\alpha_{12},\ t) = CR * f_{11}(\alpha_{11},\ t)$$

[Math Figure 27]

$$f_{12}(\alpha_{12},\ t) = CR * y_{1,t}$$

[Math Figure 28]

$$l_1(\alpha_{12}) = \frac{1}{n}\sum_{t=1}^{n}(y_{1,t} - \frac{1}{CR} * f_{12}(\alpha_{12},\ t))^2$$

[Math Figure 29]

$$l_2(\alpha_{22},\ CR) = \frac{1}{n}\sum_{t=1}^{n}(y_{2,t} - f_{12}(\alpha_{12},\ t) - f_{22}(\alpha_{22},\ t))^2$$

[Math Figure 30]

$$l_{joint}(\alpha_{12}, \alpha_{22}, CR)$$

$$= \frac{1}{\sigma_1{}^2} \sum_{t=1}^{n} (y_{1,t} - \frac{1}{CR} * f_{12}(\alpha_{12}, t))^2$$

$$+ \frac{1}{\sigma_2{}^2} \sum_{t=1}^{n} (y_{2,t} - f_{12}(\alpha_{12}, t) - f_{22}(\alpha_{22}, t))^2$$

Various embodiments of the operation in which the processor 240 performs the joint-estimation on the value of the approximate function parameter and the value of the magnitude parameter have been described above. As described above, the processor 240 may separate signal values dependent on the presence of the first target analyte at the first temperature and signal values dependent on the presence of the second target analyte at the second temperature, from the first data set and the second data set.

The processor 240 may determine a cycle threshold (Ct) used to detect each of the first target analyte and the second target analyte in the sample by using the joint-estimation result. In detail, the processor 240 may determine a first cycle threshold for the first target analyte by using the estimated value of the approximate function parameter *1*, and may determine a second cycle threshold for the second target analyte by using the estimated value of the approximate function parameter 2.

In an embodiment, the cycle threshold may be a count of the cycles at a point where signal values (e.g., RFU) dependent on each target analyte exceeds a predetermined reference signal value (e.g., 200), but is not limited thereto. For example, in FIG. 12C, the first cycle threshold for the first target analyte is 38.0808, and the second cycle threshold for the second target analyte is 29.2071. According to an embodiment, the cycle threshold may be broadly interpreted as a meaning including a signal value, the count of the cycles, or a measured value of a specific parameter when a result of a predetermined analysis (e.g., a derivative, etc.) derived from the corresponding approximate function satisfies a predetermined condition. For example, the cycle threshold may refer to the count of cycles in which the first-order, the second-order, or the multi-order derivative of the approximate function having the estimated value of the approximate function parameter is calculated and then the maximum value of the calculated derivative is obtained. As another example, the cycle threshold may be interpreted as a meaning encompassing the terms CP (cross point), TOP (take-off point), or CQ (quantization cycle) used in the art.

The processor 240 may detect each of the first target analyte and the second target analyte in the sample by using the joint-estimation result. In an embodiment, the processor 240 may determine whether each of the first target analyte and the second target analyte in the sample is positive or negative by at least partially using the first cycle threshold and the second cycle threshold. For example, the processor 240 may determine whether each target analyte is present depending on whether each cycle threshold is within a preset range (e.g., 0 to 40). In another embodiment, the processor 240 may determine whether each target analyte is present in the sample based on whether a magnitude of the cycle threshold, an amount of change in the signal values dependent on each target analyte during the entire cycles, a slope of cycles corresponding to signal values dependent on each target analyte in the exponential region, and the like satisfy a predetermined condition, and output a determination result including whether each target analyte is positive or negative and the cycle threshold. In an embodiment, the determination result may further include the joint-estimation result, and for example, function information used for the estimation, the estimated values of the parameters, and the like may be provided together.

In an embodiment, when detecting each of the first target analyte and the second target analyte in the sample, the processor 240 may use the approximate function *1* that approximates the signal values at the first temperature or the second temperature. For example, it may be determined whether each target analyte in the sample at different temperatures is present by using the approximate function *1* that approximates the signal values at the first temperature and the approximate function *2* that approximates the signal values at the second temperature. In addition, it may be determined whether each target analyte in the sample at the same second temperature is present by using the approximate function *1* that approximates the signal values at the second temperature and the approximate function 2 that approximates the signal values at the second temperature.

FIG. 13 is a flowchart exemplarily illustrating a method for obtaining the approximate signal for each of the plurality of target analytes, performed by the computer device 200 according to an embodiment.

In step S1310, the computer device 200 may obtain, from the signal generation reaction of the plurality of cycles for the first target analyte and the second target analyte in a sample, the first data set measured at the first detection temperature at each of the plurality of cycles and the second data set measured at the second detection temperature at each of the plurality of cycles.

In step S1320, by at least partially using the first data set and the second data set, the computer device 200 may perform the joint-estimation on a value(s) of at least one approximate function parameter of at least one approximate function and a value(s) of the magnitude parameter; wherein the at least one approximate function may include at least one selected from the group consisting of: the approximate function *1-1* for approximating signal values dependent on the presence of the first target analyte at the first detection temperature; the approximate function *1-2* for approximating signal values dependent on the presence of the first target analyte at the second detection temperature; and the approximate function *2* for approximating signal values dependent on the presence of the second target analyte at the second detection temperature; wherein the magnitude parameter may indicate a relationship between the signal values dependent on the presence of the first target analyte at the first detection temperature and the signal values dependent on the presence of the first target analyte at the second detection temperature.

As described above, step S1320 may be performed according to various embodiments, but is not limited thereto.

In the first exemplary embodiment, the computer device 200 may estimate the value of the approximate function parameter *1-1* by using the first data set. In addition, the computer device 200 may perform the joint-estimation on the value of the approximate function parameter *2* and the value of the magnitude parameter by at least partially using the value estimated for the approximate function parameter *1-1* and the second data set.

In the second embodiment, the computer device 200 may perform the joint-estimation on the value of the approximate function parameter *1-1*, the value of the approximate function parameter *2*, and the value of the magnitude parameter, in consideration of: (a) the sum of the differences between the first data set and the approximate function *1-1* at each of the plurality of cycles; and (b) the sum of the difference at each of the plurality of cycles between (b-1) the second data set and (b-2) the sum of the approximate function *2* and the signal value dependent on the first target analyte at the second temperature (or the approximate function *1-2*).

In the third embodiment, the computer device 200 may estimate the value of the parameter of the target signal approximate function *1-1* and the background signal approximate function *1-1* included in the approximate function *1*, by using the first data set. In addition, the computer device 200 may perform the joint-estimation on the value of the parameter of the target signal approximate function *2-2* and the background signal approximate function *2-2* included in the approximate function *2-2* and the magnitude parameter value, by at least partially using the estimated value of the parameter and the second data set.

In the fourth embodiment, the computer device 200 may perform the joint-estimation on the value of the parameter of the target signal approximate function *1-1*, the background signal approximate function *1-1*, the target signal approximate function *2-2*, the background signal approximate function *2-2*, and the magnitude parameter, in consideration of: (a) the sum of the difference between the first data set and the approximate function *1-1* at each of the plurality of cycles; and (b) the sum of the differences at each of the plurality of cycles between (b-1) the second data set and (b-2) the sum of the approximate function *2-2* and the signal value dependent on the first target analyte at the second temperature (or the approximate function *1-2*).

According to an embodiment of the present disclosure, in the process of detecting two or more target analytes in a sample, values of parameters may be estimated in a more systematic and easy-to-apply manner by performing the joint-estimation on the value of the approximate function parameter and values of the magnitude parameter that are used to separate signal values dependent on each target analyte.

According to an embodiment of the present disclosure, by estimating an optimized value of the magnitude parameter for each reaction or for each well in which the sample is accommodated, a more accurate positive/negative determination result for the target analyte may be obtained.

According to an embodiment of the present disclosure, cost efficiency may be improved by omitting numerous procedures required to previously set the value of the magnitude parameter in the related art.

Meanwhile, the method for obtaining the approximate signal according to an embodiment may be implemented as a computer-readable recording medium storing a computer program programmed to perform each step included in the above-described method, or may be implemented by a computer program stored in a computer-readable recording medium programmed to perform each step included in the above-described method.

Meanwhile, a method for detecting each of the plurality of target analytes may be performed by the computer device 200 according to another embodiment of the present disclosure.

After the above-described steps S1310 to S1320 are performed, the computer device 200 may detect each of the first target analyte and the second target analyte in the sample by using the joint-estimation result. Specifically, the computer device 200 may determine the presence or absence of the first target analyte in the sample by using the approximate function *1-1* having the value of the approximate function parameter *1-1* and/or the approximate function *1-2* having the value of the approximate function parameter *1-2* included in the joint-estimation result, and determine the presence or absence of the second target analyte in the sample by using the approximate function *2* having the value of the approximate function parameter *2* included in the joint-estimation result.

According to an embodiment of the disclosure, the computer device 200 may determine the first cycle threshold from the approximate function *1-1* having the estimated value of the approximate function parameter *1-1* or may determine the second cycle threshold determined from the approximate function *1-2* having the estimated value of the approximate function parameter *1-2*. In addition, the computer device 200 may determine the presence or absence of the first target analyte in the sample, based on whether the first cycle threshold or the second cycle threshold satisfies a preset condition (e.g., whether it is within a predetermined value range). In addition, the computer device 200 may determine the second cycle threshold from the approximate function *2* having the estimated value of the approximate function parameter *2*, and determine the presence or absence of the second target analyte in the sample based on whether the second cycle threshold satisfies the above-described condition.

Meanwhile, the computer device 200, according to another embodiment of the present disclosure, may perform the joint-estimation on the parameter of the approximate function that approximate signal values dependent on the presence of each of three target analytes and the magnitude parameter, from three data sets measured at each of the first detection temperature to the third detection temperature.

Specifically, the computer device 200 may obtain, from a signal generation reaction of the plurality of cycles for the first target analyte, the second target analyte, and the third target analyte in a sample, the first data set measured at the first detection temperature at each of the plurality of cycles, the second data set measured at the second detection temperature at each of the plurality of cycles, and the third data set measured at the third detection temperature at each of the plurality of cycles.

As described above, the first data set may include signal values dependent on the presence of the first target analyte in the sample at the first detection temperature, the second data set may include signal values dependent on the presence of the first target analyte and the second target analyte in the sample at the second detection temperature, and the third data set may include signal values dependent on the presence of the first target analyte, the second target analyte, and the third target analyte in the sample at the third detection temperature. As described above, this signal generation reaction is performed by incubating a sample with the first signal generation composition for detecting the first target analyte in the sample, the second signal generation composition for detecting the second target analyte, and the third signal generation composition for detecting the third target analyte in one reaction well, wherein the first signal generation composition, the second signal generation composition, and the third signal generation composition include the same label, and wherein signals from the label are not differentiated for each target analyte by a single type of detector.

In FIG. 15A, the first data set, the second data set, and the third data set are exemplarily illustrated. Referring to FIG. 15A, the first temperature, the second temperature, and the third temperature are 83°C., 72°C., and 60°C., respectively. In addition, target 1 refers to the first target analyte, target 2 refers to the second target analyte, and target 3 refers to the third target analyte. For convenience, an embodiment in which the background signal is not considered is exemplarily illustrated, but a case in which the background signal is considered may be understood in the same or similar manner as the above-described embodiments.

Specifically, in the case of tube 1 in which only target 1 is present, an amplification curve dependent on the presence of target 1 appears in each of the first to third data sets. In the case of tube 2 in which only target 2 is present, an amplification curve dependent on the presence of target 2 appears in the second to third data sets. In the case of tube 3 in which only target 3 is present, an amplification curve dependent on the presence of target 3 appears in the third data set. Further, it can be seen that, on the premise that each target is present with a similar high concentration to the others, the form of an exemplary amplification curve for each of tube 4 in which targets 1 and 2 are present, tube 5 in which targets 2 and 3 are present, tube 6 in which targets 1 and 3 are present, tube 7 in which targets 1 to 3 are present, and tube 8 of the control (NTC) in which none of targets 1 to 3 are present is shown in each data set shown in FIG. 15A.

FIG. 15B illustrates the signal values on the presence of each target analyte at each temperature dependent, which are joint-estimated from the first data set to the third data set shown in FIG. 15A. For example, when only any one of targets 1 to 3 is present, an amplification curve generated in the presence of each target analyte at the detection temperature of each target analyte may be extracted as in tubes 1 to 3. In addition, when two or more of targets 1 to 3 are present, an amplification curve generated in the presence of each target analyte at the detection temperature of each target analyte may be extracted as in tubes 4 to 7.

By using the first data set, the second data set, and the third data set, the computer device 200 may perform the joint-estimation on (a) a value of the approximate function parameter of at least one approximate function among the approximate function *1*, the approximate function *2*, and the approximate function *3*, and (b) a value of at least one magnitude parameter among a plurality of magnitude parameters.

According to a similar principle as described above, the approximate function *1* may include at least one of the approximate function *1-1* that approximates signal values dependent on the presence of the first target analyte at the first detection temperature, the approximate function *1-2* that approximates signal values dependent on the presence of the first target analyte at the second detection temperature, and the approximate function *1-3* that approximates signal values dependent on the presence of the first target analyte at the third detection temperature. In addition, the approximate function *2* may include at least one of the approximate function *2-1* that approximates signal value dependent on the presence of the second target analyte at the first detection temperature, the approximate function *2-2* that approximates signal value dependent on the presence of the second target analyte at the second detection temperature, and the approximate function *2-3* that approximates signal value dependent on the presence of the second target analyte at the third detection temperature.

In addition, the approximate function *3* is a function that approximates signal values dependent on the presence of the third target analyte, and may include at least one of an approximate function *3-1* that approximates signal values dependent on the presence of the third target analyte at the first detection temperature, an approximate function *3-2* that approximates signal values dependent on the presence of the third target analyte at the second detection temperature, and an approximate function *3-3* that approximates signal values dependent on the presence of the third target analyte at the third detection temperature. Likewise, the approximate function *3* may be interpreted in the same or similar manner as the above-described embodiments with respect to the approximate function *1* or the approximate function *2*. For example, the approximate function *3* may be represented by any one of Math Figures 1 to 6 described above as a constant or a polynomial function, and according to an embodiment, may include a target signal approximate function *3* that approximates signal values dependent on the presence of the third target analyte and a background signal approximate function *3* that approximates the third background signal independent from the presence of the third target analyte.

In addition, the plurality of magnitude parameters represent a relationship between (i) signal values dependent on the presence of at least one target analyte among the target analytes at any one detection temperature among from the first detection temperature to the third detection temperature, and (ii) signal values dependent on the presence of the at least one target analyte at another one detection temperature among from the first detection temperature to the third detection temperature. Specifically, each magnitude parameter may represent a relationship of changes in the signals provided by each signal generation composition at two temperatures selected from the first detection temperature, the second detection temperature, and the third detection temperature in the presence of each target analyte, and may be determined, for example, as a difference or ratio between signal values detected at the two selected temperatures.

In an embodiment, the plurality of magnitude parameters may include at least one selected from the group consisting: a first magnitude parameter indicates a relationship between signal values dependent on the presence of the first target analyte at the first detection temperature and signal values dependent on the presence of the first target analyte at the second detection temperature; a second magnitude parameter indicates a relationship between signal values dependent on the presence of the first target analyte at the second detection temperature and signal values dependent on the presence of the first target analyte at the third detection temperature; a third magnitude parameter indicates a relationship between signal values dependent on the presence of the second target analyte at the second detection temperature and signal values dependent on the presence of the second target analyte at the third detection temperature; and a fourth magnitude parameter indicates a relationship between signal values dependent on the presence of the first target analyte at the first detection temperature and signal values dependent on the presence of the first target analyte at the third detection temperature. Each magnitude parameter is a value or a set of values indicating a change pattern of signal values dependent on a specific target analyte at different temperatures, and may mean, for example, a magnitude ratio of the corresponding signal values, or a value or a set of values calculated therefrom according to a predetermined equation. Illustratively, in the case of the first magnitude parameter, it may be obtained as the ratio of the signal values dependent on the presence of the first target analyte at the first detection temperature to the signal values dependent on the presence of the first target analyte at the second detection temperature, or as the ratio of the signal values dependent on the presence of the first target analyte at the first detection temperature to the signal values dependent on the presence of the first target analyte at the second detection temperature.

Meanwhile, the magnitude parameter of the present disclosure is not limited to the above-described embodiments, and according to embodiments, the magnitude parameter may further include magnitude parameters indicating various other signal relationships. For example, the plurality of magnitude parameters may include at least one selected from the group consisting: a fifth magnitude parameter indicating a relationship between signal values dependent on the presence of the second target analyte at the first detection temperature and signal values dependent on the presence of the second target analyte at the second detection temperature; a sixth magnitude parameter indicating a relationship between signal values dependent on the presence of the second target analyte at the first detection temperature and signal values dependent on the presence of the second target analyte at the third detection temperature; a seventh magnitude parameter indicating a relationship between signal values dependent on the presence of the third target analyte at the first detection temperature and signal values dependent on the presence of the third target analyte at the second detection temperature; an eighth magnitude parameter indicating a relationship between signal values dependent on the presence of the third target analyte at the second detection temperature and signal values dependent on the presence of the third target analyte at the third detection temperature; and a ninth magnitude parameter indicating a relationship between signal values dependent on the presence of the third target analyte at the first detection temperature and signal values dependent on the presence of the third target analyte at the third detection temperature, together with the first to fourth magnitude parameters.

The joint-estimation process according to an embodiment may be performed in a method for performing the joint-estimation on (a) the value of the approximate function parameter of at least one approximate function selected from the group consisting of the approximate function *1-1*, the approximate function *1-2*, the approximate function *1-3*, the approximate function *2-2*, the approximate function *2-3*, and the approximate function *3-3*, and (b) the value of at least one magnitude parameter among the plurality of magnitude parameters.

The joint-estimation process according to another embodiment may be performed in a method for performing the joint-estimation on (a) the value of the approximate function parameter of at least one approximate function selected from the group consisting of the approximate function *1-1*, the approximate function *1-2*, the approximate function *1-3*, the approximate function *2-1*, the approximate function *2-2*, the approximate function *2-3*, the approximate function *3-1,* the approximate function *3-2*, and the approximate function *3-3*, and (b) the value of at least one magnitude parameter among the plurality of magnitude parameters.

According to a preferable exemplary embodiment, the joint-estimation may be performed on values of the third parameter group including (a) the approximate function parameters of the approximate function *1-1*, the approximate function *2-2*, and the approximate function *3-3* and (b) the first magnitude parameter to the third magnitude parameter.

According to another preferable exemplary embodiment, the joint-estimation may be performed on values of the fourth parameter group including (a) the approximate function parameters of the approximate function *1-1*, the approximate function *2-2*, and the approximate function *3-3* and (b) the first magnitude parameter, the second magnitude parameter, and the fourth magnitude parameter. However, in the disclosure, it can be seen that the combination of parameters to be subjected to the joint-estimation is not limited to the above-described embodiments, and may be modified and implemented in various ways.

The computer device 200 may prepare the joint-estimation for the above-described parameters based on the pre-stored statistical model. In the statistical model according to an embodiment, a mathematical relationship for separating signal values dependent on the presence of the first target analyte from the first data set measured at the first temperature may be defined. In addition, in the statistical model, a mathematical relationship for excluding signal values dependent on the presence of the first target analyte and separating signal values dependent on the presence of the second target analyte from the second data set measured at the second temperature may be defined. In addition, in the statistical model, a mathematical relationship for excluding signal values dependent on the presence of the first target analyte and signal values dependent on the presence of the second target analyte and separating signal values dependent on the presence of the third target analyte from the third data set measured at the third temperature may be defined. For example, the statistical model may include Math Figure 31 below defining the relationship between the data sets and the approximate function and/or the magnitude parameter, and Math Figure 31 may be expressed as Math Figures 32 to 34. Hereinafter, descriptions overlapping with the above-described embodiments will be omitted.

[Math Figure 31]

$$\begin{pmatrix} y_{1,t} \\ y_{2,t} \\ y_{3,t} \end{pmatrix} = \begin{pmatrix} 1 & 0 & 0 \\ CR_1 & 1 & 0 \\ CR_1 CR_2 & CR_3 & 1 \end{pmatrix} \begin{pmatrix} f_{11}(\alpha_{11},\ t) \\ f_{22}(\alpha_{22},\ t) \\ f_{33}(\alpha_{33},\ t) \end{pmatrix} + \begin{pmatrix} e_1 \\ e_2 \\ e_3 \end{pmatrix}$$

[Math Figure 32]

$$y_{1,\ t} = f_{11}(\alpha_{11},\ t) + e_1$$

[Math Figure 33]

$$y_{2,\ t} = CR_1 * f_{11}(\alpha_{11},\ t) + f_{22}(\alpha_{22},\ t) + e_2$$

[Math Figure 34]

$$y_{3,\ t} = CR_1 CR_2 * f_{11}(\alpha_{11},\ t) + CR_3 * f_{22}(\alpha_{22},\ t) + f_{33}(\alpha_{33},\ t) + e_3$$

where $y_{3,t}$ denotes the third data set measured at the third temperature at each of the plurality of cycles, $\alpha_{33}$ denotes the approximate function parameter *3-3* that is the parameter of the approximate function *3-3*, $CR_1$ denotes the first magnitude

parameter, $CR_2$ denotes the second magnitude parameter, and $CR_3$ denotes the third magnitude parameter. In addition, $f_{33}(\alpha_{33},t)$ denotes the approximate function 3-3 in which the approximate function parameter *3-3* and the cycle are input variables, and $e_3$ denotes a third error indicating an error component in the third data set.

In an embodiment, the computer device 200 may perform the joint-estimation on the above-described parameters based on the statistical model. In an embodiment, the statistical model may include one or more objective functions for estimating the optimized value of the parameter that approximates the data set, and the computer device 200 may obtain the value of the parameter that causes the objective function to be a preset condition (e.g., a maximum value or a minimum value) and calculate the value as an optimized estimate of the parameter. The first objective function with respect to the first error, the second objective function with respect to the second error, the third objective function with respect to the third error, and the entire objective function with respect to the total error may be represented by Math Figures 35 to 38.

[Math Figure 35]

$$l_1(\alpha_{11}) = \frac{1}{n}\sum_t (y_{1,t} - f_{11}(\alpha_{11}, t))^2$$

[Math Figure 36]

$$l_2(\alpha_{22}, CR_1) = \frac{1}{n}\sum_t (y_{2,t} - CR_1 * f_{11}(\alpha_{11}, t) - f_{22}(\alpha_{22}, t))^2$$

[Math Figure 37]

$$l_3(\alpha_{33}, CR_1, CR_1, CR_3)$$

$$= \frac{1}{n}\sum_t (y_{3,t} - CR_1 * CR_2 * f_{11}(\alpha_{11}, t) - CR_3 * f_{22}(\alpha_{22}, t) - f_{33}(\alpha_{33}, t))^2$$

[Math Figure 38]

$$l_{joint}(\alpha_{11}, \alpha_{22}, \alpha_{33}, CR_1, CR_1, CR_3)$$

$$= \frac{1}{\sigma_1{}^2}\sum_t (y_{1,t} - f_{11}(\alpha_{11}, t))^2$$

$$+ \frac{1}{\sigma_2{}^2}\sum_t (y_{2,t} - CR_1 * f_{11}(\alpha_{11}, t) - f_{22}(\alpha_{22}, t))^2$$

$$+ \frac{1}{\sigma_3{}^2}\sum_t (y_{3,t} - CR_1 * CR_2 * f_{11}(\alpha_{11}, t) - CR_3 * f_{22}(\alpha_{22}, t) - f_{33}(\alpha_{33}, t))^2$$

(where $l_3$ represents the third objective function for the third error, and $\sigma_3$ represents the standard deviation of the third error for the plurality of cycles when the sample is the third data set).

Referring to the first embodiment described above, the computer device 200 may estimate the value of the approximate function parameter *1-1* by using the first data set. Also, the computer device 200 may perform the joint-estimation on the value of the approximate function parameter *2-2* and the value of the first magnitude parameter by at least partially using the result of estimating the approximate function parameter *1-1* and the second data set. Also, the computer device 200 may perform the joint-estimation on the value of the approximate function parameter *3-3*, the value of the second magnitude parameter, and the value of the third magnitude parameter by at least partially using the result of estimating the values of the approximate function parameter *2-2* and the magnitude parameter *1-1*, and the third data set.

Referring to the second embodiment described above, the computer device 200 may perform the joint-estimation on the values of the approximate function parameters *1* to the approximate function parameters *3* and the values of the first to third magnitude parameters by at least partially using the first to third data sets. For example, the above-described backfitting algorithm may be used in the joint-estimation process, and this process may be understood with reference to the embodiments described throughout the specification.

Meanwhile, referring to the third embodiment to the fourth embodiment in which the background signal is considered, the above-described approximate function *1* to the approximate function *3* may further include the background signal approximate function for approximating the first background signal to the third background signal. In the above-described joint-estimation process, the parameter values of the background signal approximate functions may be joint-estimated together. Likewise, the corresponding process may be understood with reference to the above-described embodiments.

FIGS. 16A to 16F illustrate simulation results obtained by performing the above-described joint-estimation from the first to third data sets in consideration of the background signal, according to an embodiment. Math Figure 5 was used for each target signal approximate function, Math Figure 10 was used for each background signal approximate function as the background signal is considered as the second-order function, and equations in a modified form further including the background signal approximate function in Math Figures 31 to 38 were used for the joint-estimation for the values of the parameters.

More specifically, the first data set to the third data set in each case are illustrated in the upper graphs of FIGS. 16A to 16F. In addition, the intermediate graph shows a simulation result for each approximate function to which values of parameters estimated from corresponding data sets are applied in a case in which a value of each magnitude parameter is 1. In addition, the bottom graph shows a simulation result for each approximate function to which values of parameters estimated from corresponding data sets are applied in a case in which a plurality of values of each magnitude parameter are provided (specifically, values for each cycle). In FIG. 16, the approximate function 1, 2 and 3 represent the approximate functions 1-1, *2-2,* and 3-3.

FIG. 16A illustrates a case in which only the first target analyte is present, and corresponds to a case in which the first target analyte is present with a relatively high concentration (upper line) and a case in which the first target analyte is present with a relatively low concentration (lower line). In addition, FIGS. 16B and 16C illustrate a case in which only the second target analyte is present and a case in which only the third target analyte is present, respectively, which correspond to a case in which each target analyte is present with a relatively high concentration (upper line) and a case in which each target analyte is present with a relatively low concentration (lower line). **In** each drawing, the first to third data sets in each case are illustrated in the upper graph, each approximate function to which the values of the parameters estimated to minimize the overall error according to the above-described embodiment are applied is visualized in the middle and lower graphs, and it can be seen that each approximate function is appropriately estimated from the corresponding data sets.

FIG. 16D corresponds to a case in which all of the first to third target analytes are present, the first target analyte and the second target analyte are present with a relatively high concentration, and the third target analyte is present with a relatively low concentration. In addition, FIG. 16E corresponds to a case in which the first and third target analytes are present with a relatively high concentration and the second target analyte is present with a relatively low concentration, and FIG. 16F corresponds to a case in which the second and third target analytes are present with a relatively high concentration and the second target analyte is present with a relatively low concentration. Similarly, in each drawing, the first to third data sets in each case are illustrated in the upper graph, each approximate function to which the values of the parameters estimated to minimize the overall error according to the above-described embodiment are applied is visualized in the middle and lower graphs, and it can be seen that each approximate function is appropriately estimated from the corresponding data sets.

As shown in FIGS. 16A to 16F, it can be seen that more accurate estimation results were obtained in the case in which the value of each magnitude parameter was estimated for each cycle than in the case in which the value of the magnitude parameter was estimated as a single value. Particularly, when two or more target analytes were present in the sample, it can be seen that this difference is more pronounced. In addition, in each of the middle and bottom graphs of FIGS. 16A to 16F, a solid line shows output values of each approximate function corresponding to an estimate, and a dotted line shows signal values corresponding to raw data dependent on each target analyte in which an error is reflected. In other words, it can be seen that the estimation result and the raw data are well matched.

FIG. 17 is a flowchart exemplarily illustrating a method for obtaining the approximate signal for each of the plurality of target analytes, performed by the computer device 200 according to an embodiment.

In step S1710, the computer device 200 may obtain, from a signal generation reaction of the plurality of cycles for the first target analyte, the second target analyte and the third target analyte in a sample, the first data set measured at the first detection temperature at each of the plurality of cycles, the second data set measured at the second detection temperature at each of the plurality of cycles and the third data set measured at the third detection temperature at each of the plurality of cycles. As described above, this signal generation reaction is performed by incubating the sample with the first signal generation composition for detecting the first target analyte in the sample, the second signal generation composition for detecting the second target analyte in the sample and the third signal generation composition for detecting the third target analyte in the sample, wherein the first signal generation composition, the second signal generation composition and the third signal generation composition include the same label, and wherein signals from the label are not differentiated for each target analyte by a single type of detector.

In step S1720, by at least partially using the first data set to the third data set, the computer device 200 may perform the j oint-estimation on (a) a value(s) of at least one approximate function parameter of at least one approximate function, wherein the at least one approximate function includes at least one selected from the group consisting of: the approximate function 1-1 for approximating signal values dependent on the presence of the first target analyte at the first detection temperature; the approximate function *1-2* for approximating signal values dependent on the presence of the first target analyte at the second detection temperature; the approximate function *1-3* for approximating signal values dependent on the presence of the first target analyte at the third detection temperature; the approximate function *2-2* for approximating signal values dependent on the presence of the second target analyte at the second detection temperature; the approximate function 2-3 for approximating signal values dependent on the presence of the second target analyte at the third detection temperature; and the approximate function *3-3* for approximating signal values dependent on the presence of the third target analyte at the third detection temperature; and (b) a value(s) of at least one magnitude parameter, wherein the at least one magnitude parameter is at least one of a plurality of magnitude parameters indicate a relationship between signal value dependent on the presence of at least one target analyte among the target analytes at any one detection temperature among from the first detection temperature to the third detection temperature and signal values dependent on the presence of the at least one target analyte at another one detection temperature among from the first detection temperature to the third detection temperature.

Meanwhile, a method for detecting each of the plurality of target analytes may be performed by the computer device 200 according to another embodiment of the present disclosure. After steps S1710 to S1720 are performed, the computer device 200 may detect each of the first target analyte to the third target analyte in the sample by using the joint-estimation result, and may be understood in the same or similar manner as in the above-described embodiments.

Meanwhile, in the above, an embodiment of obtaining the approximate signal for each of three target analytes has been described, but the present disclosure is not limited thereto, and it can be seen that embodiments of obtaining the approximate signal for each of m target analytes based on the technical features described in the specification may be modified and implemented in an expandable form.

Combinations of each block of the block diagram attached to the present invention and each step of the flowchart may be performed by computer program instructions. Since the computer program instructions may be installed in an encoding processor of a general purpose computer, a special purpose computer, or other programmable data processing device, the computer program instructions performed through the encoding processor of the computer or other programmable data processing device generate means for performing the functions described in each block of the block diagram or each step of the flowchart. Since the computer program instructions may be stored in a computer-usable or computer-readable memory that may direct a computer or other programmable data processing equipment to implement functions in a specific method, the instructions stored in the computer-usable or computer-readable memory may produce manufacturing items including instruction means for performing the functions described in each block of the block diagram or each step of the flowchart. Since the computer program instructions may be mounted on a computer or other programmable data processing device, the instructions, which perform a series of operation steps on the computer or other programmable data processing device to generate a process executed by the computer, may provide steps for executing functions described in each block of the block diagram and each step of the flowchart.

In addition, each block or each step may represent a module, a segment, or a portion of code including one or more executable instructions for executing the specified logical function(s). It should also be noted that in some alternative embodiments, the functions mentioned in the blocks or steps may occur out of order. For example, two blocks or steps shown in succession may in fact be carried out substantially simultaneously or the blocks or steps may sometimes be carried out in reverse order depending on the function to which they correspond.

The above description is merely illustrative of the technical idea of the present disclosure, and those skilled in the art to which the present disclosure pertains will be able to make various modifications and variations without departing from the essential quality of the present disclosure. Therefore, the embodiments disclosed in the present disclosure are not intended to limit the technical idea of the present disclosure, but to explain it, and the scope of the technical idea of the present disclosure is not limited by these embodiments. The protection scope of the present invention should be interpreted by the following claims, and all technical ideas within the scope equivalent thereto should be interpreted as being included in the scope of the present invention.

**Claims**

1.  A method for obtaining an approximate signal for each of a plurality of target analytes performed by a computer device, the method comprising:

    obtaining, from a signal generation reaction of a plurality of cycles for a first target analyte and a second target analyte in a sample, a first data set measured at a first detection temperature at the plurality of cycles and a second data set measured at a second detection temperature at the plurality of cycles; wherein the first data set includes signal values at the first detection temperature dependent on the presence of the first target analyte in the sample, and the second data set includes signal values at the second detection temperature dependent on the presence of the first target analyte and the second target analyte in the sample; and
    wherein the signal generation reaction is performed by incubating the sample with a first signal generation composition for detecting the first target analyte in the sample and a second signal generation composition for detecting the second target analyte in the sample; wherein the first signal generation composition and the second signal generation composition include the same label, and wherein signals from the label are not differentiated for each target analyte by a single type of detector; and
    by at least partially using the first data set and the second data set, performing a joint-estimation on (a) a value of at least one approximate function parameter of at least one approximate function, wherein the at least one approximate function includes at least one selected from the group consisting of: an approximate function 1-1 for approximating signal values dependent on the presence of the first target analyte at the first detection temperature; an approximate function 1-2 for approximating signal values dependent on the presence of the first target analyte at the second detection temperature; and an approximate function 2 for approximating signal values dependent on the presence of the second target analyte at the second detection temperature; and (b) a value of a magnitude parameter, wherein the magnitude parameter indicates a relationship between the signal values dependent on the presence of the first target analyte at the first detection temperature and the signal values dependent on the presence of the first target analyte at the second detection temperature.

2.  The method of claim 1, wherein the performing the j oint-estimation includes:

    performing the j oint-estimation on a parameter value of a first parameter group including approximate function parameters of the approximate function 1-1 and the approximate function 2 and the magnitude parameter; wherein the magnitude parameter in the first parameter group indicates a ratio between the magnitude at the second detection temperature and the magnitude at the first detection temperature; or,
    performing the joint-estimation on a parameter value of a second parameter group including approximate function parameters of the approximate function 1-2 and the approximate function 2 and the magnitude parameter; wherein the magnitude parameter in the second parameter group indicates a ratio between the magnitude at the first detection temperature and the magnitude at the second detection temperature.

3.  The method of claim 1, wherein the performing the j oint-estimation includes:

    estimating a value of an approximate function parameter 1-1 of the approximate function 1-1 by using the first data set; and
    performing the joint-estimation on a value of an approximate function parameter 2 of the approximate function 2 and the value of the magnitude parameter, by at least partially using the estimated value of the approximate function parameter 1-1 and the second data set.

4.  The method of claim 3, wherein the value of the approximate function 1-1 parameter is estimated in such a manner that a sum of differences between the approximate function 1-1 and the first data set at each of the plurality of cycles is minimized.

5.  The method of claim 3, wherein the performing the joint-estimation on the value of the approximate function parameter 2 and the value of the magnitude parameter includes:

    preparing a value of the approximate function 1-2, by using the value of the magnitude parameter and the value of the approximate function 1-1 having the estimated value of the approximate function parameter 1-1;
    calculating a difference between (a) the second data set and (b) a sum of the approximate function 2 and the approximate function 1-2 at each of the plurality of cycles; and
    performing the joint-estimation on the value of the approximate function parameter 2 and the value of the magnitude parameter in such a manner that a sum of the difference at each of the plurality of cycles is minimized.

6. The method of claim 5, wherein the value of the approximate function parameter 2 and the value of the magnitude parameter are estimated based on a joint maximum likelihood estimation.

7. The method of claim 1, wherein the performing the joint-estimation includes:
performing the joint-estimation on a value of an approximate function parameter *1-1* of the approximate function *1-1,* a value of an approximate function parameter 2 of the approximate function 2, and the value of the magnitude parameter, in consideration of (a) a sum of a difference between the first data set and the approximate function *1-1* at each of the plurality of cycles and (b) a sum of a difference at each of the plurality of cycles between (b-1) the second data set and (b-2) the approximate function 2 and the approximate function *1-2.*

8. The method of claim 7, wherein the value of the approximate function parameter *1-1,* the value of the approximate function parameter 2, and the value of the magnitude parameter are estimated based on a joint maximum likelihood estimation.

9. The method of claim 7, wherein the performing the joint-estimation further includes:

applying an arbitrary value or a pre-estimated value to one or two parameters among the approximate function parameter *1-1,* the approximate function parameter *2* and the magnitude parameter, and estimating values of the remaining parameters; and
applying the estimated values of the remaining parameters and estimating values of the one or two parameters, wherein the estimating values of the remaining parameters and the estimating values of the one or two parameters are performed multiple times.

10. The method of claim 1, wherein each of the first data set and the second data set is a raw data set obtained from a detection device that detects the first target analyte and the second target analyte in the sample; a mathematically transformed data set of the raw data set; a normalized data set of the raw data set; or a normalized data set of the mathematically transformed data set.

11. The method of claim 1, wherein each of the approximate function *1-1,* the approximate function 1-2, and the approximate function 2 include at least one selected from the group consisting of a sigmoid function, a logistic function, a Gompertz function, and a Chapman function.

12. The method of claim 1,

wherein the approximate function *1-1* has an approximate function parameter *1-1* and the approximate function *1-2* has an approximate function parameter *1-2;* each of the approximate function parameter *1-1* and the approximate function parameter *1-2* includes at least one of: a parameter indicating a first slope for the cycle of the signal values dependent on the presence of the first target analyte; a parameter indicating a cycle corresponding to a point at which the first slope is maximum; a parameter indicating signal values in a baseline region; and a parameter indicating signal values in a plateau region, and
wherein the approximate function *2* has an approximate function parameter *2;* the approximate function parameter *2* includes at least one of: a parameter indicating a second slope for the cycle of signal values dependent on the presence of the second target analyte; a parameter indicating a cycle corresponding to a point at which the second slope is maximum; a parameter indicating signal values in a baseline region; and a parameter indicating signal values in a plateau region.

13. The method of claim 1, wherein each of the approximate function *1-1,* the approximate function *1-2,* and the approximate function *2* further includes a parameter of a background signal approximate function for a background signal,

wherein the parameter of the background signal approximate function in each of the approximate function *1-1* and the approximate function *1-2* includes at least one of: a parameter that approximates a slope of the cycle of a first background signal further included in the first data set; a parameter that approximates signal values in a baseline region; and a parameter that approximates a cycle corresponding to a point at which a magnitude of the first background signal is minimum; and
wherein the parameter of the background signal approximate function of the approximate function 2 includes at least one of: a parameter that approximates a slope of the cycle of a second background signal further included in the second data set; a parameter that approximates signal values in a baseline region; and a parameter that approximates a cycle corresponding to a point at which a magnitude of the second background signal is minimum.

14. The method of claim 13, wherein the parameter of the background signal approximate function is a parameter of a constant or a polynomial function.

15. The method of claim 1, wherein the first detection temperature is greater than the second detection temperature.

16. The method of claim 1, wherein the second detection temperature is greater than the first detection temperature.

17. The method of claim 1, wherein the signal generation reaction involves a nucleic acid amplification.

18. The method of claim 1, wherein the performing the joint-estimation includes joint-estimating the value of the magnitude parameter for each of two or more cycle sections selected from the plurality of cycles.

19. The method of claim 1, wherein the performing the joint-estimation includes joint-estimating the value of the magnitude parameter for each of the plurality of cycles.

20. The method of claim 1, wherein the performing the joint-estimation includes:

    estimating a value of an approximate function parameter *1-1* of the approximate function *1-1* by using the first data set; and
    performing the joint-estimation on a value of an approximate function parameter *2* of the second approximate function 2 and a value of the magnitude parameter, by using the first data set and the second data set.

21. The method of claim 20, wherein the performing the j oint-estimation on the value of the approximate function parameter 2 and the value of the magnitude parameter includes:

    calculating a difference between (a) the second data set; and (b) the first data set and the magnitude parameter; at each of the plurality of cycles, and
    performing the joint-estimation on the value of the approximate function parameter *2* and the value of the magnitude parameter in such a manner that a sum of the differences at each of the plurality of cycles is minimized.

22. A computer program stored in a computer-readable recording medium, programmed to perform each step included in the method of claim 1.

23. A non-transitory computer-readable recording medium storing a computer program programmed to perform each step included in the method of claim 1.

24. A computer device, comprising:

    a memory configured to store at least one instruction; and
    a processor configured to execute the one or more instructions stored in the memory, wherein the instructions, when executed by the processor, cause the processor to:

        obtaining, from a signal generation reaction of a plurality of cycles for a first target analyte and a second target analyte in a sample, a first data set measured at a first detection temperature at the plurality of cycles and a second data set measured at a second detection temperature at the plurality of cycles; wherein the first data set includes signal values at the first detection temperature dependent on the presence of the first target analyte in the sample, and the second data set includes signal values at the second detection temperature dependent on the presence of the first target analyte and the second target analyte in the sample; and wherein the signal generation reaction is performed by incubating the sample with a first signal generation composition for detecting the first target analyte in the sample and a second signal generation composition for detecting the second target analyte in the sample; wherein the first signal generation composition and the second signal generation composition include the same label, and wherein signals from the label are not differentiated for each target analyte by a single type of detector; and
        by at least partially using the first data set and the second data set, performing a joint-estimation on (a) a value of at least one approximate function parameter of at least one approximate function, wherein the at least one approximate function includes at least one selected from the group consisting of: an approximate function *1-1* for approximating signal values dependent on the presence of the first target analyte at the first detection temperature; an approximate function 1-2 for approximating signal values dependent on the presence of the first target analyte at the second detection temperature; and an approximate function *2* for approximating signal values dependent on the presence of the second target analyte at the second detection temperature; and (b) a value of a magnitude parameter, wherein the magnitude parameter indicates a relationship between the signal values dependent on the presence of the first target analyte at the first detection temperature and the signal values dependent on the presence of the first target analyte at the second detection temperature.

25. A method for obtaining an approximate signal for each of a plurality of target analytes performed by a computer device, the method comprising:

obtaining, from a signal generation reaction of a plurality of cycles for a first target analyte and a second target analyte in a sample, a first data set measured at a first detection temperature at the plurality of cycles and a second data set measured at a second detection temperature at the plurality of cycles; wherein the first data set includes signal values at the first detection temperature dependent on the presence of the first target analyte in the sample, and the second data set includes signal values at the second detection temperature dependent on the presence of the first target analyte and the second target analyte in the sample; and

wherein the signal generation reaction is performed by incubating the sample with a first signal generation composition for detecting the first target analyte in the sample and a second signal generation composition for detecting the second target analyte in the sample; wherein the first signal generation composition and the second signal generation composition include the same label, and wherein signals from the label are not differentiated for each target analyte by a single type of detector; and

by at least partially using the first data set and the second data set, performing a joint-estimation on (a) a value of at least one approximate function parameter of at least one approximate function, wherein the at least one approximate function includes at least one selected from the group consisting of: an approximate function *1-1* for approximating signal values dependent on the presence of the first target analyte at the first detection temperature; an approximate function *1-2* for approximating signal values dependent on the presence of the first target analyte at the second detection temperature; and an approximate function *2* for approximating signal values dependent on the presence of the second target analyte at the second detection temperature; and (b) a value of a magnitude parameter, wherein the magnitude parameter indicates a relationship between the signal values dependent on the presence of the first target analyte at the first detection temperature and the signal values dependent on the presence of the first target analyte at the second detection temperature; and

detecting each of the first target analyte and the second target analyte in the sample by using the joint-estimation result.

26. A method for obtaining an approximate signal for each of a plurality of target analytes performed by a computer device, the method comprising:

obtaining, from a signal generation reaction of a plurality of cycles for a first target analyte, a second target analyte and a third target analyte in a sample, a first data set measured at a first detection temperature at each of the plurality of cycles, a second data set measured at a second detection temperature at each of the plurality of cycles and a third data set measured at a third detection temperature at each of the plurality of cycles; wherein the first data set includes signal values at the first detection temperature dependent on the presence of the first target analyte in the sample, the second data set includes signal values at the second detection temperature dependent on the presence of the first target analyte and the second target analyte in the sample, and the third data set includes signal values at the third detection temperature dependent on the presence of the first target analyte, the second target analyte and the third target analyte in the sample;

wherein the signal generation reaction is performed by incubating the sample with a first signal generation composition for detecting the first target analyte in the sample, a second signal generation composition for detecting the second target analyte in the sample and a third signal generation composition for detecting the third target analyte in the sample; wherein the first signal generation composition, the second signal generation composition and the third signal generation composition include the same label, and wherein signals from the label are not differentiated for each target analyte by a single type of detector; and

by at least partially using the first data set to the third data set, performing a joint-estimation on (a) a value of at least one approximate function parameter of at least one approximate function, wherein the at least one approximate function includes at least one selected from the group consisting of: an approximate function *1-1* for approximating signal values dependent on the presence of the first target analyte at the first detection temperature; an approximate function *1-2* for approximating signal values dependent on the presence of the first target analyte at the second detection temperature; an approximate function *1-3* for approximating signal values dependent on the presence of the first target analyte at the third detection temperature; an approximate function *2-2* for approximating signal values dependent on the presence of the second target analyte at the second detection temperature; an approximate function *2-3* for approximating signal values dependent on the presence of the second target analyte at the third detection temperature; and an approximate function *3-3* for approximating signal values dependent on the presence of the third target analyte at the third detection temperature; and (b) a value of at least one magnitude parameter, wherein the at least one magnitude parameter is at least one of a plurality of magnitude parameters indicate a relationship between signal values dependent on the presence of at least one target analyte among the target analytes at any one detection temperature among from the first detection temperature to the third detection temperature and signal values dependent on the presence of the at least one target analyte at another one detection temperature among from the first detection temperature to the third detection temperature.

27. The method of claim 26, wherein the plurality of magnitude parameters include at least one selected from the group consisting: a first magnitude parameter indicates a relationship between signal values dependent on the presence of the first target analyte at the first detection temperature and signal values dependent on the presence of the first target analyte at the second detection temperature; a second magnitude parameter indicates a relationship between signal values dependent on the presence of the first target analyte at the second detection temperature and signal values dependent on the presence of the first target analyte at the third detection temperature; a third magnitude parameter indicates a relationship between signal values dependent on the presence of the second target analyte at the second detection temperature and signal values dependent on the presence of the second target analyte at the third detection temperature; and a fourth magnitude parameter indicates a relationship between signal values dependent on the presence of the first target analyte at the first detection temperature and signal values dependent on the presence of the first target analyte at the third detection temperature.

# FIG.1

<u>1000</u>

```
┌─────────────────────┐         ┌─────────────────────┐
│  DATA PROVISION     │◄───────►│  COMPUTER DEVICE    │
│  DEVICE (100)       │         │  (200)              │
└─────────────────────┘         └─────────────────────┘
```

# *FIG.2A*

| TUBE | TARGET[1] | THE FIRST DATA SET 72°C (THE FIRST TEMP.) | THE SECOND DATA SET 60°C (THE SECOND TEMP.) |
|------|-----------|--------------------------------------------|----------------------------------------------|
| 1 | CT | AMPLIFICATION (RFU vs CYCLE) | AMPLIFICATION (RFU vs CYCLE) |
| 2 | NG | AMPLIFICATION (RFU vs CYCLE) | AMPLIFICATION (RFU vs CYCLE) |
| 3 | CT + NG | AMPLIFICATION (RFU vs CYCLE) | AMPLIFICATION (RFU vs CYCLE) |
| 4 | CT + NG | AMPLIFICATION (RFU vs CYCLE) | AMPLIFICATION (RFU vs CYCLE) |
| 5 | NTC | AMPLIFICATION (RFU vs CYCLE) | AMPLIFICATION (RFU vs CYCLE) |

[1] TARGET IS GENOMIC DNA OF CHLAMYDIA TRACHOMATIS (CT) AND NEISSERIA GONORRHOEAE (NG). NTC REPRESENTS THE CONTROL GROUP WITHOUT TARGET.

# FIG.2B

| TUBE | TARGET[1] | SIGNAL VALUES DEPENDENT ON THE PRESENCE OF CT | SIGNAL VALUES DEPENDENT ON THE PRESENCE OF NG |
|------|-----------|----------------------------------------------|-----------------------------------------------|
| | | *72°C* (THE FIRST TEMP.) | *60°C* (THE SECOND TEMP.) |
| *1* | *CT* | AMPLIFICATION | AMPLIFICATION |
| *2* | *NG* | AMPLIFICATION | AMPLIFICATION |
| *3* | *CT + NG* | AMPLIFICATION | AMPLIFICATION |
| *4* | *CT + NG* | AMPLIFICATION | AMPLIFICATION |
| *5* | *NTC* | AMPLIFICATION | AMPLIFICATION |

[1] TARGET IS GENOMIC DNA OF CHLAMYDIA TRACHOMATIS (CT) AND NEISSERIA GONORRHOEAE (NG). NTC REPRESENTS THE CONTROL GROUP WITHOUT TARGET.

# *FIG.3*

# FIG.4A

# FIG.4B

# FIG.5

```
        ┌─────────────┐
        │    START    │
        └─────────────┘
               │
               ▼
┌─────────────────────────────────────────┐
│ ESTIMATING VALUE OF THE APPROXIMATE      │
│ FUNCTION PARAMETER 1-1 OF THE APPROXIMATE│───S510
│ FUNCTION 1-1 BY USING THE FIRST DATA SET │
└─────────────────────────────────────────┘
               │
               ▼
┌─────────────────────────────────────────┐
│ PERFORMING THE JOINT-ESTIMATION ON VALUE │
│ OF THE APPROXIMATE FUNCTION PARAMETER 2  │
│ OF THE APPROXIMATE FUNCTION 2 AND VALUE  │
│ OF THE MAGNITUDE PARAMETER, BY AT LEAST  │───S520
│ PARTIALLY USING THE RESULT OF ESTIMATING │
│ THE APPROXIMATE FUNCTION PARAMETER 1-1   │
│ AND THE SECOND DATA SET                  │
└─────────────────────────────────────────┘
               │
               ▼
        ┌─────────────┐
        │     END     │
        └─────────────┘
```

# FIG.6

```
              ┌──────────────┐
              │    START     │
              └──────┬───────┘
                     │
                     ▼
  ┌─────────────────────────────────────────────┐
  │ APPLYING ANY VALUE OR A PRE-ESTIMATED VALUE  │
  │     TO ANY ONE OR TWO PARAMETERS AMONG       │
  │ THE APPROXIMATE FUNCTION PARAMETER 1-1, THE  │──── S610
  │    APPROXIMATE FUNCTION PARAMETER 2, AND     │
  │    THE MAGNITUDE PARAMETER, AND ESTIMATING   │
  │       VALUE OF THE REMAINING PARAMETER       │
  └─────────────────────┬───────────────────────┘
                        │
                        ▼
  ┌─────────────────────────────────────────────┐
  │    APPLYING THE ESTIMATED VALUES OF THE      │
  │    ABOVE REMAINING PARAMETER AND ESTIMATING  │──── S620
  │  VALUE OF THE ABOVE ONE OR TWO PARAMETERS    │
  └─────────────────────┬───────────────────────┘
                        │
                        ▼
  ┌─────────────────────────────────────────────┐
  │  PERFORM AT LEAST ONE OF STEPS S610 TO S620  │──── S630
  │               MULTIPLE TIMES                 │
  └─────────────────────┬───────────────────────┘
                        │
                        ▼
              ┌──────────────┐
              │     END      │
              └──────────────┘
```

# FIG.7A

| TUBE | TARGET[1] | THE FIRST DATA SET 72℃ (THE FIRST TEMP.) | THE SECOND DATA SET 60℃ (THE SECOND TEMP.) |
|---|---|---|---|
| 1 | CT | | |
| 2 | NG | | |
| 3 | CT + NG | | |
| 4 | CT + NG | | |
| 5 | NTC | | |

[1] TARGET IS GENOMIC DNA OF CHLAMYDIA TRACHOMATIS (CT) AND NEISSERIA GONORRHOEAE (NG). NTC REPRESENTS THE CONTROL GROUP WITHOUT TARGET.

# FIG.7B

| TUBE | TARGET[1] | SIGNAL VALUES DEPENDENT ON THE PRESENCE OF CT | BACKGROUND SIGNAL | SIGNAL VALUES DEPENDENT ON THE PRESENCE OF NG | BACKGROUND SIGNAL |
|---|---|---|---|---|---|
| | | 72°C (THE FIRST TEMP.) | | 60°C (THE SECOND TEMP.) | |
| 1 | CT | AMPLIFICATION | AMPLIFICATION | AMPLIFICATION | AMPLIFICATION |
| 2 | NG | AMPLIFICATION | AMPLIFICATION | AMPLIFICATION | AMPLIFICATION |
| 3 | CT+NG | AMPLIFICATION | AMPLIFICATION | AMPLIFICATION | AMPLIFICATION |
| 4 | CT+NG | AMPLIFICATION | AMPLIFICATION | AMPLIFICATION | AMPLIFICATION |
| 5 | NTC | AMPLIFICATION | AMPLIFICATION | AMPLIFICATION | AMPLIFICATION |

[1] TARGET IS GENOMIC DNA OF CHLAMYDIA TRACHOMATIS (CT) AND NEISSERIA GONORRHOEAE (NG).
NTC REPRESENTS THE CONTROL GROUP WITHOUT TARGET.

EP 4 553 489 A1

# FIG.8

START

↓

ESTIMATING VALUE OF PARAMETER OF THE TARGET SIGNAL APPROXIMATE FUNCTION *1-1* FOR SIGNAL VALUES DEPENDENT ON THE FIRST TARGET ANALYTE INCLUDED IN THE APPROXIMATE FUNCTION *1-1* AND VALUE OF PARAMETER OF THE BACKGROUND SIGNAL APPROXIMATE FUNCTION *1-1* FOR THE FIRST BACKGROUND SIGNAL, BY USING THE FIRST DATA SET — *S810*

↓

PERFORMING THE JOINT-ESTIMATION ON VALUES OF PARAMETER OF THE TARGET SIGNAL APPROXIMATE FUNCTION *2* FOR SIGNAL VALUES DEPENDENT ON THE SECOND TARGET ANALYTE INCLUDED IN THE APPROXIMATE FUNCTION *2*, PARAMETER OF THE BACKGROUND SIGNAL APPROXIMATE FUNCTION *2* FOR THE SECOND BACKGROUND SIGNAL, AND MAGNITUDE PARAMETER, BY AT LEAST PARTIALLY USING THE ESTIMATED VALUES OF PARAMETERS OF THE TARGET SIGNAL APPROXIMATE FUNCTION *1-1*, THE BACKGROUND SIGNAL APPROXIMATE FUNCTION *1-1*, AND THE SECOND DATA SET — *S820*

↓

END

# FIG.9

```
        ┌─────────────┐
        │    START    │
        └──────┬──────┘
               │
               ▼
┌──────────────────────────────────────────┐
│ APPLYING ARBITRARY VALUE OR PRE-ESTIMATED │
│ VALUE TO SOME PARAMETERS AMONG PARAMETERS │
│ OF THE TARGET SIGNAL APPROXIMATE FUNCTION │
│ 1-1, THE BACKGROUND SIGNAL APPROXIMATE    │── S910
│ FUNCTION 1-1, THE TARGET SIGNAL           │
│ APPROXIMATE FUNCTION 2, THE BACKGROUND    │
│ SIGNAL APPROXIMATE FUNCTION 2, AND        │
│ MANITUDE PARAMETER, AND ESTIMATING VLAUES │
│ OF THE REMAINING PARAMETERS               │
└──────────────────┬───────────────────────┘
                   │
                   ▼
┌──────────────────────────────────────────┐
│ APPLYING THE ESTIMATED VALUES OF THE      │
│ ABOVE REMAINING PARAMETERS AND            │── S920
│ ESTIMATING VALUES OF THE ABOVE SOME       │
│ PARAMETERS                                │
└──────────────────┬───────────────────────┘
                   │
                   ▼
┌──────────────────────────────────────────┐
│ PERFORMING AT LEAST ONE OF STEPS S910 TO  │── S930
│ S920 MULTIPLE TIMES                       │
└──────────────────┬───────────────────────┘
                   │
                   ▼
        ┌─────────────┐
        │     END     │
        └─────────────┘
```

# FIG.10A

| THE FIRST DATA SET | THE SECOND DATA SET |
|---|---|
| 72°C (THE FIRST TEMP.) | 60°C (THE SECOND TEMP.) |

| THE APPROXIMATE FUNCTION 1-1 | THE APPROXIMATE FUNCTION 2-2 |
|---|---|
| 72°C (THE FIRST TEMP.) | 60°C (THE SECOND TEMP.) |

# FIG.10B

| THE FIRST DATA SET | THE SECOND DATA SET |
|---|---|
| 72℃ (THE FIRST TEMP.) | 60℃ (THE SECOND TEMP.) |

| THE APPROXIMATE FUNCTION 1-1 | THE APPROXIMATE FUNCTION 2-2 |
|---|---|
| 72℃ (THE FIRST TEMP.) | 60℃ (THE SECOND TEMP.) |

# FIG.10C

| THE FIRST DATA SET | THE SECOND DATA SET |
|---|---|
| 72℃ (THE FIRST TEMP.) | 60℃ (THE SECOND TEMP.) |

| THE APPROXIMATE FUNCTION 1-1 | THE APPROXIMATE FUNCTION 2-2 |
|---|---|
| 72℃ (THE FIRST TEMP.) | 60℃ (THE SECOND TEMP.) |

# FIG.10D

| THE FIRST DATA SET | THE SECOND DATA SET |
| 72°C (THE FIRST TEMP.) | 60°C (THE SECOND TEMP.) |
| THE APPROXIMATE FUNCTION 1-1 | THE APPROXIMATE FUNCTION 2-2 |
| 72°C (THE FIRST TEMP.) | 60°C (THE SECOND TEMP.) |

# FIG. 11A

THE FIRST TARGET ANALYTE IS PRESENT AMONG THE FIRST AND SECOND TARGET ANALYTE
WITH A RELATIVELY HIGH CONCENTRATION

| THE FIRST DATA SET | THE SECOND DATA SET |
|---|---|
| 72°C (THE FIRST TEMP.) | 60°C (THE SECOND TEMP.) |

| SIGNAL VALUES DEPENDENT ON THE PRESENCE OF THE FIRST TARGET ANALYTE | SIGNAL VALUES DEPENDENT ON THE PRESENCE OF THE SECOND TARGET ANALYTE |
|---|---|
| 72°C (THE FIRST TEMP.) | 60°C (THE SECOND TEMP.) |

| THE FIRST BACKGROUND SIGNAL | THE SECOND BACKGROUND SIGNAL |
|---|---|
| 72°C (THE FIRST TEMP.) | 60°C (THE SECOND TEMP.) |

# FIG.11B

THE SECOND TARGET ANALYTE IS PRESENT AMONG THE FIRST AND SECOND TARGET ANALYTE
WITH A RELATIVELY HIGH CONCENTRATION

| THE FIRST DATA SET | THE SECOND DATA SET |
|---|---|
| 72℃ (THE FIRST TEMP.) | 60℃ (THE SECOND TEMP.) |

| SIGNAL VALUES DEPENDENT ON THE PRESENCE OF THE FIRST TARGET ANALYTE | SIGNAL VALUES DEPENDENT ON THE PRESENCE OF THE SECOND TARGET ANALYTE |
|---|---|
| 72℃ (THE FIRST TEMP.) | 60℃ (THE SECOND TEMP.) |

| THE FIRST BACKGROUND SIGNAL | THE SECOND BACKGROUND SIGNAL |
|---|---|
| 72℃ (THE FIRST TEMP.) | 60℃ (THE SECOND TEMP.) |

# FIG.11C

THE FIRST TARGET ANALYTE AND THE SECOND TARGET ANALYTE ARE PRESENT
WITH DIFFERENT CONCENTRATIONS

| THE FIRST DATA SET | THE SECOND DATA SET |
|---|---|
| 72°C (THE FIRST TEMP.) | 60°C (THE SECOND TEMP.) |

| SIGNAL VALUES DEPENDENT ON THE PRESENCE OF THE FIRST TARGET ANALYTE | SIGNAL VALUES DEPENDENT ON THE PRESENCE OF THE SECOND TARGET ANALYTE |
|---|---|
| 72°C (THE FIRST TEMP.) | 60°C (THE SECOND TEMP.) |

| THE FIRST BACKGROUND SIGNAL | THE SECOND BACKGROUND SIGNAL |
|---|---|
| 72°C (THE FIRST TEMP.) | 60°C (THE SECOND TEMP.) |

# FIG.12A

THE FIRST TARGET ANALYTE IS PRESENT AMONG THE FIRST AND SECOND TARGET ANALYTE
WITH A RELATIVELY HIGH CONCENTRATION

| THE FIRST DATA SET | THE SECOND DATA SET |
| --- | --- |
| 72℃ (THE FIRST TEMP.) | 60℃ (THE SECOND TEMP.) |

| SIGNAL VALUES DEPENDENT ON THE PRESENCE OF THE FIRST TARGET ANALYTE | SIGNAL VALUES DEPENDENT ON THE PRESENCE OF THE SECOND TARGET ANALYTE |
| --- | --- |
| 72℃ (THE FIRST TEMP.) | 60℃ (THE SECOND TEMP.) |

| THE FIRST BACKGROUND SIGNAL | THE SECOND BACKGROUND SIGNAL |
| --- | --- |
| 72℃ (THE FIRST TEMP.) | 60℃ (THE SECOND TEMP.) |

# FIG.12B

THE SECOND TARGET ANALYTE IS PRESENT AMONG THE FIRST AND SECOND TARGET ANALYTE
WITH A RELATIVELY HIGH CONCENTRATION

| THE FIRST DATA SET | THE SECOND DATA SET |
|---|---|
| 72℃ (THE FIRST TEMP.) | 60℃ (THE SECOND TEMP.) |

| SIGNAL VALUES DEPENDENT ON THE PRESENCE OF THE FIRST TARGET ANALYTE | SIGNAL VALUES DEPENDENT ON THE PRESENCE OF THE SECOND TARGET ANALYTE |
|---|---|
| 72℃ (THE FIRST TEMP.) | 60℃ (THE SECOND TEMP.) |

| THE FIRST BACKGROUND SIGNAL | THE SECOND BACKGROUND SIGNAL |
|---|---|
| 72℃ (THE FIRST TEMP.) | 60℃ (THE SECOND TEMP.) |

# FIG.12C

THE FIRST TARGET ANALYTE AND THE SECOND TARGET ANALYTE ARE PRESENT IN SAMPLE
WITH DIFFERENT CONCENTRATIONS

| THE FIRST DATA SET | THE SECOND DATA SET |
|---|---|
| 72℃ (THE FIRST TEMP.) | 60℃ (THE SECOND TEMP.) |

| SIGNAL VALUES DEPENDENT ON THE PRESENCE OF THE FIRST TARGET ANALYTE | SIGNAL VALUES DEPENDENT ON THE PRESENCE OF THE SECOND TARGET ANALYTE |
|---|---|
| 72℃ (THE FIRST TEMP.) | 60℃ (THE SECOND TEMP.) |

| THE FIRST BACKGROUND SIGNAL | THE SECOND BACKGROUND SIGNAL |
|---|---|
| 72℃ (THE FIRST TEMP.) | 60℃ (THE SECOND TEMP.) |

# FIG.13

START

OBTAINING, FROM SIGNAL GENERATION REACTION OF
THE PLURALITY OF CYCLES FOR THE FIRST TARGET ANALYTE
AND THE SECOND TARGET ANALYTE IN THE SAMPLE,
THE FIRST DATA SET MEASURED AT THE FIRST DETECTION
TEMPERATURE AT EACH OF THE PLURALITY OF CYCLES AND
THE SECOND DATA SET MEASURED AT THE SECOND DETECTION
TEMPERATURE AT EACH OF THE PLURALITY OF CYCLES

~S1310

BY AT LEAST PARTIALLY USING THE FIRST DATA SET AND THE SECOND DATA SET,
PERFORMING THE JOINT-ESTIMATION ON VALUE OF AT LEAST ONE APPROXIMATE FUNCTION
PARAMETER OF AT LEAST ONE APPROXIMATE FUNCTION AND VALUE OF MAGNITUDE PARAMETER;
WHEREIN THE AT LEAST ONE APPROXIMATE FUNCTION INCLUDES AT LEAST ONE SELECTED FROM
THE GROUP CONSISTING OF: THE APPROXIMATE FUNCTION 1-1 FOR APPROXIMATING SIGNAL VALUES
DEPENDENT ON THE PRESENCE OF THE FIRST TARGET ANALYTE AT THE FIRST DETECTION
TEMPERATURE; THE APPROXIMATE FUNCTION 1-2 FOR APPROXIMATING SIGNAL VALUES DEPENDENT
ON THE PRESENCE OF THE FIRST TARGET ANALYTE AT THE SECOND DETECTION TEMPERATURE; AND
THE APPROXIMATE FUNCTION 2 FOR APPROXIMATING SIGNAL VALUES DEPENDENT ON THE PRESENCE
OF THE SECOND TARGET ANALYTE AT THE SECOND DETECTION TEMPERATURE;
WHEREIN THE MAGNITUDE PARAMETER INDICATES RELATIONSHIP BETWEEN SIGNAL VALUES
DEPENDENT ON THE PRESENCE OF THE FIRST TARGET ANALYTE AT THE FIRST DETECTION
TEMPERATURE AND SIGNAL VALUES DEPENDENT ON THE PRESENCE OF THE FIRST TARGET ANALYTE
AT THE SECOND DETECTION TEMPERATURE

~S1320

END

# FIG.14A

| THE FIRST DATA SET | THE SECOND DATA SET |
|---|---|
| 72℃ (THE FIRST TEMP.) | 60℃ (THE SECOND TEMP.) |

| THE APPROXIMATE FUNCTION 1-1 | THE APPROXIMATE FUNCTION 2-2 |
|---|---|
| 72℃ (THE FIRST TEMP.) | 60℃ (THE SECOND TEMP.) |

# FIG.14B

| THE FIRST DATA SET | THE SECOND DATA SET |
|---|---|
| 72°C (THE FIRST TEMP.) | 60°C (THE SECOND TEMP.) |

| THE APPROXIMATE FUNCTION 1-1 | THE APPROXIMATE FUNCTION 2-2 |
|---|---|
| 72°C (THE FIRST TEMP.) | 60°C (THE SECOND TEMP.) |

# FIG.14C

| THE FIRST DATA SET | THE SECOND DATA SET |
|---|---|
| 72°C (THE FIRST TEMP.) | 60°C (THE SECOND TEMP.) |

| THE APPROXIMATE FUNCTION 1-1 | THE APPROXIMATE FUNCTION 2-2 |
|---|---|
| 72°C (THE FIRST TEMP.) | 60°C (THE SECOND TEMP.) |

# FIG.14D

| THE FIRST DATA SET | THE SECOND DATA SET |
|---|---|
| 72℃ (THE FIRST TEMP.) | 60℃ (THE SECOND TEMP.) |

| THE APPROXIMATE FUNCTION 1-1 | THE APPROXIMATE FUNCTION 2-2 |
|---|---|
| 72℃ (THE FIRST TEMP.) | 60℃ (THE SECOND TEMP.) |

EP 4 553 489 A1

# *FIG.15A*

| TUBE | TARGET | THE FIRST DATA SET 83℃ (THE FIRST TEMP.) | THE SECOND DATA SET 72℃ (THE SECOND TEMP.) | THE THIRD DATA SET 60℃ (THE THIRD TEMP.) |
|------|--------|------|------|------|
| 1 | 1 | | | |
| 2 | 2 | | | |
| 3 | 3 | | | |
| 4 | 1+2 | | | |
| 5 | 2+3 | | | |
| 6 | 1+3 | | | |
| 7 | 1+2+3 | | | |
| 8 | NTC | | | |

# FIG.15B

| TUBE | TARGET | SIGNAL VALUES DEPENDENT ON THE FIRST TARGET ANALYTE | SIGNAL VALUES DEPENDENT ON THE SECOND TARGET ANALYTE | SIGNAL VALUES DEPENDENT ON THE THIRD TARGET ANALYTE |
|------|--------|---|---|---|
| | | 83℃ (THE FIRST TEMP.) | 72℃ (THE SECOND TEMP.) | 60℃ (THE THIRD TEMP.) |
| 1 | 1 | | | |
| 2 | 2 | | | |
| 3 | 3 | | | |
| 4 | 1+2 | | | |
| 5 | 2+3 | | | |
| 6 | 1+3 | | | |
| 7 | 1+2+3 | | | |
| 8 | NTC | | | |

# FIG.16A

| | THE FIRST DATA SET | THE SECOND DATA SET | THE THIRD DATA SET |
|---|---|---|---|
| TUBE TARGET | 83°C (THE FIRST TEMP.) | 72°C (THE SECOND TEMP.) | 60°C (THE THIRD TEMP.) |

CASE IN WHICH EACH MAGNITUDE PARAMETER HAS SINGLE VALUE

| THE APPROXIMATE FUNCTION 1-1 | THE APPROXIMATE FUNCTION 2-2 | THE APPROXIMATE FUNCTION 3-3 |
|---|---|---|
| 83°C (THE FIRST TEMP.) | 72°C (THE SECOND TEMP.) | 60°C (THE THIRD TEMP.) |

CASE IN WHICH EACH MAGNITUDE PARAMETER HAS A PLURALITY OF VALUES

| THE APPROXIMATE FUNCTION 1-1 | THE APPROXIMATE FUNCTION 2-2 | THE APPROXIMATE FUNCTION 3-3 |
|---|---|---|
| 83°C (THE FIRST TEMP.) | 72°C (THE SECOND TEMP.) | 60°C (THE THIRD TEMP.) |

# FIG.16B

| TUBE TARGET | THE FIRST DATA SET | THE SECOND DATA SET | THE THIRD DATA SET |
|---|---|---|---|
| | 83℃ (THE FIRST TEMP.) | 72℃ (THE SECOND TEMP.) | 60℃ (THE THIRD TEMP.) |

CASE IN WHICH EACH MAGNITUDE PARAMETER HAS SINGLE VALUE

| THE APPROXIMATE FUNCTION 1-1 | THE APPROXIMATE FUNCTION 2-2 | THE APPROXIMATE FUNCTION 3-3 |
|---|---|---|
| 83℃ (THE FIRST TEMP.) | 72℃ (THE SECOND TEMP.) | 60℃ (THE THIRD TEMP.) |

CASE IN WHICH EACH MAGNITUDE PARAMETER HAS A PLURALITY OF VALUES

| THE APPROXIMATE FUNCTION 1-1 | THE APPROXIMATE FUNCTION 2-2 | THE APPROXIMATE FUNCTION 3-3 |
|---|---|---|
| 83℃ (THE FIRST TEMP.) | 72℃ (THE SECOND TEMP.) | 60℃ (THE THIRD TEMP.) |

# FIG.16C

| THE FIRST DATA SET | THE SECOND DATA SET | THE THIRD DATA SET |
|---|---|---|

TUBE TARGET

| 83°C (THE FIRST TEMP.) | 72°C (THE SECOND TEMP.) | 60°C (THE THIRD TEMP.) |
|---|---|---|

CASE IN WHICH EACH MAGNITUDE PARAMETER HAS SINGLE VALUE

| THE APPROXIMATE FUNCTION 1-1 | THE APPROXIMATE FUNCTION 2-2 | THE APPROXIMATE FUNCTION 3-3 |
|---|---|---|

| 83°C (THE FIRST TEMP.) | 72°C (THE SECOND TEMP.) | 60°C (THE THIRD TEMP.) |
|---|---|---|

CASE IN WHICH EACH MAGNITUDE PARAMETER HAS A PLURALITY OF VALUES

| THE APPROXIMATE FUNCTION 1-1 | THE APPROXIMATE FUNCTION 2-2 | THE APPROXIMATE FUNCTION 3-3 |
|---|---|---|

| 83°C (THE FIRST TEMP.) | 72°C (THE SECOND TEMP.) | 60°C (THE THIRD TEMP.) |
|---|---|---|

# FIG.16D

|  | THE FIRST DATA SET | THE SECOND DATA SET | THE THIRD DATA SET |
| TUBE TARGET | 83℃ (THE FIRST TEMP.) | 72℃ (THE SECOND TEMP.) | 60℃ (THE THIRD TEMP.) |

CASE IN WHICH EACH MAGNITUDE PARAMETER HAS SINGLE VALUE

| THE APPROXIMATE FUNCTION 1-1 | THE APPROXIMATE FUNCTION 2-2 | THE APPROXIMATE FUNCTION 3-3 |
| 83℃ (THE FIRST TEMP.) | 72℃ (THE SECOND TEMP.) | 60℃ (THE THIRD TEMP.) |

CASE IN WHICH EACH MAGNITUDE PARAMETER HAS A PLURALITY OF VALUES

| THE APPROXIMATE FUNCTION 1-1 | THE APPROXIMATE FUNCTION 2-2 | THE APPROXIMATE FUNCTION 3-3 |
| 83℃ (THE FIRST TEMP.) | 72℃ (THE SECOND TEMP.) | 60℃ (THE THIRD TEMP.) |

# FIG.16E

| | THE FIRST DATA SET | THE SECOND DATA SET | THE THIRD DATA SET |
|---|---|---|---|
| TUBE TARGET | 83℃ (THE FIRST TEMP.) | 72℃ (THE SECOND TEMP.) | 60℃ (THE THIRD TEMP.) |

CASE IN WHICH EACH MAGNITUDE PARAMETER HAS SINGLE VALUE

| THE APPROXIMATE FUNCTION 1-1 | THE APPROXIMATE FUNCTION 2-2 | THE APPROXIMATE FUNCTION 3-3 |
|---|---|---|
| 83℃ (THE FIRST TEMP.) | 72℃ (THE SECOND TEMP.) | 60℃ (THE THIRD TEMP.) |

CASE IN WHICH EACH MAGNITUDE PARAMETER HAS A PLURALITY OF VALUES

| THE APPROXIMATE FUNCTION 1-1 | THE APPROXIMATE FUNCTION 2-2 | THE APPROXIMATE FUNCTION 3-3 |
|---|---|---|
| 83℃ (THE FIRST TEMP.) | 72℃ (THE SECOND TEMP.) | 60℃ (THE THIRD TEMP.) |

# FIG.16F

| TUBE TARGET | THE FIRST DATA SET | THE SECOND DATA SET | THE THIRD DATA SET |
|---|---|---|---|
| | 83℃ (THE FIRST TEMP.) | 72℃ (THE SECOND TEMP.) | 60℃ (THE THIRD TEMP.) |

CASE IN WHICH EACH MAGNITUDE PARAMETER HAS SINGLE VALUE

| THE APPROXIMATE FUNCTION 1-1 | THE APPROXIMATE FUNCTION 2-2 | THE APPROXIMATE FUNCTION 3-3 |
|---|---|---|
| 83℃ (THE FIRST TEMP.) | 72℃ (THE SECOND TEMP.) | 60℃ (THE THIRD TEMP.) |

CASE IN WHICH EACH MAGNITUDE PARAMETER HAS A PLURALITY OF VALUES

| THE APPROXIMATE FUNCTION 1-1 | THE APPROXIMATE FUNCTION 2-2 | THE APPROXIMATE FUNCTION 3-3 |
|---|---|---|
| 83℃ (THE FIRST TEMP.) | 72℃ (THE SECOND TEMP.) | 60℃ (THE THIRD TEMP.) |

# FIG.17

START

OBTAINING, FROM SIGNAL GENERATION REACTION OF THE PLURALITY OF CYCLES
FOR THE FIRST TARGET ANALYTE, THE SECOND TARGET ANALYTE
AND THE THIRD TARGET ANALYTE IN SAMPLE,
WHEREIN THE FIRST DATA SET MEASURED AT THE FIRST DETECTION
TEMPERATURE AT EACH OF THE PLURALITY OF CYCLES, THE SECOND DATA SET
MEASURED AT THE SECOND DETECTION TEMPERATURE AT EACH OF
THE PLURALITY OF CYCLES AND THE THIRD DATA SET MEASURED
AT THE THIRD DETECTION TEMPERATURE AT EACH OF THE PLURALITY OF CYCLES

~S1710

BY AT LEAST PARTIALLY USING THE FIRST DATA SET TO THE THIRD DATA SET, PERFORMING
THE JOINT-ESTIMATION ON (a) VALUE OF AT LEAST ONE APPROXIMATE FUNCTION PARAMETER OF AT
LEAST ONE APPROXIMATE FUNCTION, WHEREIN THE AT LEAST ONE APPROXIMATE FUNCTION INCLUDES
AT LEAST ONE SELECTED FROM THE GROUP CONSISTING OF: THE APPROXIMATE FUNCTION *1-1* FOR
APPROXIMATING SIGNAL VALUES DEPENDENT ON THE PRESENCE OF THE FIRST TARGET ANALYTE AT
THE FIRST DETECTION TEMPERATURE; THE APPROXIMATE FUNCTION *1-2* FOR APPROXIMATING SIGNAL
VALUES DEPENDENT ON THE PRESENCE OF THE FIRST TARGET ANALYTE AT THE SECOND DETECTION
TEMPERATURE; THE APPROXIMATE FUNCTION *1-3* FOR APPROXIMATING SIGNAL VALUES DEPENDENT ON
THE PRESENCE OF THE FIRST TARGET ANALYTE AT THE THIRD DETECTION TEMPERATURE; THE
APPROXIMATE FUNCTION *2-2* FOR APPROXIMATING SIGNAL VALUES DEPENDENT ON THE PRESENCE OF
THE SECOND TARGET ANALYTE AT THE SECOND DETECTION TEMPERATURE; THE APPROXIMATE
FUNCTION *2-3* FOR APPROXIMATING SIGNAL VALUES DEPENDENT ON THE PRESENCE OF THE SECOND
TARGET ANALYTE AT THE THIRD DETECTION TEMPERATURE; AND THE APPROXIMATE FUNCTION *3-3* FOR
APPROXIMATING SIGNAL VALUES DEPENDENT ON THE PRESENCE OF THE THIRD TARGET ANALYTE AT
THE THIRD DETECTION TEMPERATURE; AND (B) VALUE OF AT LEAST ONE MAGNITUDE PARAMETER,
WHEREIN THE AT LEAST ONE MAGNITUDE PARAMETER IS AT LEAST ONE OF A PLURALITY OF
MAGNITUDE PARAMETERS INDICATES RELATION SHIP BETWEEN SIGNAL VALUES DEPENDENT ON THE
PRESENCE OF AT LEAST ONE TARGET ANALYTE AMONG THE TARGET ANALYTES AT ANY ONE DETECTION
TEMPERATURE AMONG FROM THE FIRST DETECTION TEMPERATURE TO THE THIRD DETECTION
TEMPERAURE AND SIGNAL VALUES DEPENDENT ON THE PRESENCE OF THE AT LEAST ONE TARGET
ANALYTE AT ANOTHER ONE DETECTION TEMPERATURE AMONG FROM THE FIRST DETECTION
TEMPERATURE TO THE THIRD DETECTION TEMPERAURE

~S1720

END

<div align="center">

**INTERNATIONAL SEARCH REPORT**

</div>

| International application No. |
| --- |
| **PCT/KR2023/009459** |

## A. CLASSIFICATION OF SUBJECT MATTER

**G01N 21/64**(2006.01)i; **C12Q 1/686**(2018.01)i

According to International Patent Classification (IPC) or to both national classification and IPC

## B. FIELDS SEARCHED

Minimum documentation searched (classification system followed by classification symbols)

G01N 21/64(2006.01); C12Q 1/68(2006.01); G01N 15/06(2006.01); G01N 27/02(2006.01); G01N 33/50(2006.01); G16B 15/00(2019.01); G16B 30/00(2019.01); G16B 5/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 복수의 표적 분석물(a plurality of the targets analyte), 데이터 세트(data set), 검출온도(detection temperature), 근사 함수(approximation function), 크기 파라미터(magnitude parameter)

## C. DOCUMENTS CONSIDERED TO BE RELEVANT

| Category* | Citation of document, with indication, where appropriate, of the relevant passages | Relevant to claim No. |
| --- | --- | --- |
| DA | KR 10-2050601 B1 (SEEGENE, INC.) 04 December 2019 (2019-12-04)<br>See claim 1 and figure 1a. | 1-27 |
| A | KR 10-2018-0122481 A (SEEGENE, INC.) 12 November 2018 (2018-11-12)<br>See paragraphs [0027]-[0037] and figure 1. | 1-27 |
| A | KR 10-2018-0074800 A (SEEGENE, INC.) 03 July 2018 (2018-07-03)<br>See claims 1-5 and figure 1. | 1-27 |
| A | JP WO2009-093458 A1 (PANASONIC CORPORATION) 26 May 2011 (2011-05-26)<br>See paragraphs [0104]-[0120] and figure 4. | 1-27 |
| A | JP 2006-141394 A (F. HOFFMANN-LA ROCHE AKTIENGESELLSCHAFT) 08 June 2006 (2006-06-08)<br>See paragraphs [0002]-[0034]. | 1-27 |

| ☐ Further documents are listed in the continuation of Box C. | ☑ See patent family annex. |
| --- | --- |

| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| --- | --- |

| Date of the actual completion of the international search | Date of mailing of the international search report |
| --- | --- |
| **20 October 2023** | **20 October 2023** |

| Name and mailing address of the ISA/KR<br><br>**Korean Intellectual Property Office**<br>**Government Complex-Daejeon Building 4, 189 Cheongsa-ro, Seo-gu, Daejeon 35208** | Authorized officer |
| --- | --- |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2023/009459**

| Patent document cited in search report | | | Publication date (day/month/year) | Patent family member(s) | | | Publication date (day/month/year) |
|---|---|---|---|---|---|---|---|
| KR | 10-2050601 | B1 | 04 December 2019 | AU | 2014-387732 | A1 | 10 November 2016 |
| | | | | AU | 2014-387732 | B2 | 19 July 2018 |
| | | | | AU | 2018-204665 | A1 | 12 July 2018 |
| | | | | AU | 2018-204665 | B2 | 14 May 2020 |
| | | | | BR | 112016021782 | A2 | 17 October 2017 |
| | | | | BR | 112016021782 | B1 | 25 October 2022 |
| | | | | CA | 2941880 | A1 | 01 October 2015 |
| | | | | CN | 106103740 | A | 09 November 2016 |
| | | | | CN | 110452962 | A | 15 November 2019 |
| | | | | CN | 111893171 | A | 06 November 2020 |
| | | | | EP | 3122896 | A1 | 01 February 2017 |
| | | | | EP | 3122896 | A4 | 15 November 2017 |
| | | | | EP | 3122896 | B1 | 06 September 2023 |
| | | | | EP | 3122897 | A1 | 01 February 2017 |
| | | | | EP | 3122897 | A4 | 22 November 2017 |
| | | | | EP | 3122897 | B1 | 06 September 2023 |
| | | | | IL | 248034 | A | 30 November 2016 |
| | | | | IL | 248034 | B | 01 February 2022 |
| | | | | JP | 2017-518028 | A | 06 July 2017 |
| | | | | JP | 6620110 | B2 | 11 December 2019 |
| | | | | KR | 10-2016748 | B1 | 02 September 2019 |
| | | | | KR | 10-2018-0030945 | A | 26 March 2018 |
| | | | | KR | 10-2019-0133301 | A | 02 December 2019 |
| | | | | KR | 10-2021-0012072 | A | 02 February 2021 |
| | | | | KR | 10-2022-0020414 | A | 18 February 2022 |
| | | | | KR | 10-2022-0137812 | A | 12 October 2022 |
| | | | | KR | 10-2210548 | B1 | 01 February 2021 |
| | | | | KR | 10-2358734 | B1 | 08 February 2022 |
| | | | | KR | 10-2509130 | B1 | 14 March 2023 |
| | | | | MX | 2016012508 | A | 21 June 2017 |
| | | | | MY | 181022 | A | 16 December 2020 |
| | | | | NZ | 725593 | A | 29 June 2018 |
| | | | | RU | 2016-139287 | A | 03 May 2018 |
| | | | | RU | 2016-139287 | A3 | 03 May 2018 |
| | | | | RU | 2019-100495 | A | 31 January 2019 |
| | | | | RU | 2019-100495 | A3 | 21 February 2022 |
| | | | | RU | 2678403 | C2 | 28 January 2019 |
| | | | | SA | 516371903 | B1 | 02 October 2022 |
| | | | | SG | 11201607510 | QA | 28 October 2016 |
| | | | | US | 10752938 | B2 | 25 August 2020 |
| | | | | US | 2017-0016049 | A1 | 19 January 2017 |
| | | | | US | 2017-0247750 | A1 | 31 August 2017 |
| | | | | US | 2020-0340043 | A1 | 29 October 2020 |
| | | | | WO | 2015-147370 | A1 | 01 October 2015 |
| | | | | WO | 2015-147377 | A1 | 01 October 2015 |
| | | | | WO | 2015-147382 | A1 | 01 October 2015 |
| | | | | WO | 2015-147412 | A1 | 01 October 2015 |
| KR | 10-2018-0122481 | A | 12 November 2018 | KR | 10-2165933 | B1 | 14 October 2020 |
| | | | | US | 2019-0284605 | A1 | 19 September 2019 |
| | | | | WO | 2017-171469 | A1 | 05 October 2017 |

Form PCT/ISA/210 (patent family annex) (July 2022)

International application No.

**PCT/KR2023/009459**

| Patent document cited in search report | | | Publication date (day/month/year) | Patent family member(s) | | | Publication date (day/month/year) |
|---|---|---|---|---|---|---|---|
| KR | 10-2018-0074800 | A | 03 July 2018 | AU | 2016-356474 | A1 | 21 June 2018 |
| | | | | AU | 2020-256417 | A1 | 12 November 2020 |
| | | | | AU | 2020-256417 | B2 | 23 February 2023 |
| | | | | CA | 3005600 | A1 | 26 May 2017 |
| | | | | CA | 3005600 | C | 10 August 2021 |
| | | | | CN | 108351918 | A | 31 July 2018 |
| | | | | CN | 108351918 | B | 17 September 2021 |
| | | | | EP | 3378000 | A1 | 26 September 2018 |
| | | | | EP | 3378000 | A4 | 08 May 2019 |
| | | | | EP | 3378000 | B1 | 01 September 2021 |
| | | | | ES | 2899242 | T3 | 10 March 2022 |
| | | | | IL | 259466 | A | 31 July 2018 |
| | | | | IL | 259466 | B | 01 September 2022 |
| | | | | JP | 2019-503658 | A | 14 February 2019 |
| | | | | JP | 6685396 | B2 | 22 April 2020 |
| | | | | KR | 10-2270771 | B1 | 29 June 2021 |
| | | | | US | 2018-0336315 | A1 | 22 November 2018 |
| | | | | WO | 2017-086762 | A1 | 26 May 2017 |
| JP | WO2009-093458 | A1 | 26 May 2011 | WO | 2009-093458 | A1 | 30 July 2009 |
| JP | 2006-141394 | A | 08 June 2006 | CA | 2520619 | A1 | 29 March 2006 |
| | | | | EP | 1647910 | A1 | 19 April 2006 |
| | | | | US | 2006-0068427 | A1 | 30 March 2006 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4683195 A, Mullis
- US 4683202 A
- US 4800159 A
- KR 102050601
- US 5210015 A
- US 6117635 A
- US 7537886 B
- US 6977295 B
- US 7348141 B
- US 5876930 A
- WO 2012096523 A
- WO 2013115442 A
- KR 2013012312 W
- WO 2011037306 A
- US 6358691 B
- US 6194149 B
- US 8560247 B

**Non-patent literature cited in the description**

- **SAIKI et al.** *Science*, 1985, vol. 230, 1350-1354
- **SAMBROOK, J. et al.** Molecular Cloning. A Laboratory Manual. Cold Spring Harbor Press, 2001
- **TYAGI**. *Nature Biotechnology*, 14 March 1996
- **WHITCOMBE et al.** *Nature Biotechnology*, 1999, vol. 17, 804-807
- **NAZARENKO et al.** *Nucleic Acids Research*, 1997, vol. 25 (12), 2516-2521
- **DUCK P et al.** *Biotechniques*, 1990, vol. 9, 142-148
- **SHERRILL CB et al.** *Journal of the American Chemical Society*, 2004, vol. 126, 4550-4556
- **D. J. FRENCH et al.** *Molecular and Cellular Probes*, 2001, vol. 13, 363-374
- **BERNARD PS et al.** *Clin Chem*, 2000, vol. 46, 147-148
- PCR Protocols: A Guide to Methods and Applications. 1990
- **WALKER et al.** *Nucleic Acids Res.*, 1992, vol. 20 (7), 1691-6
- **WALKER**. *PCR Methods Appl*, 1993, vol. 3 (1), 1-6
- **PHYFFER et al.** *J. Clin. Microbiol.*, 1996, vol. 34, 834-841
- **VUORINEN et al.** *J. Clin. Microbiol.*, 1995, vol. 33, 1856-1859
- **COMPTON**. *Nature*, 1991, vol. 350 (6313), 91-2
- **LISBY**. *Mol. Biotechnol.*, 1999, vol. 12 (1), 75-99
- **HATCH et al.** *Genet. Anal.*, 1999, vol. 15 (2), 35-40
- **LIZARDI et al.** *BiolTechnology*, 1988, vol. 6, 1197
- **Y. MORI ; H. KANDA ; T. NOTOMI**. *J. Infect. Chemother*, 2013, vol. 19, 404-411
- **J. LI ; J. MACDONALD ; F. VON STETTEN**. *Analyst*, 2018, vol. 144, 31-67